(19) Europäisches Patentamt
European Patent Office
Office européen des brevets

(11) **EP 4 563 164 A1**

(12) **EUROPEAN PATENT APPLICATION**
published in accordance with Art. 153(4) EPC


(43) Date of publication:
**04.06.2025 Bulletin 2025/23**

(21) Application number: **23860896.2**

(22) Date of filing: **30.08.2023**

(51) International Patent Classification (IPC):
***A61K 47/68*** *(2017.01)* ***A61K 31/4745*** *(2006.01)*
***A61P 35/00*** *(2006.01)* ***C07D 491/22*** *(2006.01)*
***A61K 47/65*** *(2017.01)* ***G01N 33/574*** *(2006.01)*

(52) Cooperative Patent Classification (CPC):
**A61K 31/4745; A61K 47/65; A61K 47/68; A61P 35/00; C07D 491/22; G01N 33/574**

(86) International application number:
**PCT/KR2023/012935**

(87) International publication number:
**WO 2024/049220 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.08.2022 KR 20220109436**
**07.12.2022 KR 20220170146**
**17.01.2023 KR 20230006435**
**13.03.2023 KR 20230032597**



(71) Applicant: **Pinotbio, Inc.**
**Suwon-si, Gyeonggi-do 16506 (KR)**

(72) Inventors:
- **JUNG, Doo Young**
  **Suwon-si Gyeonggi-do 16506 (KR)**
- **LEE, Jin Soo**
  **Suwon-si Gyeonggi-do 16506 (KR)**
- **CHO, Hyun Yong**
  **Suwon-si Gyeonggi-do 16506 (KR)**
- **LEE, Byeong Sung**
  **Suwon-si Gyeonggi-do 16056 (KR)**
- **CHUN, Young Hwa**
  **Suwon-si Gyeonggi-do 16506 (KR)**
- **SHIN, Seung Gun**
  **Suwon-si Gyeonggi-do 16506 (KR)**
- **KWEON, So Hui**
  **Suwon-si Gyeonggi-do 16505 (KR)**
- **GO, Areum**
  **Suwon-si Gyeonggi-do 16506 (KR)**

(74) Representative: **Prüfer & Partner mbB**
**Patentanwälte · Rechtsanwälte**
**Sohnckestraße 12**
**81479 München (DE)**


(54) **ANTIBODY-DRUG CONJUGATE HAVING CAMPTOTHECIN-BASED DRUG LINKED TO ANTIBODY HAVING LOW ANTIGEN-BINDING AFFINITY THROUGH LINKER**


(57) The present invention relates to an immunoconjugate or a pharmaceutically acceptable salt thereof characterized in that a camptothecin-based drug (A) is bound, through a linker (C), to: an antibody or an antigen-binding fragment (B-1) thereof having a dissociation constant $K_d$ value of $1*10^{-8}$ M to $1*10^{-6}$ M (exclusive of $1*10^{-6}$ M) with respect to an epitope of a cell surface protein; or an antibody or antigen-binding fragment (B-2) thereof which binds to a cell surface protein expressed in both normal cells and cancer cells and which has a higher binding affinity with respect to an epitope of the cell surface protein when binding divalently than when binding monovalently.


[FIG. 12]

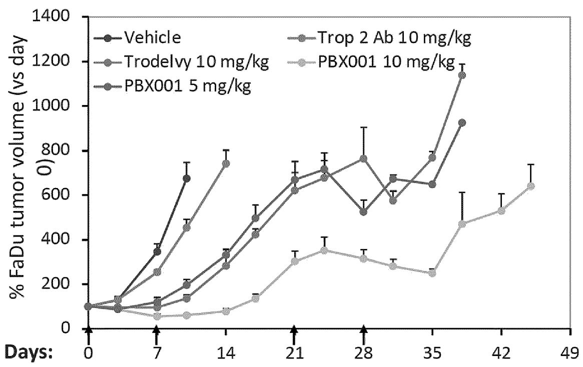


EP 4 563 164 A1

## Description

## TECHNICAL FIELD

The present invention relates to an immunoconjugate or a pharmaceutically acceptable salt thereof, wherein a camptothecin-based drug (A) that degrades DDX5 protein is bound, through a linker (C), to: an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

## BACKGROUND ART

Organs are made up of different types of cells and matrix components that are arranged to form specific microstructures that evolve to optimize structure, efficacy and function. The epithelial cell membrane of the small intestine, muscle cells of muscles and nerve cells form specific fine anatomical structures during development, and each cell maintains the homeostasis of the tissue or organ even as an adult cell.

As cancer grows, the surrounding normal tissues and cancerous tissues dynamically and continuously undergo structural changes, and homeostasis is disrupted. The excessive division of cancer cells, proteolytic enzyme activity and matrix production can all alter cell-cell or cellmatrix relationships and affect morphogenesis. The well-known epithelial-mesenchymal transition (EMT) is an example of morphogenetic changes in which cells change from an epithelial state, where only a two-dimensional surface is affected, to a permeable mesenchymal state, by being surrounded by a three-dimensional extracellular matrix.

Camptothecin is a selective inhibitor of type I topoisomerase, which is an isomerase involved in DNA replication and recombination. It is a natural anti-tumor alkaloid isolated from *Camptotheca acuminata*, which is native to China, by Wall et al. in the United States in 1966. Since it was found to exhibit strong cytotoxicity *in vitro,* development through clinical trials was initiated at the National Cancer Research Center (NCI) and the like, but due to the limitation of extremely poor solubility, various side effects such as bone marrow suppression and hemorrhagic cystitis were exhibited, and as a result, development was halted. However, since 1990, it has been confirmed that camptothecin exhibits an antitumor effect by selectively inhibiting DNA type I topoisomerase, unlike its unique mechanism of action, which is to inhibit DNA type II topoisomerase.

DNA topoisomerase is a member of the gyrase enzyme family. These are enzymes in the nucleus that temporarily cleave DNA or unwind the double helix when the cell needs access to genetic material for replication or transcription. They also participate in a variety of intracellular activities, including chromosome condensation and recombination and DNA repair. The genetic code of the topoisomerase enzyme is highly conserved among species.

Type I topoisomerase, which is the drug target of camptothecin, has been observed to have increased levels in various malignant tumors. This drug does not inhibit free enzymes, but stabilizes the covalent bonds of the topo-DNA complex and prevents the reconnection of cleaved DNA fragments. Therefore, the sensitivity of cells to these topoisomerase-targeting drugs is associated with the level of enzymes that are present in the nucleus. This drug prevents transcription from proceeding by interfering with DNA recombination. The higher the amount of type I topoisomerase, the more cleavable complexes are formed, which means higher drug sensitivity. This has important clinical relevance, and type I topoisomerase inhibitors are used to increase the expression of type II topoisomerases, thereby making them more sensitive to type-2 topoisomerases inhibitors. This result is supported by the antagonistic relationship between type I and type II topoisomerases. Unlike type II topoisomerase, type I topoisomerase is not closely related to proliferation in normal tissues, and is present in large quantities in solid tumors of the "S" phase with active cell division, such as colon cancer, ovarian cancer and esophageal cancer, including lymphoma, compared to surrounding normal tissues. Actually, it is known to cause cell death by blocking the replication and transcription of genes in tumor cells during the "S" phase of the cell cycle.

Camptothecin (CPT) has low water solubility, and in preparation for clinical trials, the National Cancer Institute (NCI) prepared a water-soluble sodium salt (NSC100880). Phase I and II clinical trials were not completed due to the high toxicity exhibited by the compound (hemorrhagic cystitis, gastrointestinal toxicity such as nausea, vomiting, diarrhea and bone marrow suppression, especially leukopenia and thrombocytopenia).

Subsequently, a number of CPT analogs were synthesized to obtain compounds with lower toxicity and higher water solubility. There are two medications, irinotecan (CPT-11) and topotecan.

Irinotecan (CPT-11), jointly developed by Japan's Dallchi and Yakult, was the world's first camptothecin-based anticancer drug in 1994 and was released in Europe and Japan after proving its effectiveness in lung cancer (small cell and non-small cell lung cancer). In 1995, its effectiveness against colon cancer and breast cancer was additionally proven. In addition, topotecan, developed by Glaxo Smith Kline, was approved for effectiveness against metastatic ovarian cancer by

the U.S. FDA in April 1995 and was released. CKD-602 (belotecan), which is a new camptothecin derivative recently developed in Korea, has a strong type I topoisomerase inhibitor effect and is water-soluble, and it successfully overcame the toxicity caused by existing poor solubility.

All camptothecin derivatives identified to date contain a parent structure with five rings that are essential for cytotoxicity (FIG. 1). In terms of molecular structure, the E-ring and A-, B-ring regions were identified as important regions. Camptothecin consists of a pentacyclic structure with a lactone in the E-ring, which is essential for cytotoxicity. Among these, the lactone group and alpha hydroxyl group located at carbon 20 of the E-ring are important for the stability of type I topoisomerase-DNA by-products, and the fact that modification of the A- and B-rings can increase water solubility and activity has been proven. Modifications on the first ring have been proven, for example, in the case of the above-mentioned drugs, to increase aqueous solubility and allow greater tolerability of the drugs.

In the case of CKD-602, it was also attempted to substitute the B-ring region at carbon 7 to increase water solubility and an anticancer effect. Lee et al. reported that CKD-602 has superior anticancer effects compared to camptothecin and topotecan in a wide range of cancer cell lines. In addition, it was confirmed to be a relatively safe drug with a maximum tolerated dose (MTD) of 25 mg/kg in the L1210 leukemia nude mouse model. The generally known side effects of camptothecin-based drugs can be broadly classified into hematological side effects and non-hematological side effects. Hematological side effects include neutropenia with fever, sepsis and bleeding, while non-hematological side effects include skin side effects such as nausea, vomiting and hair loss, and toxicity to the gastrointestinal tract, kidneys and nervous system. In an animal study of CKD-602, Kim et al. found no adverse drug reactions other than increased gastric secretion, even when it was administered at doses 10 times higher than the clinically applicable dose, among the side effects of these camptothecin drugs. In addition, recent domestic clinical studies have proven its relative safety, with only reversible and controllable side effects such as neutropenia and leukopenia reported rather than serious systemic toxicity. However, to date, it is mainly used in a limited manner with the indications being the treatment of refractory or recurrent ovarian and colon cancers that have relapsed or worsened after standard treatment and are unlikely to respond to further chemotherapy or surgery, in patients who have failed standard chemotherapy or cannot be treated with standard chemotherapy, in resistant or recurrent limited disease small cell lung cancer that has failed first-line chemotherapy, and in the treatment of extensive disease small cell lung cancer.

SN-38 is a potent topoisomerase-I inhibitor with $IC_{50}$ values in the nanomolar range in several cell lines. It is an active form of irinotecan, which is a prodrug used to treat colorectal cancer, and is also active in lung, breast and brain cancer. In the case of Trop-2-SN-38 ADC, it is currently being successfully developed in a number of cancer types such as TNBC, bladder and gastric cancer, but resistance issues of SN-38 still remain (overexpression of drug efflux transporter, epigenetic silencing of Top1, anti-apoptic protein increase, etc.).

According to US Patent No. 9,629,926 B, the biodistribution of hRS7-CL2A-SN-38 is uptaken by tumors similar to the parent hRS7 IgG, but is quickly eliminated with a two-fold higher liver uptake due to the hydrophobicity of SN-38. As the ADC is eliminated through the liver, hepatic and gastrointestinal toxicity was expected to be dose limiting.

Derivatives of the camptothecin family, such as SN-38 and Dxd (a derivative of the clinical-stage topoisomerase I inhibitor Exatecan), have recently been successfully developed as novel payloads for antibody-drug conjugates (ADCs). As a result, two ADCs using camptothecin payloads, that is, Trodelvy (Sacituzumab Govitecan) with SN-38 payload and Enhertu (Trastuzumab Deruxtecan) with Dxd payload, were recently approved by the FDA. These payloads have moderate cytotoxicity compared to traditional ultratoxic ADC payloads such as MMAE, MMAF or PBD, but the superior safety profile allows for higher drug-to-antibody ratios (DARs), such as 8, rather than 2 or 4 in conventional ADCs. Additionally, these payloads allow the use of linker systems with faster drug release profiles, such as the CL-2A or GGFG linker systems. The combination of these components has led to the development of new ADCs with a broader therapeutic spectrum.

Antibody-drug conjugates (ADCs) are a novel drug development modality that attaches a potent anticancer drug (payload) that can efficiently inhibit the growth of cancer cells even at low doses to an antibody that can selectively recognize cancer cells through a linker of a special structure. Since ADC selectively delivers drugs with strong anticancer efficacy to cancer cells through antibodies, it shows strong anticancer efficacy without systemic side effects, but its effectiveness is limited due to the limitation of the amount of drug delivered.

In general, when linking a mostly hydrophobic linker-payload to a hydrophilic antibody, the DAR, which indicates the number of drugs attached to each antibody, does not present a major problem in the range of 1 to 8, but if it exceeds this range, aggregation occurs, there may be problems with manufacturing/transportation/use, or safety issues may arise due to the formation of aggregates in the blood. Due to high lipid solubility, drug absorption and payload release do not occur by depending on the drug target of the antibody, and non-selective uptake occurs in macrophages and the like, and unexpected side effects occur. In addition, it is possible to increase the dosage of the antibody while fixing the DAR at a specific value, but in this case, (1) problems of non-selective uptake of ADCs and subsequent off-target toxicity may occur, and (2) the antibodies that constitute ADCs bind to drug targets on the surface of cancer cells with strong affinity, but when these happen, the drug targets around blood vessels are first saturated, and after a certain amount of antibodies are bound, additional antibodies (ADCs) cannot penetrate into cancer tissues, resulting in problems such as the administered

ADCs becoming useless.

Among these problems, in order to solve the problems of off-target toxicity that may occur after non-selective uptake, many ADCs using camptothecin compounds that show relatively high safety in normal tissues are being developed, and particularly, through the success of Daiichi-Sankyo's Enhertu and Gilead's Trodelvy, it can be seen that this approach effectively works. In other words, part of Enhertu's excellent success is due to the use of a safe payload, which increases the dosage of ADC from the previously commonly used level of 2.7 mpk to 4.8 to 5.4 mpk, thereby ensuring that the payload released from the ADC is evenly distributed throughout the cancer tissue.

## DISCLOSURE

## TECHNICAL PROBLEM

It is very important to easily deliver ADC or other targeted-drug conjugate substances into cancer tissue, and in fact, looking at the results of the Peptide-Drug Conjugate targeting Nectin-IV, which was recently announced by Bicycle, the Peptide-Drug Conjugate (DAR 1) with low DAR shows superior efficacy compared to Padcev (DAR 4), which is a commercial ADC with high DAR, thereby demonstrating the importance of efficiently distributing carriers to cancer tissues. Nevertheless, most of the antibodies used in the development of ADCs until recently are those that bind strongly to drug targets with a $K_d$ value of $1*10^{-10}$ M or more to less than $1*10^{-8}$M, and as a result, the ADC and the payloads released from the ADC are mainly concentrated around the vessels inside the cancer, which is showing limitations in their effectiveness.

Accordingly, the inventors of the present invention (1) used an antibody with a $K_d$ value of $1*10^{-8}$ M or more to less than $1*10^{-6}$M to ensure smooth penetration of the antibody into the cancer tissue, and (2) derived an ADC using a camptothecin compound as a payload to ensure sufficient ADC dosage, and through this, it was possible to simultaneously secure sufficient ADC usage and strong efficacy through penetration into cancer tissue.

In addition, due to the characteristics of antibodies that internalize through receptors, the stronger the binding force to an epitope of the antigen, the more internalization can occur, and thus, by using the binding power to the antigen to increase the therapeutic effect in the opposite way, the present invention aims to design an ADC that shows an anticancer effect while solving the problems of on-target toxicity by using an antibody that targets cell surface proteins expressed on both normal cells and cancer cells and has a higher binding affinity when binding to an epitope of the cell surface protein bivalently than when binding monovalently.

## TECHNICAL SOLUTION

A first aspect of the present invention provides an immunoconjugate or a pharmaceutically acceptable salt thereof, wherein a camptothecin-based drug (A) that degrades DDX5 protein is bound, through a linker (C), to: an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

A second aspect of the present invention provides a pharmaceutical composition for preventing or treating cancer, including the immunoconjugate of the first aspect or a pharmaceutically acceptable salt thereof as an active ingredient.

Hereinafter, the present invention will be described.

In the present specification, cancer and tumor may be used interchangeably.

The therapeutic index of a drug is a measure of the safety and efficacy of a drug in medical treatment. It is defined as a ratio between the dose of a drug that causes therapeutic effects and the dose that causes toxicity or adverse effects. In other words, it represents a range between the therapeutic and toxic doses of a drug.

A high therapeutic index indicates a wide margin of safety where the effective dose is significantly lower than the toxic dose. This means that the drug may be administered at therapeutic levels without causing serious side effects or toxicity. Drugs with a higher therapeutic index are generally considered safer and more desirable for clinical use.

On the other hand, a low therapeutic index means a narrow margin of safety. In these cases, the effective dose and toxic dose are relatively close, and the risk of side effects or toxicity is high when using the drug. Drugs with a low therapeutic index require careful monitoring and accurate dosing to avoid harm to patients.

The therapeutic index is an important consideration in drug development because it helps determine the dosage range that can provide the desired therapeutic effect while minimizing the risk of side effects. It provides useful information when prescribing drugs and allows a medical professional to evaluate the overall benefit-to-risk ratio of the drug.

In order for a drug to act effectively *in vivo,* the concentration of a drug in the body must be maintained within the therapeutic range for a certain period of time or longer. If the drug is present in excessive amounts in the body, it becomes

toxic, and if the drug is present in too small amounts, it has no therapeutic effect.

Drug efficacy means that it remains in the body without being decomposed for the expected period of time to exert an effect on the target indication. If the metabolic rate is low, the blood concentration is maintained for a long period of time, and thus, the efficacy lasts longer.

As used herein, the term "antibody" includes immunoglobulin molecules that are immunologically reactive with a specific antigen, and includes, for example, protein molecules that serve as a ligand that specifically recognizes a cell membrane receptor that is an antigen, and it includes all of polyclonal antibodies, monoclonal antibodies and whole antibodies. In addition, the term also includes chimeric antibodies and bivalent or bispecific molecules, diabodies, triabodies and tetrabodies. The term further includes single-chain antibodies possessing a binding function to FcRn, scaps, derivatives of antibody constant regions and artificial antibodies based on protein scaffolds. A total antibody is structured with two full-length light chains and two full-length heavy chains, with each light chain linked to the heavy chain by a disulfide bond. The total antibody includes IgA, IgD, IgE, IgM and IgG, and IgG subtypes include IgG1, IgG2, IgG3 and IgG4.

As used herein, the term "antigen-binding site-containing fragment" may be any fragment of an antibody that retains the antigen-binding activity of the antibody. Exemplary antibody fragments include, but are not limited to, single-chain antibodies, Fd, Fab, Fab', $F(ab')_2$, dsFv or scFv. The Fd refers to a heavy chain portion included in the Fab fragment. The Fab has a structure that includes the variable regions of light and heavy chains, the constant region of a light chain, and the first constant region (CH1 domain) of a heavy chain, and has one antigen binding site. Fab' differs from Fab in that it has a hinge region including one or more cysteine residues at the C terminus of the heavy chain CH1 domain. The $F(ab')_2$ antibody is produced when the cysteine residue in the hinge region of Fab' forms a disulfide bond. Fv (variable fragment) refers to the minimum antibody fragment containing only the heavy chain variable region and the light chain variable region. In double disulfide Fv (dsFv), the heavy chain variable region and light chain variable region are connected by a disulfide bond, and in single chain Fv (scFv), the heavy chain variable region and light chain variable region are generally covalently connected through a peptide linker. Such antibody fragments may be obtained using proteolytic enzymes (e.g., Fab can be obtained by restriction digestion of the total antibody with papain, and $F(ab')_2$ fragments can be obtained by digestion with pepsin), preferably, it may be produced through genetic recombination technology.

In the present invention, the term "drug-linker conjugate" refers to a material for the preparation of an immunoconjugate or carrier-drug conjugate, which not only refers to an antibody, an antigen-binding site-containing fragment thereof or a carrier that is not linked. Depending on the purpose, it may be used as an immunoconjugate or carrier-drug conjugate by combining with any antibody, an antigen-binding site-containing fragment thereof or a carrier.

In the present invention, the term "immunoconjugate" refers to a complex in which a cytotoxic drug-linker conjugate is linked to an antibody or antigen-binding fragment thereof.

Since the antibody-drug conjugate (ADC) is an example of an immunoconjugate, the description of ADC and the description of immunoconjugate may be used interchangeably in the present invention.

When the immunoconjugate is administered *in vivo,* the antibody or antigen-binding site-containing fragment thereof, which is a component thereof, binds to a target antigen and then releases a drug, thereby allowing the drug to act on target cells and/or surrounding cells, and thus, excellent efficacy and reduced side effects as a target drug may be expected.

Factors that significantly affect the effectiveness of immunoconjugates are, in particular, (1) drug potency, (2) drug linker stability, (3) efficient on-target drug release and the like. Since multiple factors have a complex impact on efficacy, it is extremely difficult to predict the efficacy of an immunoconjugate, which is a combination of these factors, based solely on what is known about each individual factor.

In the present invention, the immunoconjugate may have an average drug-to-antibody ratio (DAR) of 2 to 12, and preferably, DAR, which indicates the number of drugs attached to one antibody based on antibodies, is 4 to 8, or the DAR may be 1 to 4 based on the antigen-binding site-containing fragment.

Knowing the properties of a drug can help a medical professional use it properly. Pharmacodynamic and pharmacokinetic parameters of a drug are helpful in understanding the properties of a drug.

Pharmacodynamics explains the size and pattern of changes (effect) (cell viability, clinical effect) (therapeutic action, toxic effect, adverse effect) that occur in cells or the body after a drug binds to a receptor in terms of their relationship with drug concentration.

Pharmaco-kinetics (PK) shows how drug concentration changes as it moves through different compartments of the body through ADME, depending on the drug or the modality of the drug.

Meanwhile, the *in vivo* efficacy and *in vivo* side effects of anticancer drugs are closely related to the absorption, distribution, metabolism and excretion (ADME) characteristics of anticancer drugs. The ADME of a drug determines its pharmacokinetics, which describes how the drug moves through the body and interacts with tissues and organs.

The immunoconjugate or pharmaceutically acceptable salt thereof according to the present invention is constituted such that

a camptothecin-based drug (A) that degrades DDX5 protein is bound, through a linker (C), to: an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

In the present invention, the camptothecin-based drug that degrades DDX5 protein may be an active camptothecin derivative represented by Chemical Formula 1, Chemical Formula 2 or Chemical Formula 1-1 below, which is designed to bind to DDX5 protein and E3 ligase.

[Chemical Formula 1]

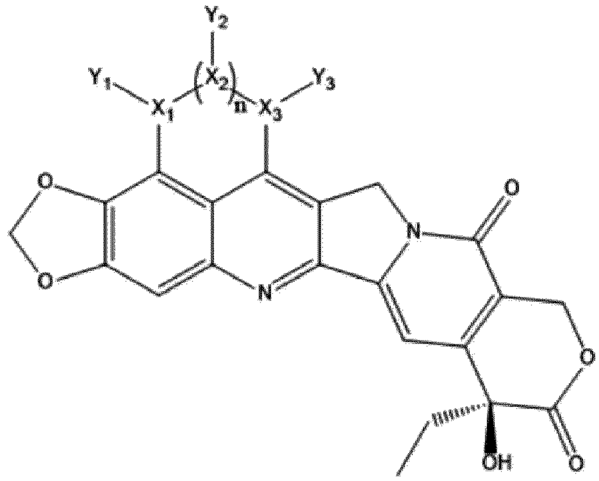

[Chemical Formula 2]

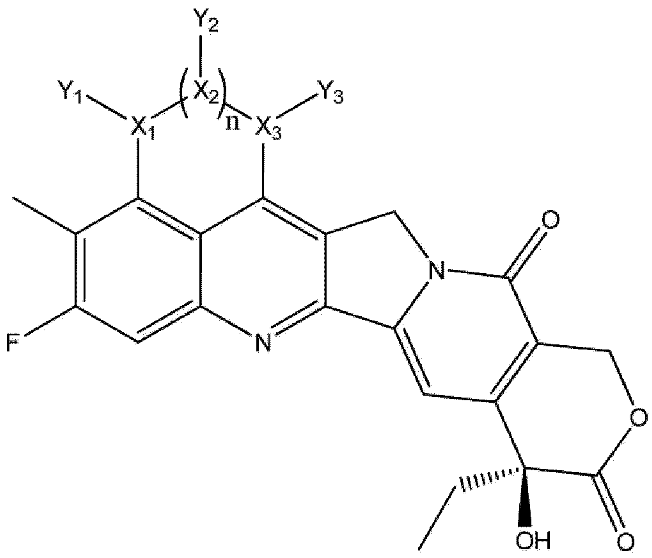

$X_1$ and $X_3$ may each independently be carbon, oxygen, nitrogen or sulfur, and $X_1$ and $X_3$ may be the same or different,
$X_2$ may be carbon, oxygen, nitrogen, sulfur, single bond or double bond,
$X_1$, $(X_2)_n$ and $X_3$ may form a pentagonal, hexagonal or heptagonal ring (values of n = 0 to 2), and
$Y_1$, $Y_2$ and $Y_3$ may each independently be hydrogen or a functional group including oxygen, nitrogen, phosphorus or sulfur.

[Chemical Formula 1-1]

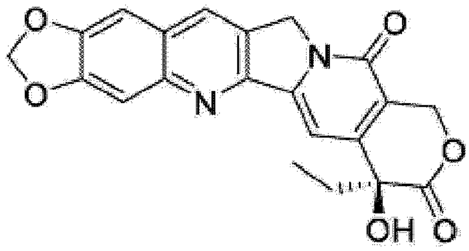

FL118

Herein, the non-limiting examples of a functional group including oxygen, nitrogen, phosphorus or sulfur may include a functional group selected from the group consisting of -CHO, -COOH, $-NH_2$, -SH, $-CONH_2$, $-PO_3H$, $-PO_4H_2$, $-OPO_4H$, $-PO_2(OR^1)(OR^2)$($R^1$, $R^2$=$C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-SO_3H$, $-OSO_3H$, $-NO_2$, $-N_3$, $-NR_3OH$(R=$C_nH_{2n+1}$, $0 \le n \le 16$), $-NR_3^+X^-$(R= $C_nH_m$, $0 \le n \le 16$, $0 \le m \le 34$, X = OH, Cl or Br), $NR_4^+X^-$(R= $C_nH_m$, $0 \le n \le 16$, $0 \le m \le 34$, X = OH, Cl or Br), -COSH, -COOCO-, -CORCO- (R = $C_lH_m$, $0 \le l \le 3$, $0 \le m \le 2l+1$), -COOR, -CN, $-N_3$, $-N_2$, -NROH(R = $C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-NR^1NR^2R^3$($R^1$, $R^2$, $R^3$ = $C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-CONHNR^1R^2$($R^1$, $R^2$ = $C_sH_tN_uO_wS_xP_yX_z$, X = -F, -Cl, -Br or -I, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), $-NR^1R^2R^3X'$($R^1$, $R^2$, $R^3$ = $C_sH_tN_uO_wS_xP_yX_z$, X = -F, -Cl, -Br or -I, X'= $F^-$,$Cl^-$,Br, or $I^-$, $0 \le s \le 20$, $0 \le t \le 2(s+u)+1$, $0 \le u \le 2s$, $0 \le w \le 2s$, $0 \le x \le 2s$, $0 \le y \le 2s$, $0 \le z \le 2s$), -OH, -O-, >C=O, -SS-, -SO-, $-NO_2$, -COX(X = F, Cl, Br or I), -COOCO-, -CONH-, -CN, $-SCOCH_3$, -SCN, -NCS, -NCO, -OCN, -CN, -F, -Cl, -I, -Br, an epoxy group, -hydrazone, $-ONO_2$, $-PO(OH)_2$, - $C=NNH_2$, -HC=CH-, -C=C-, -C≡C- and hydrocarbons having 2 or more carbon atoms.

The non-limiting examples of an active camptothecin derivative represented by Chemical Formula 1 are as follows.

[Chemical Formula 3]

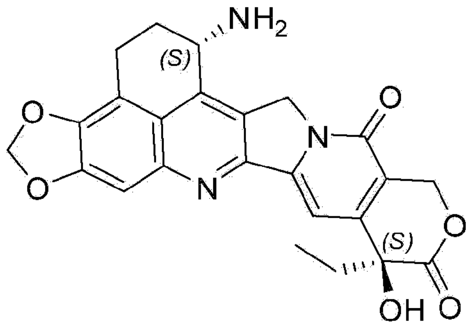

[Chemical Formula 3-1]

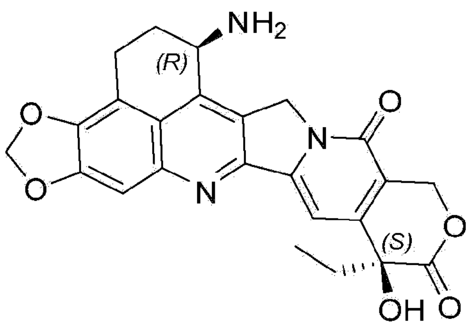

[Chemical Formula 4]

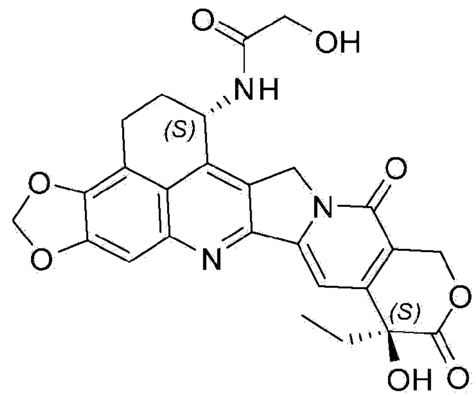

[Chemical Formula 4-1]

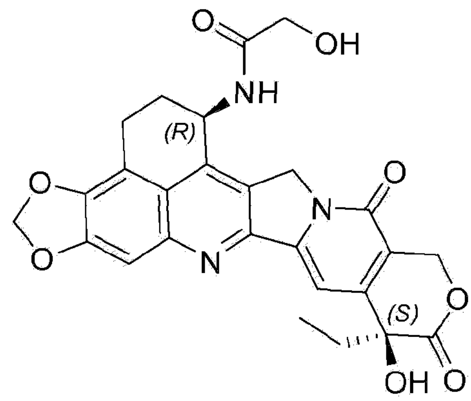

[Chemical Formula 5]

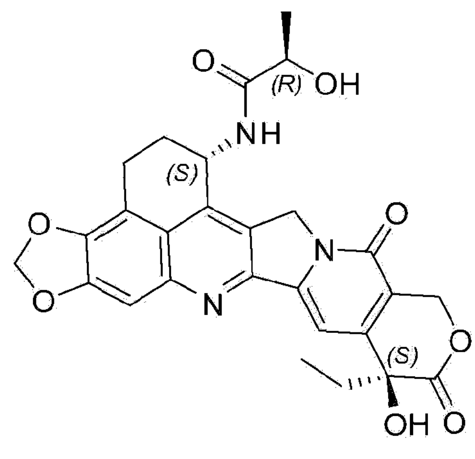

[Chemical Formula 5-1]

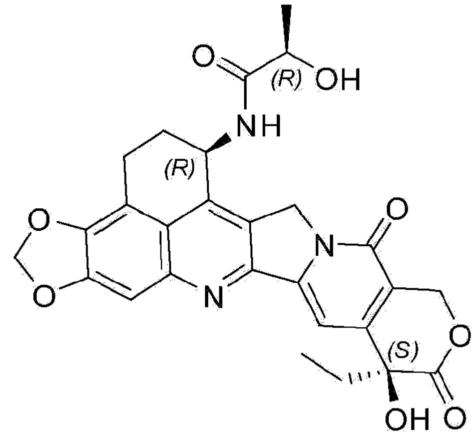

[Chemical Formula 6]

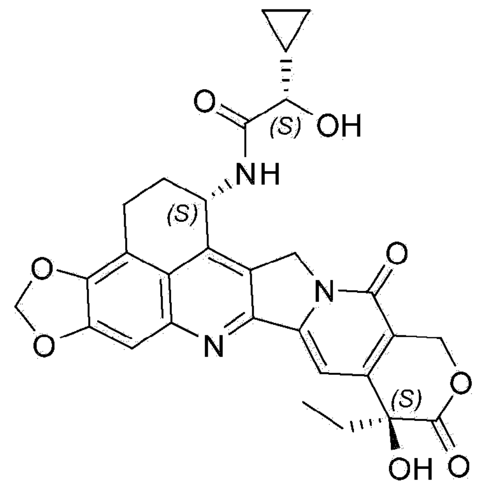

Considering the design intent of ADCs to deliver cytotoxic molecules to a target by linking tumor-specific antigens to antibodies targeting the same, ADCs are expected to be less toxic than conventional chemotherapy. However, most ADCs are still burdened with not only off-target toxicities similar to cytotoxic payloads, but also on-target toxicities and other unknown and potentially life-threatening side effects. As the clinical indications for ADCs are rapidly expanding by including use in therapeutic settings and various combinations, extensive efforts are underway to improve safety. Current approaches include clinical trials to optimize dosing and treatment schedules, modifying individual ADC components, identifying biomarkers predictive of toxicity, and developing innovative diagnostic tools.

In consideration of this point, due to the active camptothecin derivative designed to bind to DDX5 protein and E3 ligase according to the present invention, the dosage of the immunoconjugate of the present invention or a pharmaceutically acceptable salt thereof is preferably 4.8 mpk or less, and preferably, it may be at the level of 2 mpk to 4.5 mpk.

In addition, the immunoconjugate of the present invention or a pharmaceutically acceptable salt thereof may have a DAR of 4 to 8, which indicates the number of drugs attached to each antibody based on the antibody, or a DAR of 1 to 4 based on the antigen-binding site-containing fragment.

When the antibodies that constitute the ADC bind to a drug target on the surface of cancer cells with strong affinity, the drug target around the blood vessels is first saturated, which prevents additional antibodies (ADC) from penetrating into the cancer tissue after a certain amount of antibody binds, and in order to solve this problem of the administered ADC being useless and to ensure that the payload released from the ADC is evenly distributed throughout the cancer tissue, the immunoconjugate of the present invention is characterized by using an antibody or an antigen-binding site-containing fragment thereof (B-1) having a dissociation constant $K_d$ value of $1*10^{-8}$ M or more and less than $1*10^{-6}$ M for an epitope of a cell surface protein; or an antibody or an antigen-binding site-containing fragment thereof (B-2) that binds to a cell surface protein expressed on both normal cells and cancer cells but has higher binding affinity when binding divalently than

monovalently to an epitope of the cell surface protein (FIG. 22).

Due to the above-described antibody or antigen-binding site-containing fragment (B-2) thereof, the immunoconjugate and/or the payload released from the immunoconjugate of the present invention is not only accumulated around the blood vessels inside the cancer, but also it may be penetrated inside the cancer tissue.

In addition, by the above-described antibody or an antigen-binding site-containing fragment thereof (B-2), the immunoconjugate of the present invention not only increases the therapeutic effect by (i) attenuating internalization through receptor-mediated endocytosis, and as a result, (ii) it may exert an anti-cancer effect while solving the problem of on-target toxicity of the immunoconjugate, and (iii) the therapeutic effect of the immunoconjugate that has penetrated into the cancer tissue may alleviate the solid stress within the cancer microenvironment formed as the cancer cells proliferate, and optionally increase the access of anticancer drugs and/or immunotherapeutic agents.

In this case, the anticancer agent may include the immunoconjugate of the present invention and/or a payload released from the immunoconjugate. Therefore, the payload released from the immunoconjugate of the present invention may effectively exert a bystander effect.

Additionally, in the immunoconjugate of the present invention, the camptothecin-based drug (A), which degrades the DDX5 protein, is converted from the active lactone form to the inactive carboxylate form as a TOP1 inhibitor at the pH of 7.4 in blood or extracellular fluid (FIG. 24 and FIG. 25), an antibody or an antigen-binding site-containing fragment thereof may selectively recognize cancer cells that want to be killed through camptothecin-based drugs.

Therefore, the present invention not only shows strong anticancer efficacy without systemic side effects because it selectively delivers a drug with strong anticancer efficacy to cancer cells through an antibody or an antigen-binding site-containing fragment thereof, but also uses a camptothecin-based drug (A) that degrades DDX5 protein as a payload such that it is possible to solve the problem of limited efficacy due to limitations in the amount of a delivered payload drug.

[Antibody-Drug Conjugate (ADC) and Side Effects Thereof]

Antibody-drug conjugates (ADCs) can be used as a type of targeted therapy in cancer treatment. For example, a monoclonal antibody and a cytotoxic drug are combined to specifically deliver the drug to cancer cells. ADC is a new anticancer treatment modality that combines an anticancer drug that exhibits strong anticancer efficacy at low concentrations of several pM to several nM, by binding to a specific antigen expressed on the surface of cancer cells, and using a linker that maintains the bound state without releasing a drug until the ADC is internalized inside the cell in an antibody that can selectively recognize cancer cells, and then releases the drug immediately after it is internalized inside the cell.

After binding to the target cell, ADC is internalized into the cell by a process called receptor-mediated endocytosis. In this case, a sufficient concentration of the active drug must enter the cell, but the internalization process by the antigen-antibody complex is generally inefficient, and the number of antigens on the cell surface is generally limited to $<1 \times 10^5$ receptors/cell, and thus, very potent drugs must be used to be able to kill tumor cells sufficiently even at low drug concentrations. Additionally, since the drug must be conjugated with minimal effect on the antibody, the amount of payload that can be transported is limited. Therefore, drugs bound to antibodies and used as ADCs are drugs that are 100 to 1,000 times more cytotoxic than commonly used anticancer drugs.

Most of the powerful cytotoxic drugs introduced into ADCs were so toxic that they even affected normal cells due to the bystander effect. This indicates that cytotoxic drugs to be applied to ADC must be able to kill most tumor cells at low concentrations (nM or pM) and must be controlled to exhibit therapeutic effects.

Although ADCs show new possibilities in combining the selectivity of antibodies and the strong cytotoxicity of anticancer drugs to provide powerful anticancer treatment efficacy to many cancer patients while lowering the risk of systemic side effects, many existing ADCs have shown various limitations in practical applications. When the DAR exceeds a certain number, the ADC is indiscriminately absorbed by normal cells/normal tissues other than cancer cells due to the hydrophobicity of the drug and linker used, and the drug is released, resulting in a poor PK profile, and there was a problem of unexpected toxicity.

In the case of Daiichi-Sankyo's Enhertu, which is a high DAR ADC that uses a combination of a relatively less toxic payload and a hydrophilic linker, in order to conjugate drugs and linkers to antibodies, all interchain disulfide bonds of the IgG1 format are cleaved, and all 8 free thiol functional groups obtained are reacted with the drug-linker combination to obtain a pseudo-homogeneous ADC with DAR of about 8. In this case, the linker and payload used have a stronger hydrophilicity compared to the previously used linker and payload, and thus, there is almost no aggregation problem even at high DAR levels, and the PK profile also has the characteristic of being stably maintained. Nevertheless, the problem of non-selective uptake still exists, resulting in severe inflammatory side effects in 10 to 15% of patients.

The term "non-selective uptake" generally refers to the uptake of a substance by a cell without a specific target or preference. Non-selective uptake in the context of ADC means that cells that do not express the target antigen absorb the ADC or a payload thereof.

It is estimated that only approximately 0.1% of the ADC injected dose is delivered to the targeted disease cell population, with the majority of the administered dose being metabolized "off-site" within non-target healthy cells, thereby

potentially resulting in unwanted toxicity. Off-site ADC toxicity may be classified as "on-target" or "off-target," with on-target toxicity proceeding through ADC binding to target cell surface proteins on healthy cells. Each component of the ADC, including the antibody, linker and payload, may affect the degree of toxicity caused by the ADC.

The mechanism of ADC toxicity is illustrated in FIG. 26. The uptake of intact ADCs into normal cells may occur through non-specific intracellular uptake or through internalization by binding to target antigens or Fc/C-type lectin receptors. Payloads released from ADC deconjugation or other targeted/non-targeted apoptotic cells in the extracellular fluid may also enter normal cells, through passive diffusion in the case of membrane-permeable payloads.

Conceptually, ADCs may enhance the selectivity of chemotherapy by promoting the targeted delivery of cytotoxic payload molecules to the desired cell population (on-target, on-site toxicity), while simultaneously expanding the therapeutic index by reducing payload delivery to non-target healthy tissues.

In the early stages of technology development, an expected safety issue for anticancer ADCs was on-target (i.e., target-mediated) toxicity in tissues where the target antigen was expressed to some extent, and differential expression of the target in cancer cells and healthy tissues was expected to become an important factor for determining the therapeutic index of the ADC. However, subsequent clinical experience with ADCs has demonstrated that dose-limiting toxicities (DLTs) are rarely caused by target expression in healthy tissues.

Preclinical and clinical data from 20 ADC investigational new drug (IND) applications submitted between 2012 and 2013 show that ADCs with the same type of linker/payload generally share very similar toxicity profiles, DLTs and maximum tolerated doses (MTDs), regardless of the target antigen and the degree of antigen expression in healthy tissues.

Lipophilic payloads are highly permeable to the plasma membrane, thereby allowing the released payload to efficiently enter non-target cells (e.g., via membrane diffusion) and potentially causing unwanted cytotoxicity.

Accordingly, in order to control the bystander effect and/or ADME profile of the active camptothecin derivative such that it is quickly or slowly eliminated through lymphatic drainage by providing a desired degree of hydrophobicity and/or controlling aggregation, in consideration of this, the camptothecin-based drug that degrades DDX5 protein in the immunoconjugate of the present invention may be selected from an active camptothecin derivative represented by Chemical Formula 1, Chemical Formula 2 or Chemical Formula 1-1 below, which is designed to bind to DDX5 protein and E3 ligase, and the anticancer formulation containing the immunoconjugate of the present invention, a dose and a dosage thereof may be designed in various ways.

As a method to reduce non-selective uptake of ADC, lowering DAR is already a wellestablished method, but it is also important for the immunoconjugate of the present invention, which uses a drug with relatively low cytotoxicity, such as a camptothecin series payload and the like, to maintain a high DAR of more than 4 to secure sufficient efficacy.

In MDA-MB-468, which is a Her2 negative cell line corresponding to "non-selective uptake" in ADC, the IC50 result was 2.5 times lower for Trastuzumab-25-6 (DAR 6) (IC50= 97.61 nM) than for Trastuzumab-25-6 (DAR 4) (IC50= 240.7 nM).

This may increase the therapeutic index in terms of off-target toxicity (by reducing payload delivery to non-target healthy tissues) by controlling the DAR of the camptothecin-based payload. In other words, even if a higher dose of ADC is administered in the case of DAR = 4 than in the case of DAR = 6, the by-stander cell-killing effect, which is a type of "non-selective uptake" of the payload, may be maintained to be low.

**[Clinical Results and Side Effects of ADCs with Camptothecin Payloads]**

Trastuzumab Deruxtecan (Enhertu) is a humanized anti-HER2 antibody linked to deruxtecan, which is a camptothecin derivative, through a stable linker. In the first phase 1 dose-escalation study, 22 patients with HER-2-positive advanced or metastatic breast cancer, gastric cancer or other HER-2-expressing solid tumors were treated with trastuzumab deruxtecan at 0.8 mg/kg to 8.0 mg/kg once every 3 weeks. No dose-limiting toxicities were observed, and the MTD was not reached. The target drug exposure was achieved at a dose of 6.4 mg/kg, which was selected as the recommended phase 2 dose.

The DESTINY-Breast01 trial, which is a pivotal single-arm phase 2 clinical trial, consisted of two parts. In the first part, patients with advanced/metastatic breast cancer who were previously treated with two or more anti-HER2 therapies were randomly assigned to be administered with trastuzumab deruxtecan at 5.4 mg/kg (n = 50), 6.4 mg/kg (n = 48) or 7.4 mg/kg (n = 21) once every 3 weeks. In the second part, 134 patients were administered a dose of 5.4 mg/kg based on efficacy and toxicity data obtained in the first part 1. Among 184 patients treated with trastuzumab deruxtecan at 5.4 mg/kg, the most common adverse reactions of any grade (≥20%) were nausea (77.5%), fatigue (49.8%), hair loss (49.8%)., vomiting (44.3%), neutropenia (40.3%), constipation (37.5%), anemia (33.6%), decreased appetite (33.2%), diarrhea (29.2%), leukopenia (26.9%) and thrombocytopenia (24.9%). Grade 3 or higher adverse reactions occurred in 57.1% of patients, and neutropenia (20.7%), anemia (8.7%), nausea (7.6%), leukopenia (6.5%), lymphopenia (6.5%) and fatigue (6.0%).) appeared most commonly. Discontinuation of medication, dosage reduction and treatment discontinuation due to adverse reactions occurred in 35.3%, 23.4% and 15.2% of patients, respectively, with pneumonia (11 patients) and

interstitial lung disease (5 patients) being the most common reasons. Black box warnings for patients who were treated with trastuzumab deruxtecan include interstitial lung disease (ILD) and pneumonia. Treatment-related interstitial lung disease and fatal outcomes occurred in 9% and 2.6% of patients who were treated with trastuzumab deruxtecan, respectively. As with other HER-2 targeting ADCs, patients who were treated with trastuzumab deruxtecan also had an increased risk of embryo-fetal toxicity and left ventricular dysfunction.

Meanwhile, Sacituzumab Govitecan (Trodelvy) is a humanized anti-TOP-2 IgG linked to the active metabolite of irinotecan (SN-38) through a pH-sensitive linker. In a first-in-human dose-escalation phase 1/2 clinical trial 1, 25 patients with various metastatic solid tumors were administered with Sacituzumab Govitecan at doses ranging from 8 mg/kg to 18 mg/kg on days 1 and 8 of a 21-day cycle. The MTD for the first cycle was determined to be 12 mg/kg, with neutropenia appearing as the dose-limiting toxicity. However, this dose level was too toxic for subsequent cycles, and thus, doses of 8 mg/kg and 10 mg/kg were selected for phase 2 clinical trials. In this phase 2 clinical trial, Sacituzumab Govitecan was administered at a dose of 8 mg/kg (n = 81) or 10 mg/kg (n = 97) to patients with a variety of metastatic epithelial cancers who had previously received multiple treatments. The most common adverse reactions of any grade (≥25%) reported in the 8 mg/kg and 10 mg/kg cohorts were nausea (59% vs. 63%), diarrhea (53% vs. 62%), neutropenia (42% vs. 58%), fatigue (61% vs. 52%), vomiting (36% vs. 43%), anemia (38% vs. 42%), hair loss (46% vs. 37%) and constipation (33% vs. 37%), respectively. The most common adverse reactions grade 3 or higher (≥10%) reported in the 8 mg/kg and 10 mg/kg administration groups were neutropenia (30% vs. 36%), anemia (13% vs. 12%), diarrhea (4% vs. 10%) and leukopenia (6% vs. 12%). Dose reductions occurred in 19% and 28% of patients in the 8 mg/kg and 10 mg/kg cohorts, respectively. Neutropenia was the most common adverse reaction leading to dose delay or reduction. Significantly more patients in the 10 mg/kg cohort experienced grade 3 or higher neutropenia after the first dose than in the 8 mg/kg cohort (47% vs. 21%).

Black box warnings for severe or life-threatening neutropenia and severe diarrhea have been added to the Sacituzumab Govitecan label. These side effects are likely mediated by released ("free") SN-38, and this is because it is associated with the same toxicity as ionotecan, which is the SN-38 prodrug. Among all patients treated with Sacituzumab Govitecan, any grade and grade 3 or higher neutropenia occurred in 61% and 47%, respectively. Febrile neutropenia occurred in 7% of patients. All grade and grade 3 or higher diarrhea occurred in 65% and 12% of all patients treated with Sacituzumab Govitecan, respectively. Neutropenic colitis occurred in 0.5% of patients.

**[Camptothecin-based Drug (A) That Degrades DDX5 protein and Anticancer Mechanism Thereof]**

Targeted therapy has a different treatment mechanism from the cytotoxic anticancer drugs that have been used previously. Cytotoxic anticancer drugs cannot avoid toxicity to normal cells because they act on the DNA or microtubules of rapidly dividing cells, but targeted therapies selectively attack only cancer cells using molecular targets. Molecular targets of targeted therapies include angiogenesis, apoptosis, cell signaling pathways and cell cycle regulators, and targeted therapies targeting various targets are being developed. Since the therapeutic effect of targeted therapy is usually to inhibit cell proliferation, it is generally used alone for a long period of time or in combination with other cytotoxic anticancer drugs.

Antibodies are considered as important components in determining the effectiveness of ADCs, but it is cytotoxic drugs that carry out tumor cell killing. Cytotoxic drugs are small molecule drugs that induce the killing of tumor cells. Cytotoxic drugs, which correspond to the payload in ADC composition, play an important role in killing cancer cells and are a very important component that exhibits drug efficacy.

Therefore, more powerful cytotoxic drugs were introduced into ADCs, but most of them were too toxic and even affected normal cells due to the bystander effect. Additionally, because the drug must be conjugated with minimal effect on the antibody, the amount of payload that can be transported is limited. This indicates that cytotoxic drugs to be applied to ADC must be able to kill most tumor cells at low concentrations (nM or pM) and must be controlled to exhibit therapeutic effects. Therefore, it can be seen that the cytotoxic drugs that can be applied as payload are very diverse, but above all, they must have low immunogenicity and have stable properties during circulation in the body. This is because unstable cytotoxic payload may lead to drug deformation during conjugation or storage.

Anticancer drugs used as payloads in immunoconjugates such as ADC inhibit cancerpromoting factors (oncoproteins) related to promoting cell growth, promoting cell survival and promoting cell migration, or it may be to restore/reactivate/stabilize tumor suppressor factors (e.g., p53) involved in cell growth inhibition, cell death promotion and cell motility inhibition.

Apoptosis plays a very important role in maintaining tissue homeostasis and shape, regulating cell number, removing damaged or abnormal cells, and as a defense mechanism against infection, and living organisms maintain their lives normally by precisely regulating the processes of cell growth, differentiation and death. The type of cell death can be classified into necrosis and programmed cell death (apoptosis), of which apoptosis is accompanied by the expression of various genes and the activation of proteins that are expression products, and it is explained as a complex process that induces cell death. Apoptosis is an intracellular physiological and active suicide mechanism that plays an essential role in the development and differentiation of multicellular organisms and in maintaining homeostasis. Apoptosis occurs in various types of cells due to many factors, and unlike cell necrosis, there is no release of lysosome enzymes, apoptotic

bodies are found, and cell shrinkage, nuclear condensation and characteristic ladder-shaped DNA fragmentation are observed.

As shown in FIGS. 7 and 8 showing the degree of inhibition of anti-apoptotic proteins, the camptothecin-based drug that binds to and degrades oncoprotein DDX5 may control apoptosis-related proteins selected from the group consisting of c-Myc, surviving, cIAP2, Bcl-2, XIAP and mutant Kras as molecular targets.

Therefore, the [camptothecin-based drug that binds to and degrades oncoprotein DDX5] may kill most tumor cells at low concentrations (nM or pM) as payloads for application to ADCs.

In consideration of the above, the payload in the immunoconjugate of the present invention is characterized by using a camptothecin-based drug that binds to DDX5 protein and E3 ligase and degrades DDX5 protein, and it may be selected from active camptothecin derivatives represented by Chemical Formula 1, Chemical Formula 2 or Chemical Formula 1-1 that are designed to bind to DDX5 protein and E3 ligase.

The camptothecin-based drug (A), which degrades the DDX5 protein that is the payload of the immunoconjugate according to the present invention, is a hydrophobic small molecule that can penetrate the cell membrane, and thus, it can penetrate deep into the cancer tissue and accumulate at high concentrations, and it can penetrate the cell membrane, exert cytotoxicity inside the cell, kill the cell, and then be released, and then move into the surrounding cells by penetrating the cell membrane and acting on them.

The camptothecin-based drug (A), which degrades the DDX5 protein that is the payload of the immunoconjugate according to the present invention, may be selected from active camptothecin derivatives represented by Chemical Formula 1, Chemical Formula 2 or Chemical Formula 1-1 below that are designed to bind to DDX5 protein and E3 ligase, in order to control the bystander effect and/or ADME profile of the active camptothecin derivatives by providing a desired degree of hydrophobicity and/or controlling aggregation such that they are removed rapidly or slowly via lymphatic drainage, and the anticancer formulation containing the immunoconjugate of the present invention, a dose and dosage thereof may be designed in various ways.

Anti-apoptotic proteins that are the main cause of anticancer drug resistance include Survivin, cIAP2, XIAP and the like (FIG. 6).

Unlike SN-38, non-limiting examples of camptothecin-based cytotoxic drugs that inhibit Bcl family members, such as Survivin which is a resistance protein involved in the resistance mechanism, or bind to and degrade oncoprotein DDX5 (p68) that controls c-Myc, survivin and mutant Kras include FL118 (Chemical Formula 1-1), active camptothecin derivatives represented by Chemical Formula 1 or Chemical Formula 2 (Exatecan, Dxd) (FIG. 7).

Inducing cell death by targeting the apoptosis pathway is also a good cancer treatment method. In addition, since camptothecin-based cytotoxic drugs that inhibit type I topoisomerase are DNA modifying agents, they can kill cells regardless of the cell cycle. Therefore, the camptothecin-based cytotoxic drug that degrades the DDX5 protein is designed to bind to the DDX5 protein and E3 ligase, and not only can it kill cells through its mechanism of action (MoA) that degrades the oncoprotein DDX5 along with its ability to inhibit type 1 topoisomerase, it also has an excellent cell killing effect regardless of the cell cycle.

FL118 (Chemical Formula 1-1) may act as a molecular glue degrader that degrades DDX5 by directly attaching DDX5 to a ubiquitination regulator.

[Chemical Formula 1-1]

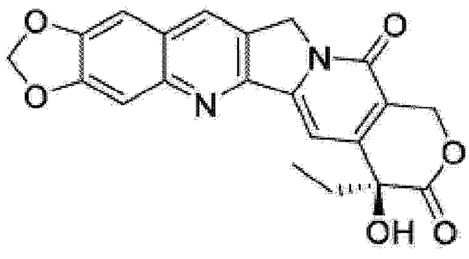

FL118

FL118, which acts as a molecular glue, directly binds to the tumor protein DDX5, which is a multifunctional master regulator, through the proteasome degradation pathway without reducing DDX5 mRNA, and has the function of dephosphorylating and degrading the same, and DDX5 silencing indicates that DDX5 is a master regulator that regulates the expressions of several oncogenic proteins, including survivin, Mcl-1, XIAP, cIAP2, c-Myc and mutant Kras.

In addition, it is possible to avoid resistance by fundamentally blocking the overexpression of anti-apoptotic protein, which is a common resistance development mechanism in most cancers; and biomarkers (DDX5, K-ras, p53) and companion diagnostic techniques that can predict anticancer response in patients have already been established, and by securing customized biomarkers, personalized treatment is possible through companion diagnosis, and it shows particularly strong efficacy in p53/K-ras mutant cancer cells with a poor prognosis.

FL118 indirectly controls DDX5 downstream targets to inhibit cancer initiation, development, metastasis, recurrence and treatment resistance with high potency as demonstrated in studies using human colorectal/pancreatic ductal adenocarcinoma cells and tumor models.

Genetic manipulation of DDX5 in PDAC cells affects tumor growth. PDAC cells with DDX5 KO are resistant to FL118 treatment. Studies in human tumor animal models showed that FL118 showed high efficacy in eliminating human PDAC and CRC tumors with high DDX5 expression, whereas FL118 was less effective in PDAC and CRC tumors with low DDX5 expression.

DDX5 protein is a direct target of the FL118 drug and may serve as a biomarker to predict PDAC and CRC tumor sensitivity to FL118.

Meanwhile, the FL118 drug has a Top1 inhibitory effect that is equivalent to or higher than that of SN-38 in cancer cells, and is 5 to 20 times more potent than SN-38 in various cancer cell lines, that is, it exhibits cytotoxicity with a low IC50 value. In addition, the evaluation results of 140 cell lines originating from various cancer types showed very strong anticancer efficacy with an IC50 of <100nM against the majority of cancer cells. The FL118 drug has secured excellent safety through GLP-toxicity tests in rats and beagle dogs, and has shown superior efficacy compared to SN-38 in various cancer cell line Xenograft models. Meanwhile, camptothecin-based anticancer drugs show excellent anticancer responses initially when used in patients, but strong resistance to these drugs appears through Epigenetic Silencing of the Top1 gene and Top2 Dependence of cancer cells. On the other hand, FL118 drug showed strong efficacy even in the Xenograft model of cancer cell lines in which Top1 is not expressed through Epigenetic Silencing or Knock-out.

In addition, the FL118 drug directly targets type I topoisomerase, which is a wellestablished anticancer target, and simultaneously inhibits Bcl family members such as Survivin, which is a resistance protein involved in resistance mechanisms, and it is a triple-target anticancer drug that inhibits the action of efflux pumps.

Specifically, camptothecin-type anticancer drugs such as SN-38 show resistance in the way that the drug is released out of the cell due to overexpression of the ABCG2 Transporter. However, since the FL118 drug is not affected by the ABCG2 Transporter, it is possible to overcome this resistance. The FL118 drug can block the development of resistance by strongly inhibiting the expression of anti-apoptotic proteins (Survivin, cIAP2, XIAP, etc.), which is another main cause of anticancer drug resistance, at low concentrations.

Therefore, the FL118 drug is not expelled out of the cell by ABCG2, which is an efflux pump, and can block resistance caused by various anti-apoptotic proteins. As a result, the FL118 drug may overcome the various resistance mechanisms of SN-38/Exatecan.

When administered *in vivo* in the same amount as SN-38, the FL118 drug showed stronger tumor regression efficacy than SN-38 in colon cancer, head and neck cancer, and pancreatic cancer.

The FL118 drug possessed strong anticancer efficacy even when FL118 was administered after inducing SN-38 resistance in tumor xenograft.

Moreover, the FL118 drug has an optimal PK/safety profile for targeted drug delivery (e.g., Carrier-drug Conjugate) application. When the FL118 drug is administered systemically alone, it is rapidly metabolized and excreted from the blood and only shows a low concentration, but it accumulates quickly in cancer tissue immediately after the administration and maintains a high concentration for a long period of time. For example, when applying ADC, maximum selectivity between tumor tissue and normal tissue is ensured.

Exatecan is a camptothecin derivative and an anti-tumor small molecule compound that inhibits type I topoisomerase. Exatecan is a substance that has been confirmed to have cellular cytotoxicity that is 5 to 10 times more powerful than SN-38.

Exatecan is different from irinotecan and does not require activation by enzymes. In addition, it has stronger type I topoisomerase inhibitory activity than SN-38, which is the main medicinal product of irinotecan, and topotecan, which is used in clinical trials, and has stronger cytotoxic activity against various cancer cells *in vitro.* In particular, it was effective against cancer cells that are resistant to SN-38 and other drugs due to the expression of P-glycoprotein. In addition, it showed a strong anti-tumor effect in a human tumor subcutaneous transplant model in mice, and clinical trials were conducted.

Dxd (Exatecan derivative for ADC) is a potent DNA topoisomerase I inhibitor with an IC50 of 0.31 $\mu$M, which is used as a conjugate drug for HER2 targeting ADC (DS-8201a).

Surprisingly, as will be described below, based on the structure of the FL118 drug, which is differentiated from the SN38 drug, while the inventors of the present invention were designing camptothecin derivatives having a structure that exerts a dual mechanism of action (dual MoA) in terms of type I topoisomerase inhibition and DDX5 degradation (FIG. 1), which are the advantages of the FL118 drug, it was discovered that exatecan or Dxd also exerts a mechanism of action

(MoA) to degrade DDX5 protein (FIGS. 7 to 10).

Based on this, various camptothecin-based drugs of Chemical Formula 1 or Chemical Formula 2 were designed according to the present invention, in order to demonstrate various advantages as anticancer drugs exemplified by FL118 drug, and it is another feature of using the same as payloads of the immunoconjugate of the present invention.

[Chemical Formula 1]

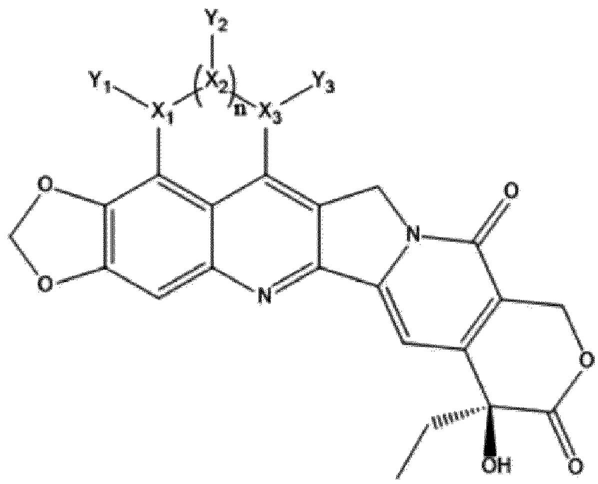

[Chemical Formula 2]

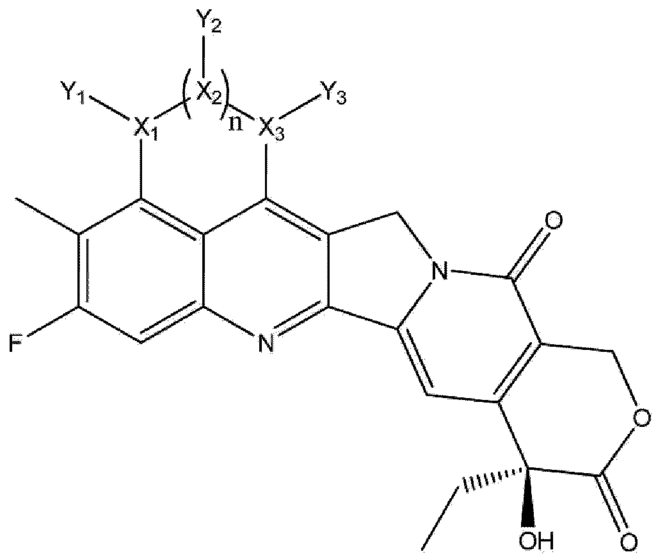

$X_1$ and $X_3$ may each independently be carbon, oxygen, nitrogen or sulfur, and $X_1$ and $X_3$ may be the same or different,
$X_2$ may be carbon, oxygen, nitrogen, sulfur, single bond or double bond,
$X_1$, $(X_2)_n$ and $X_3$ may form a pentagonal, hexagonal or heptagonal ring (values of n = 0 to 2), and
$Y_1$, $Y_2$ and $Y_3$ may each independently be hydrogen or a functional group including oxygen, nitrogen, phosphorus or sulfur.

The camptothecin-based drug of Chemical Formula 1 or Chemical Formula 2 used as a payload in the present invention is characterized in that $R_1$ and $R_2$ (Group A) on ring A in General Formula 1 are designed to be identical/-structurally extremely similar to the FL118 compound or Exadecane/dxd (FIG. 1) such that it exerts an anti-cancer mechanism that degrades the intracellular oncoprotein DDX5 (FIGS. 7 to 10).

[General Formula 1]

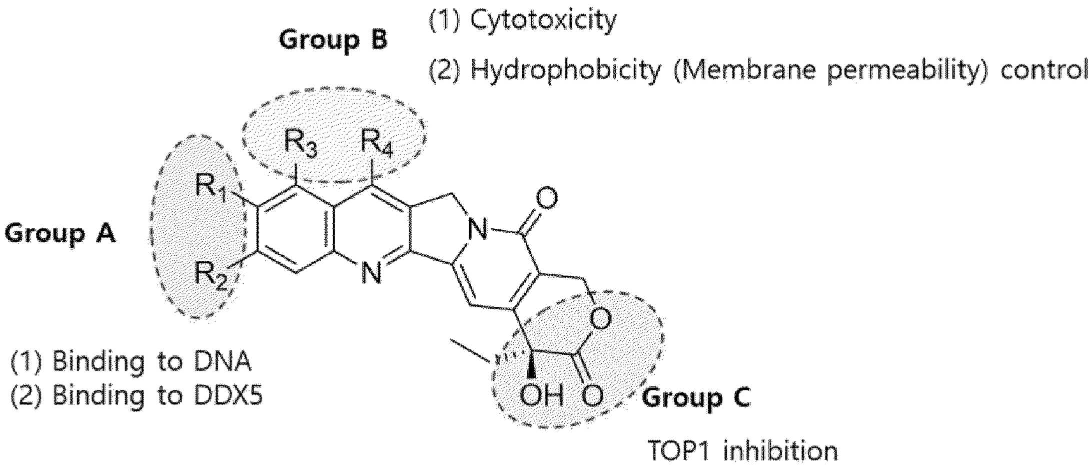


The synthetic design concept of an active camptothecin derivative with a type I topoisomerase inhibitory ability and a dual mechanism of action (MoA) to degrade the oncoprotein DDX5 maintains the structure of Group C, which is a type I topoisomerase inhibition region, based on the Structure Activity Relationship (SAR), and also maintains the same structure as FL118 or the same structure as exadecane and dxd, which is a binding site for DDX5 degradation, but improves the physicochemical properties of a drug by controlling the structure (R3 and R4) of Group B in General Formula 1 as in Chemical Formula 1 or Chemical Formula 2 to solve the aggregation problem of ADC using the same as a payload, and it is possible to design a structure that can precisely control the bystander effect by controlling the cytotoxicity of the payload (active camptothecin drug) released from the ADC and/or the tumor tissue penetration and/or cell membrane permeability of the drug.

In Chemical Formula 1, the Group C site binds to type I topoisomerase, and the Group A site binds to DNA and stabilizes the covalent bond of the topoisomerase-DNA complex, thereby preventing the cleaved DNA fragments from being reconnected. Alternatively, in Chemical Formula 1, the Group A site may bind to DDX5, and the Group C site may bind to E3 ligase to induce DDX5 degradation (see PCT/KR2023/005380, which is incorporated herein in its entirety).

The present invention preferably designs a camptothecin-based drug of Chemical Formula 1 or Chemical Formula 2 such that the cell membrane permeability is controlled as desired through modification of $R_3$ and/or $R_4$ in General Formula 1, thereby exhibiting an appropriate bystander effect in tumor tissue.

Therefore, considering not only various side effects but also problems such as reduced therapeutic coefficient, another key feature of the present invention is that it allows expanding the selection range of payloads of ADCs that exhibit appropriate anticancer efficacy to a diverse group of candidates, including active camptothecin derivatives of Chemical Formula 1 or Chemical Formula 2, which are designed to bind to DDX5 protein and E3 ligase as molecular glue degraders.

According to the present invention, the camptothecin-based drug of Chemical Formula 1 or Chemical Formula 2, which is designed to bind to DDX5 protein and/or E3 ligase, may kill target cells expressing DDX5 protein through a molecular glue degrader mechanism (MoA).

Target cells may be cancer cells or senescent cells. Senescent cells also include cells that do not perform organ-specific functions.

Preferably, the camptothecin-based drug of Chemical Formula 1 or Chemical Formula 2, which is designed to bind to the DDX5 protein and/or E3 ligase according to the present invention, may have the ability to inhibit type I topoisomerase and also have multiple mechanisms of action (MoA) that degrade the oncoprotein DDX5.

According to the present invention, camptothecin derivatives represented by Chemical Formula 1, such as PBX-7011 and PBX-7012, have a pentacyclic structure with a lactone in the E-ring that is essential for cytotoxicity, like camptothecin shown in FIG. 1, and it is designed to maintain the lactone group and alpha hydroxyl group located at carbon 20 of the E loop, which are important for the stability of the type I topoisomerase-DNA byproduct. In addition, among the structural features of the Exatecan-based drugs in General Formula 1, that is, (1) while maintaining the structural features of R1 and R2 (CH3-C=C-F) which are similar in orientation to the FL118 drug (-OCH2O- (methylenedioxo) pentagonal ring) that binds to the DDX5 protein, (2) through R3 and R4, by $\pi$-$\pi$ stacking of aromatic rings formed by the A- and B-rings, compared to SN-38 in which aggregation is induced, for example, various orientations of successive carbon-carbon single bonds of hexagonal or heptameric rings extended from the A- and B-rings form a dynamic equilibrium such that the stacking of aromatic rings formed by the A- and B-rings may be weakened or suppressed.

Additionally, the PBX-7011 compound allows rotation of the molecular bond with respect to the carbon-carbon single bond in the hexagonal ring extending from the A- and B-rings such that $-NH_2$, which has a large degree of freedom,

may be exposed to water ($H_2O$) to acquire a (+) charge or form hydrogen bonds with water, thereby increasing water dispersibility.

Additionally, the PBX-7014 compound has a lactic acid-type functional group $CH_2(OH)CONH$- of -NH2, which has a large degree of freedom in molecular bond rotation about the carbon-carbon single bond in the hexagonal ring extended from the A- and B-rings, and when exposed to water ($H_2O$), it rotates like a propeller to form hydrogen bonds with water, thereby increasing water dispersibility.

Additionally, the PBX-7016 compound introduces a methyl group, which is a metabolically unstable functional group, into the PBX-7014 compound to reduce the lifespan of the drug. Since drugs that are extremely stable and therefore metabolized very slowly may have toxicity and serious side effects due to accumulation, it is necessary to secure an appropriate retention time.

Meanwhile, the DXd payload used in Enhertu was originally made from the compound Exatecan, which is almost unaffected by ABCG2, but due to the presence of a glycolic acid (alpha-hydroxy acetic acid) functional group used to convert Exatecan to DXd, it was strongly influenced by ABCG2. However, the glycolic acid functional group plays a very important role in Enhertu's excellent safety/efficacy profile, and if it is removed, in addition to the difficulties in producing ADCs, it also brings with it the problem of poor performance of ADCs in animal models and clinical trials (emergence of safety issues or reduction in efficacy). As such, there is a very high need for new camptothecin derivatives that can be easily used in ADC production and are not affected by ABCG2. The PBX-7024 compound is a compound derived from the PBX-7011 compound and is a new camptothecin compound that is not affected by ABCG2 and can be easily used in ADC production.

In order to confirm the anticancer efficacy of PBX-7024, efficacy evaluation was performed in FaDu, which is a cancer cell that does not express ABCG2, and A549, which is a cancer cell that overexpresses ABCG2. In A549, which is a cancer cell line overexpressing ABCG2, as shown in FIG. 9, it can be confirmed that camptothecin compounds including DXd showed increased IC50 values, while PBX-7016 and PBX-7024 still maintained strong efficacy.

As shown in FIG. 9, by treating FaDu, which is a Her2-low/mid cancer cell line that does not express ABCG2, and A549, which is a cancer cell line that overexpresses ABCG2, with various camptothecin-based drugs at various concentrations, the degree of intracellular DDX5 protein degradation and the resulting inhibitory activity on the expression of cancer-associated survival genes, survivin, Mcl-1, XIAP and cIAP2, could be confirmed, and cell membrane permeability could be indirectly compared and confirmed.

**[Anticancer Mechanism as a Molecular Glue Degrader That Binds to DDX5]**

The ubiquitin-proteasome system (UPS) is an important pathway for the degradation of intracellular proteins that regulates a wide range of cellular processes. Ubiquitin is a small protein that is covalently attached to lysine residues of substrate proteins by a series of enzymatic reactions involving ubiquitin activating enzymes (E1s), ubiquitin conjugating enzymes (E2s) and ubiquitin ligases (E3s). This process is called ubiquitination and is an important mechanism that regulates protein degradation, signal transduction, and trafficking.

Ubiquitin ligase is responsible for substrate specificity in the ubiquitination pathway.

In this regard, the camptothecin-based drug of Chemical Formula 1 or Chemical Formula 2 according to the present invention is designed to bind to DDX5 protein and E3 ligase.

The transformation of normal cells into cancerous cells occurs through deregulation of different metabolic pathways involving a complex network of protein-protein interactions. Cellular enzymes and DDX5 play important roles in maintaining normal cellular metabolism, but when deregulated, they may accelerate tumor transformation. DDX5 interacts with hundreds of other cellular proteins, and depending on the specific pathways involved, both proteins may act as either tumor suppressors or oncogenes.

DDX5 (also known as p68) is a multifunctional master regulator that acts in the following mechanisms: (1) a biological process that co-activates the transcription of many oncogenes through direct interactions with various transcription factors (e.g., c-Myc) at oncogenic gene promoters, (2) a biological process that regulates miRNA and pre-RNA splicing (e.g., U1, U2, U3, ... snRNP), and (3) ribosome biogenesis (e.g., 32S rRNA, pre-ribosome).

The camptothecin-based drug of Chemical Formula 1 or Chemical Formula 2 according to the present invention binds to DDX5 protein without reducing DDX5 mRNA and functionally degrades the same through dephosphorylation and proteasome degradation pathways, and this suggests that camptothecin drugs of Chemical Formula 1 or Chemical Formula 2 may bind to both DDX5 and ubiquitin-involved protein stability/degradation regulators, and it acts as "molecular adhesive degraders."

DDX5 downstream protein targets are all known to be involved in cancer initiation, development, metastasis, recurrence and treatment resistance. Therefore, if the DDX5 downstream target is indirectly blocked through the degradation of DDX5 protein by the camptothecin-based drug of Chemical Formula 1 or Chemical Formula 2 according to the present invention, the camptothecin-based drug of Chemical Formula 1 or Chemical Formula 2 may exhibit high antitumor efficacy.

DDX5 (p68) is a well-known multifunctional DEAD-box RNA helicase and transcription cofactor. Therefore, when cancer occurs due to deregulation of the transcription factor as a result of the physiological state of DDX5, the cancer disease may be treated by selectively degrading DDX5 (p68), which is a transcription cofactor. Likewise, cancer disease may be prevented by selectively degrading DDX5 (p68), which is a transcription cofactor.

Since the camptothecin-based drug of Chemical Formula 1 or Chemical Formula 2 according to the present invention has the DDX5 protein as a drug target, it may avoid drug resistance, resistance to targeted therapy and/or resistance during treatment. In addition, the transcriptional induction of anti-apoptotic genes may be blocked (off) by the camptothecin-based drug of Chemical Formula 1 or Chemical Formula 2. Furthermore, the DDX5 protein, which is a transcription cofactor, is degraded by the camptothecin-based drug of Chemical Formula 1 or Chemical Formula 2, thereby maintaining or improving the sensitivity of cancer cells to chemotherapy or radiotherapy.

**[Advantages of the Drug Modality Called Molecular Glue Degrader]**

Proteins within the cells of the body are naturally decomposed within a few hours to a few days after performing their function. All cells in the body have a purification system called the Ubiquitin proteasome system (UPS) that degrades proteins. In this process, ubiquitin acts as a marker to indicate which proteins need to be decomposed, and the proteasome acts as a shredder that recognizes ubiquitin tags and destroys the corresponding proteins. In other words, several substances called ubiquitin are attached like a mark next to a protein that has completed its function, and a substance called proteasome selects only the proteins with this mark and breaks them down like a shredder. E3 ligase is an enzyme that initiates the protein degradation system in the body and is responsible for substrate specificity in the ubiquitination pathway.

Molecular glue degrader or molecular glue is a compound that functions as an adhesive to attach a target protein to a specific enzyme (E3 ligase) in our body. One of the advantages of molecular glue is that it acts as a catalyst, and after decomposing a target protein, and it is separated again to degrade another target protein.

When the E3 ligase enzyme attaches to a tumor protein through molecular glue, the tumor protein is degraded, and other tumor proteins are successively degraded until the target tumor protein disappears, thereby preventing cancer cell proliferation. Therefore, molecular adhesives for tumor proteins overcome drug resistance, which is a problem with targeted anticancer drugs, and have high therapeutic effects even at low administration doses.

The camptothecin-based drug of Chemical Formula 1 or Chemical Formula 2 used as a payload according to the present invention is a molecular glue degrader that binds to DDX5 protein and E3 ligase, that is, it is a molecular glue degrader that activates the degradation of the tumor protein DDX5 or its phosphorylated DDX5 protein (p-DDX5) (FIG. 3, FIG. 26, FIG. 27).

A molecular glue degrader is not only a ligand (warhead) that binds to a target protein, but may also act as an E3 ligase ligand (binder).

Therefore, since the camptothecin-based drug of Chemical Formula 1 or Chemical Formula 2 according to the present invention is a molecular glue degrader that activates the degradation of the tumor protein DDX5, it may be used as a ligand that targets DDX5 protein or a ligand that binds to DDX5 protein.

Unlike kinase inhibitors, molecular glue degraders are 'proximity-driven' and their ability to induce degradation is 'event-driven', depending on the formation of a transient 'target proteinmolecular glue-E3 ligase' triple complex. After degradation occurs, the separated molecular glue forms an additional triple complex with the target protein, thereby allowing multiple degradation processes to proceed until the target protein disappears.

Most proteins that are drug targets develop resistance that evolves by adapting to the drug. However, the molecular glue, which is a low-molecular-weight compound that acts as an adhesive that binds tumor proteins and E3 ligase together, is a suitable modality for cancer treatment because it is highly resistant to resistance.

Each time a genetic mutation occurs, the shape of a drug target protein changes slightly. It would be nice to block the activity of all variants with one drug, but this is not possible due to selectivity issues. In order to block the activity of all variants with one drug, the drug target protein is degraded and completely removed.

Therefore, the camptothecin-based drug of Chemical Formula 1 or Chemical Formula 2 according to the present invention is a drug that can accurately bind to the DDX5 tumor protein, and through a molecular glue approach that selectively degrades the protein, it may avoid the problem of resistance to targeted therapeutic agents.

The camptothecin-based drug of Chemical Formula 1 or Chemical Formula 2 according to the present invention has a binding strength to the target protein, DDX5 tumor protein, depending on its physicochemical properties, and is preferably an irreversible drug that binds so strongly that it cannot return to its previous state.

Unlike existing SN38 drugs, the camptothecin-based drug of Chemical Formula 1 or Chemical Formula 2 according to the present invention has a high affinity for DDX5 and does not fall off easily, and degrades DDX5 through a molecular glue function, thereby irreversibly inhibiting signal transduction of cancer cells related to DDX5. In addition, the drug's cell membrane permeability may be adjusted as desired depending on its physicochemical properties, thereby exerting a bystander effect or controlling the degree of its effect, and thus, not only is it advantageous in the treatment of

heterogeneous tumors due to its high peripheral cell killing effect, but it may also suppress long-term cancer progression, reduce the risk of resistance development, and increase the treatment response rate.

The camptothecin-based drug of Chemical Formula 1 or Chemical Formula 2 according to the present invention may bind to DDX5, which acts as an oncoprotein within cells, and induce cell death through DDX5 protein degradation (FIG. 9 and FIG. 10).

In cancer treatment, intrinsic drug resistance may be caused by abnormally expressed transcription factors, which are critical regulators of cell proliferation and apoptosis. Therefore, the camptothecin-based drug of Chemical Formula 1 or Chemical Formula 2 according to the present invention degrades DDX5 protein through its mechanism of action as a molecular glue degrader that binds to DDX5, which is an electron cofactor and an oncoprotein, and as a result, this may induce cell death. In this case, DDX5 protein degradation may downregulate the transcription of anti-apoptotic genes (FIGS. 26 and 27). Therefore, unlike other targeted therapies, drug resistance caused by abnormally expressed cell proliferation and/or cell deathrelated transcription factors is less likely to occur, and/or it may suppress acquired drug resistance induced through the activation of dysregulated transcription factors during chemotherapy and/or radiotherapy.

In other words, the anticancer mechanism of the camptothecin-based drug of Chemical Formula 1 or Chemical Formula 2 according to the present invention may bypass the molecular mechanism of cancer treatment resistance, and thus, it may be free from the problem of drug resistance. Since the therapeutic effect is lower when resistance develops, the camptothecin-based drug of Chemical Formula 1 or Chemical Formula 2 according to the present invention may be preferred as a standard or primary treatment after cancer diagnosis.

In short, according to the present invention, the camptothecin-based drug of Chemical Formula 1 or Chemical Formula 2 used as a payload may become a targeted anticancer agent that acts on the DDX5 protein, which plays an important role in the growth, survival (cell survivor), proliferation, metastasis and/or metabolism of cancer cells.

**[ADME Profile of Antibody-Drug Conjugate (ADC)]**

The *in vivo* efficacy and *in vivo* side effects of anticancer drugs may be affected by the absorption, distribution, metabolism and excretion (ADME) characteristics of ADC and the payload released therefrom. A drug's ADME profile may affect its ability to reach and act on cancer cells. In other words, the ADME characteristics of the ADC and the payload released therefrom may have a significant impact on *in vivo* efficacy and *in vivo* side effects.

The ability of ADC and the payload released therefrom to penetrate tumor tissue may also be affected by factors such as the tumor microenvironment, including blood flow and cell density.

Therefore, understanding the ADME characteristics of ADCs and the payloads released therefrom and optimizing drug/drug modality selection and dosage/regimen may improve the therapeutic index of these ADCs and the payloads released therefrom, thereby leading to better outcomes for cancer patients.

Antibodies (Abs) and antibody-drug conjugates (ADCs) have different life times and ADME profiles due to their different structures and mechanisms of action.

Antibodies are large proteins naturally produced by the immune system in response to foreign substances (antigens). Due to their size and complex structure, they have a long circulating half-life (several weeks to months) and may protect from degradation and elimination. Antibodies are distributed throughout the body, including tissues, and may interact with target antigens with high specificity and affinity. Antibodies are primarily eliminated by catabolism in the reticuloendothelial system (RES), which includes the liver and spleen, as well as the kidneys and other organs.

ADCs consist of an antibody (usually a monoclonal antibody) conjugated to a cytotoxic drug molecule. The antibody component provides specificity and targeting to the tumor or diseased tissue, whereas the drug component (payload released from the ADC) provides cytotoxic activity to kill target cells. ADCs have shorter half-lives than antibodies, typically ranging from a few days to a week. This is because ADC is internalized into target cells, resulting in lysosomal degradation of ADC and release of drug payload. Although ADC is primarily eliminated by RES, the drug payload released from ADC may also undergo lymphatic drainage and subsequent metabolism and excretion in the liver and kidneys via the circulatory system (Example 2).

As expected from the pharmacokinetics and biodistribution of monoclonal antibodies, even in ADCs, high-affinity mAb binding to cell membrane proteins may localize a significant portion of the mAb to the target cell population, and chemical conjugation of the anticancer mAb with the payload increases the therapeutic index of the payload by increasing the selectivity with which it is delivered to cancer cells.

Antibodies are usually administered subcutaneously or intravenously and are absorbed into the bloodstream. They can be distributed throughout the body, including tissues, but are generally restricted to the extracellular space due to their large size. ADCs are also administered by injection and absorbed into the bloodstream, but after targeting to tumors or diseased tissues and, in some cases, receptor-mediated endocytosis due to the antibody component, the drug payload released from the ADC may penetrate into cells and tissues depending on its physicochemical properties (e.g., hydrophobicity, size, aggregation), and may exert a bystander effect through non-selective uptake (FIG. 26). Furthermore, the metabolism and excretion of ADCs vary depending on the specific structure and the specific drug or antibody used.

DAR, which is a drug-to-antibody ratio, is a very important feature that determines pharmacokinetic properties and biodistribution in ADC development.

ADCs with higher DAR showed higher efficacy in *in vitro* tests. However, contrary to expectations, ADCs with high DAR showed low *in vivo* efficacy, which was presumed to be because ADCs with more bound drugs had higher plasma clearance. Accordingly, the DAR of ADC formulations has been set at around 2 to 4 for some time. For this reason, the technology used to bind drugs to cysteine or lysine residues of antibodies is mainly used.

Since many of the commonly used cytotoxic drugs and linkers are hydrophobic, problems such as ADC aggregation, loss of affinity for the target antigen or increased plasma clearance occur. Hydrophilic linkers containing sulfonate or polyethylene glycol (PEG) solve the problems caused by hydrophobic linkers. The PEG linker has the advantage of being water-soluble, having low toxicity and low immunogenicity.

It has long been thought that a DAR of around 4 is optimal, but in fact, this applies to 2nd generation linkers that use MMAE or DM1 as a drug, and for 3rd generation linkers, a higher DAR is better. Many recently approved ADCs have DAR values of nearly 8, and new ADCs currently in clinical trials have DAR values ranging from 1 to 15.

Antibody-drug conjugates (ADCs) using camptothecin-based payloads are receiving great attention as a novel approach to treat various cancers, especially solid tumors. Among these ADCs, new ADCs (e.g., Enhertu) including newly synthesized camptothecin-based payloads (e.g., DXd) optimized for ADC modality (e.g., Enhertu) have recently received special attention due to their successful clinical results.

The success of these ADCs may be due in part to the fact that the new camptothecin (1) exhibits superior cell growth inhibition; (2) better safety profile than existing ADC payloads; (3) optimized bystander effect; and (4) excellent physicochemical properties that are suitable for generating high DAR ADCs. However, despite the remarkable success of ADCs utilizing camptothecin-based payloads, such as Enhertu or DS-1062a, as described above, there are still clear unmet requirements, including improving the safety profile (minimizing interstitial lung disease (ILD) and neutropenia) and developing novel ADCs with multiple MoA payloads to address cancer heterogeneity.

In order to address these unmet requirements, the present invention provides active camptothecin derivatives of Chemical Formula 1 or Chemical Formula 2 that are designed to bind to DDX5 protein and E3 ligase as molecular glue degraders, with various physicochemical properties, as candidate drugs, and furthermore, new camptothecin-based payloads (PBX series compounds) represented by Chemical Formula 1 or Chemical Formula 2 were synthesized (Preparation Examples 1 to 4) and evaluated (FIGS. 18 to 23).

The novel PBX series compound represented by Chemical Formula 1 is a derivative of FL118, which is a potent dual Top1/anti-apoptotic pathway inhibitor represented by Chemical Formula 1-1, with an excellent safety profile, and exhibits potent *ex vivo* cytotoxicity comparable to Dxd represented by Chemical Formula 2, and exhibit an excellent safety profile in preliminary toxicity studies in mice and rapid clearance in PK studies (Example 2, Tables 3 and 4). This will minimize systemic side effects upon premature linker cleavage when used as payloads of ADCs.

As shown in FIG. 24, which is the result of a conversion experiment from lactone form to carboxylate at pH 7.4 (Example 3), at pH 7.4 which is the pH of blood or extracellular fluid, it was confirmed that PBX-7014, PBX-7016 and PBX-7024 compounds, represented by Chemical Formula 1, were converted from the lactone form (active form) to the carboxylate form (inactive form) which is inactive as TOP1 detoxifiers faster and at a higher rate than other reference compounds (exatecan, DXd, SN-38, FL118).

In particular, the formation degree (63 to 86%) and speed (0.4 to 0.8%/minute) of the carboxylate form, which is inactive as a TOP1 inhibitor at pH 7.4 in the blood, varies greatly depending on the type of camptothecin derivative (FIG. 24). PBX-7014 and PBX-7016 formed more than 86% of the carboxylate form, and both converted from the lactone form to the carboxylate form relatively quickly at the level of 0.8% per minute. DXd, which is a competing drug, formed the carboxylate form at a level of 76.5%, and was converted from the lactone form to the carboxylate form at a level of 0.6% per minute.

It was confirmed that the formation rate of lactone form (measurement of carboxylate form), which is active as a TOP1 inhibitor at pH 6.0 of the microtumor environment (TME), was similar for all evaluated drugs (carboxylate form formation at the level of 20% after approximately 800 minutes) (FIG. 25).

Through this, compounds represented by Chemical Formula 1, such as PBX-7014, PBX-7016 and PBX-7024, are predicted to have relatively low toxicity, that is, high safety in normal cells by forming an inactive carboxylate form rather than an active lactone form that exhibits cytotoxicity at a relatively fast and high rate in blood or extracellular fluid (normal tissue other than tumor tissue) compared to reference compounds. However, in the microtumor environment, the PBX-7014, PBX-7016 and PBX-7024 compounds represented by Chemical Formula 1 all form a lactone form with activity at the same level as other TOP1 inhibitors, and thus, there is no reduction in efficacy.

The immuno-anticancer agent of the present invention may be designed to have excellent dose-dependent anticancer efficacy (conc. vs. effect) and/or time-dependent anticancer efficacy (effect vs. time), but in some cases, the superior anticancer efficacy over time may also result in increased side effects (chronic antigen exposure resulting in sustained T cell activation and accumulation of inhibitory signals and resulting T cell exhaustion, side effects due to the cytotoxic effect on surrounding connective tissue-derived fibroblasts, ILD, neutropenia). For example, depending on the

drug modality (small molecule vs. ADC) of the active camptothecin derivative of Chemical Formula 1 or Chemical Formula 2, it may be important to exceed a specific drug concentration (high), and in some cases, the length of time that the drug is maintained above a particular drug concentration (low) may be important, and rapid or slow removal via lymphatic drainage may be important, by providing a desired degree of hydrophobicity and/or controlling aggregation.

Therefore, considering not only various side effects but also problems such as reduced therapeutic coefficient, another key feature of the present invention is that it allows expanding the selection range of payloads of ADCs that exhibit appropriate anticancer efficacy to a diverse group of candidates, including active camptothecin derivatives of Chemical Formula 1 or Chemical Formula 2, which are designed to bind to DDX5 protein and E3 ligase as molecular glue degraders.

Despite the remarkable success of ADCs utilizing camptothecin-based payloads, such as Enhertu or DS-1062a, by providing a variety of active camptothecin derivatives represented by Chemical Formula 1 or Chemical Formula 2 that are designed to bind to DDX5 protein and E3 ligase according to the present invention as candidates for multiple MoA payloads to improve safety profile (minimize ILD and neutropenia) and address cancer heterogeneity, it is possible to select an appropriate drug among the active camptothecin derivatives represented by Chemical Formula 1 or Chemical Formula 2, and furthermore, it is possible to select an appropriate linker therefor, and apply an appropriate dose-dosage so as to precisely control the desired efficacy (e.g., anticancer, combination therapy) and side effects (e.g., pro-carcinogenic inflammation, drug resistance, ILD, neutropenia).

[ADC's Toxicity Profile, Non-Selective Uptake and Bystander Effect]

The therapeutic index of a drug is a measure of the safety and efficacy of a drug in medical treatment. It is defined as a ratio between the dose of a drug that causes therapeutic effects and the dose that causes toxicity or adverse effects. In other words, it represents a range between the therapeutic and toxic doses of a drug.

A high therapeutic index indicates a wide margin of safety where the effective dose is significantly lower than the toxic dose. This means that the drug can be administered at therapeutic levels without causing serious side effects or toxicity. Drugs with a higher therapeutic index are generally considered safer and more desirable for clinical use.

On the other hand, a low therapeutic index means a narrow margin of safety. In these cases, the effective dose and toxic dose are relatively close, and the risk of side effects or toxicity is high when using the drug. Drugs with a low therapeutic index require careful monitoring and accurate dosing to avoid harm to patients.

The therapeutic index is an important consideration in drug development because it helps determine the dosage range that can provide the desired therapeutic effect while minimizing the risk of side effects. It provides useful information when prescribing drugs and allows a practitioner to evaluate the overall benefit-to-risk ratio of the drug.

Antibody-drug conjugates (ADCs) are a rapidly growing class of anticancer therapeutics, with more than 100 ADCs in clinical research. Currently, 12 ADCS, such as gemtuzumab ozogamicin (Mylotarg), brentuximab vedotin (Adcetris), inotuzumab ozogamicin (Besponsa), trastuzumab emtansine (Kadcyla), polatuzumab vedotin (Polivy), enfortumab vedotin (Padcev), trastuzumab deruxtecan (Enhertu), sacituzumab govitecan (Trodelvy), belantamab mafodotin (Blenrep), loncastuximab tesirine (Zynlonta), tisotumab vedotin (Tivdak) and mirvetuximab soravtansine (Elahere) have been approved by the U.S. Food and Drug Administration (FDA). Furthermore, a relatively small number of payload molecules (e.g., MMAE, MMAF, DM1, DM4, calisemicin, SN38, Dxd, PBD) are used in most approved ADCs and ADCs in development.

Although some ADCs have demonstrated sufficient efficacy and safety to receive FDA approval, clinical use of all ADCs causes significant toxicity in treated patients, and many ADCs have failed during clinical development due to unacceptable toxicity profiles. This is because off-site toxicity remains a problem, limiting tolerable ADC doses below those required for substantial anticancer efficacy. Even for FDA-approved ADCs, a significant proportion of treated patients require adjuvant treatment to reduce the severity of ADC-related toxicities, and many require dose reduction, treatment delay or treatment discontinuation.

The analysis of clinical data has demonstrated that dose-limiting toxicities (DLTs) are often shared by multiple ADCs delivering the same cytotoxic payload, regardless of target antigen and/or cancer type being treated. DLTs are generally associated with cells and tissues that do not express the target antigen (i.e., off-target toxicity), and there are many cases of limiting ADC doses below those required for optimal anticancer efficacy.

ADCs have the potential to safely improve the efficacy of cytotoxic drugs compared to when used as a single drug. The ADC attaches a drug to an antibody such that the antibody specifically targets only the lesion site, and thus, the drug is delivered only to the lesion site and not to normal tissues. In the case of cancer cells, antibodies that specifically bind to specific antigens expressed on the surface of cancer cells are used to specifically deliver highly toxic drugs to cancer cells, so as to kill only the cancer cells.

For the ADC to work, it must enter the target cell. The ADC's antibody binds specifically to a specific antigen expressed on the surface of a target cell, such as a cancer cell, and then enters the target cell through the clathrin-coated pit mechanism in the cell membrane.

The ADC incorporated into the cell is separated from clathrin, fuses with other vesicles within the cell, and then

follows the endosome-lysosome pathway. After reaching the endosome, the drug is separated from the antibody by specific elements of the specific tumor cell internal environment. Free cytotoxic drugs separated from antibodies pass through the lysosomal membrane and enter the cytoplasm. The activated drug exerts its pharmacological effect by binding to its own molecular target in the surrounding area, inducing cell death and killing cancer cells.

Among these activities, some cytotoxic drugs diffuse passively, are actively transported, or escape out of cells through dead cells. When the drug spread to the surroundings like this penetrates the cell membrane, it enters adjacent cells and causes a cell-killing effect that kills the surrounding cells as well (the so-called by-stander cell-killing phenomenon).

Most of the powerful cytotoxic drugs introduced into ADC are too toxic, and the drug payload released from ADC may also affect normal cells due to the bystander effect (FIG. 26).

Therefore, it is necessary to develop an ADC that acts very specifically on target cancer cells without causing serious side effects in normal cells.

Fibroblasts, which are a member of connective tissue, are the predominant cells in the stroma, especially in breast, prostate and pancreatic cancers.

Stromal fibroblasts, which are also called cancer-associated fibroblasts (CAPs), are a specific type of fibroblast found within the tumor microenvironment. Stromal fibroblasts have received great attention because they play an important role in tumor growth, invasion and metastasis. CAPs are responsible for the production of paracrine growth factors, proteolytic enzymes and ECM components.

According to the present invention, the camptothecin-based drug represented by Chemical Formula 1 or Chemical Formula 2 is a hydrophobic small molecule that can penetrate cell membranes, and thus, it can accumulate in high concentrations while penetrating deep into cancerous tissue, penetrate cell membranes, exert cytotoxicity inside cells, kill cells and then is released, and can continuously move into surrounding cells by penetrating cell membranes and acting inside the cells. In this case, there may be an advantage in the treatment of heterogeneous tumors due to the high effect of killing surrounding cells, but in some cases, in order to provide a modality for alleviating or preventing camptothecin-based payload-based ADC toxicity, such as ILD, which is a type of autoimmune disease, a payload that does not exert a cytotoxic effect on fibroblasts surrounding cancer cells when released from an ADC may be designed/-selected from a variety of candidates including active camptothecin derivatives of Chemical Formula 1 or Chemical Formula 2, for example, a payload that does not penetrate into cancer tissue, does not penetrate the cell membrane of fibroblasts, has a high IC50 or is rapidly eliminated through lymphatic drainage and does not accumulate at high concentrations. Furthermore, by appropriately applying the dosage-administration method, the desired efficacy (e.g., anticancer, combination therapy) and side effects (chronic cancer antigen exposure, drug resistance, ILD) may be precisely controlled.

Additionally, in order to increase the therapeutic index of the camptothecin-based drug as a payload while inhibiting the non-selective uptake of the camptothecin-based drug and/or ADC released from apoptotic cells, the present invention may select the payload of ADC from a group of camptothecin drug candidates represented by Chemical Formula 1 or Chemical Formula 2. For example, by selecting a camptothecin-based drug that has a high killing effect on target cancer cells at a low concentration of drug (payload) from the candidate drugs represented by Chemical Formula 1 or Chemical Formula 2, through the ADC dosage that lowers the total concentration of the payload of ADC, as shown in FIG. 26, it may suppress the by-stander cell-killing effect of free drugs (payloads) released from apoptotic cells on normal cells or overcome the problem of off-target toxicity of free camptothecin-based drugs (payloads) released from target/non-target apoptotic cells.

**[FL118 Drug (Chemical Formula 1-1) - ADC Provided with Acid-Sensitive Linker]**

The inventors of the present invention synthesized a new ADC using FL118 of Chemical Formula 1-1, which is a novel camptothecin payload with excellent *in vitro*/*in vivo* antitumor efficacy and excellent safety profile (Example 4). Since the FL118 payload exhibits a similar safety profile to Trodelvy's payload, SN-38, it was confirmed that the CL2A linker system with a highly efficient release profile in the tumor microenvironment may be utilized without significant toxicity in mouse and monkey models. Additionally, by using the hydrophilic CL2A linker system, it was confirmed that the minimal aggregation of ADC was ensured despite FL118 itself being extremely hydrophobic.

Specifically, as shown in FIG. 3, PBX-001 is an ADC composed of a humanized anti-Trop2 monoclonal antibody hRS7, a novel topoisomerase I inhibitor FL118, and a CL2A linker system. PBX-001 has a high DAR of approximately 7 to 8 and can release FL118 payload very efficiently in the tumor microenvironment at low pH values. As shown in FIGS. 11 to 13, the *in vitro* and *in vivo* evaluation of PBX-001 showed better efficacy than Trodelvy. Additionally, non-human primate toxicity studies demonstrated that PBX-001 has excellent safety.

Based on this, in order to propose an FL118-based ADC platform that can be switched to other targets using alternative monoclonal antibodies, the present invention newly designed Nimotuzumab FL118 (ADC having Nimotuzumab-CL2A-FL118), which is an ADC composed of humanized anti-EGFR monoclonal antibody h-R3 instead of humanized anti-Trop2 monoclonal antibody hRS7, and topoisomerase I inhibitor FL118 and CL2A linker system (Example 4).

In other words, for EGFR which is one of the targets that is difficult to develop as an ADC due to its distribution in normal cells, an ADC was produced by using Nimotuzumab, which is an antibody drug with low binding affinity, instead of the existing antibody drug Cetuximab, and through this, it was intended to develop an ADC of the EGFR target with reduced on-target toxicity.

The $K_d$ value of Cetuximab is 1.8*$10^{-9}$ M, whereas the $K_d$ value of Nimotuzumab is 2.9*$10^{-8}$ M.

Example 4 and FIG. 22 show the synthesis method of Nimotuzumab-CL2A-FL118, which is a new ADC of the EGFR target, and the *in vivo* evaluation results of ADC using two types of Cetuximab and Nimotuzumab.

Although Nimotuzumab-CL2A-FL118 was designed to solve the toxicity problem, surprisingly, it was found that Nimotuzumab-CL2A-FL118 showed better *in vivo* efficacy compared to Cetuximab-CL2A-FL118, which served as a control group (reference) (FIG. 22). In the ADC *in vitro* assay of FIGS. 18 to 20, it was expected that the two ADCs would show similar efficacy or Cetuximab-CL2A-FL118 would show slightly better efficacy, but the *in vivo* assay yielded results that exceeded expectations (FIG. 22).

By using an acid-sensitive linker, the immunoconjugate of the present invention may efficiently release camptothecin-based drugs not only inside cancer cells but also around cancer tissues after antigen binding, thereby overcoming resistance mechanisms related to ADC processing (FIG. 3).

Therefore, one embodiment of the present invention relates to an immunoconjugate including [FL118 drug of Chemical Formula 1] - [acid-sensitive linker] - [nimotuzumab or an antigen-binding site-containing fragment thereof].

(i) One or more FL118 drugs of Chemical Formula 1 are linked to nimotuzumab or an antigen-binding site-containing fragment thereof through an acid-sensitive linker.
(ii) After targeting to cancer cells by an antigen-binding site that targets the EGFR antigen of cancer cells, the acid-sensitive linker is decomposed in an acidic atmosphere (pH ≤ 7) surrounding the cancer, and at least part of the FL118 drug of Chemical Formula 1 is released, and the free FL118 drug of Chemical Formula 1 penetrates the cell membrane and moves into the cell.
(iii) The FL118 drug of Chemical Formula 1 inhibits the action of the efflux pump, thereby enriching the free FL118 drug of Chemical Formula 1 in cells.
(iv) Optionally, the immunoconjugate to which the FL118 drug of Chemical Formula 1 is linked may be internalized into cells, thereby liberating the FL118 drug of Chemical Formula 1 from lysosomes.

### [Receptor-Ligand Association and Dissociation Constant $K_d$]

According to the present invention, in an antibody or antigen-binding site-containing fragment (B-1) thereof having a dissociation constant $K_d$ value of 1*$10^{-8}$ M or more to less than 1*$10^{-6}$ M for an epitope of a cell surface protein; or, in an antibody or antigen-binding site-containing fragment (B-2) thereof that binds to a cell surface protein expressed in both normal and cancer cells, but has a higher binding affinity when binding divalently than monovalently to the epitope of the cell surface protein, the binding between the epitope of a cell surface protein and an antibody or an antigen-binding site-containing fragment thereof corresponds to a type of receptor-ligand binding.

Herein, the dissociation constant $K_d$ is a scale that can quantitatively measure the strength of the ligand binding (corresponding to the binding of the antibody).

Every cell in a multicellular organism must receive and respond to signals from other cells to know when to grow, divide, differentiate, connect, disperse or die. Cells must receive molecular signals through membrane-bound or intracellular receptors and translate the received signals into meaningful cellular responses, such as cell division or differentiation.

After the binding interaction between a receptor and its cognate (matching) ligand, a responding cell must convey knowledge of this receptor-ligand interaction to members of a molecular signaling pathway that can elicit an appropriate cellular response. This process is called signal transmission. The end result of the signal transduction pathway is a change in the behavior of the responding cell. These changes collectively represent the result (or response) of the receptor's encounter with its ligand, and it may include a combination of cell division, differentiation, migration, changes in metabolic state, changes in the expression of surface or cytoplasmic molecules, and secretion of new compounds such as chemokines or cytokines.

Enzyme-substrate binding interactions are generally monovalent (one enzyme molecule bound to one substrate molecule) and of moderate affinity, with only a few enzymes able to interact with any one substrate. In contrast, interactions between antigen and receptor molecules can have very high affinities, and the diversity of receptors that can recognize specific antigens is surprising.

The receptor molecule attaches to the ligand through the same type of noncovalent chemical bond that enzymes use to bind to their substrates. These include hydrogen and ionic bonds, hydrophobic and van der Waals interactions. The key to a meaningful receptor-ligand interaction is that the sum of binding interactions holds the two interaction surfaces

together with sufficient binding energy and for sufficient time such that a molecular signal indicating that the receptor has bound its matching ligand can be received by the cell. Since these noncovalent interactions are individually weak, many of these interactions are required to form biologically relevant receptor-ligand associations. Additionally, since each of these noncovalent interactions operates only over very short distances (typically about 1 angstrom (1 Å = $10^{-10}$ meters)), high-affinity receptor-ligand interactions depend on a very close "fit" or degree of complementarity between the receptor and the ligand. In an aqueous environment, noncovalent interactions are weak and depend on the close complementarity of receptor and ligand shapes.

In short, multiple noncovalent bonds allow receptor-ligand interactions to achieve sufficient binding energies and interaction times to activate molecular reactions.

In the case where the immunoconjugate of the present invention binds to an epitope of a surface protein of the target cell through the antigen binding site, and as a result, the immunoconjugate is internalized into the cell together with the payload drug through a process called receptor-mediated endocytosis, as described above. it follows the rules of chemistry regarding receptor-ligand interaction/binding.

Antibodies that recognize antigens on cancer cells must be internalized into cells through receptor-mediated endocytosis together with the drug. Bispecific antibodies are being developed to increase cell internalization in cancer cells.

MEDI4267 (Trastuzumab-META), which is being developed by Medimmune and AstraZeneca, is a biparatopic antibody targeting two non-overlapping epitopes in HER2, which induces HER2 receptor clustering and through this, it was shown that it promotes cell internalization, lysosomal trafficking and degradation.

The strength of the binding interaction between the receptor binding site (S) and the ligand (L) can be described by using the following formula:

(Formula) $K_d = [S][L]/[SL]$

Herein, the dissociation constant of the reaction, $K_d$, defines the relationship between the concentration of the receptor-ligand pair [SL] and the product of the free receptor site concentration [S] and the free ligand concentration [L]. The unit of dissociation constant is molarity (M). The lower $K_d$, the higher the affinity of the interaction. When 50% of the binding site is occupied, [SL] = [S], $K_d$ = concentration of free ligand.

The dissociation constant $K_d$ is a measure that can quantitatively measure the strength of ligand binding. The lower $K_d$, the higher the affinity of the interaction. When the concentration of free ligand is equal to $K_d$, 50% of the ligand binds to the receptor.

For example, in FIG. 17, $BC_{50}$ means binding concentration at 50% maximum.

For comparison, the $K_d$ values of many enzyme-substrate interactions are in the range of $10^{-3}$ to $10^{-5}$ M, similar to the $K_d$ values of somewhat lower affinity antigen-antibody interactions early in the immune response.

However, since the antibody genes expressed upon initial antigen stimulation are mutated and selected during the immune response, the $K_d$ of antigen-antibody interactions in the later stages of the immune response may be as low as $10^{-12}$ M. This is a very strong interaction, and even if the antigen concentration is as low as $10^{-12}$ M, half of the antigen molecules are completely bound to the receptor. Recent studies on innate toll-like receptors have shown that the $K_d$ of interaction of these receptors with their respective ligands ranges from $10^{-7}$ to $10^{-8}$ M.

The affinity of receptor-ligand interactions may be measured by equilibrium dialysis or surface plasmon resonance (SPR).

Interactions between receptors and ligands may be multivalent.

Many biological receptors, including B cell receptors, are characterized as multivalent because they have more than one ligand binding site per molecule. When both receptor and ligand are divalent (e.g., a bivalent B-cell receptor binds to two identical ligands on the bacterial surface), the overall binding interaction is the same, but becomes significantly stronger than the interaction between a monovalent receptor and a ligand (however, the strength of binding to two identical ligands on the same cell through two identical receptor sites on a molecule may be slightly less than twice the strength of binding through a single receptor site. This is because divalent binding may slightly alter the geometry of the receptor or ligand, thus slightly disrupting the "fit" of the individual interactions).

Many of the advantages of bivalent bonding stem from the fact that noncovalent interactions are inherently reversible. The ligand spends part of its time bound to the receptor and part of its time unbound, or "off." When more than one binding site is involved, it is less likely that all receptor sites will be "off" at the same time, making the receptor less likely to release its ligand. FIG. 28 compares monovalent interaction (a) and divalent interaction (b). In FIG. 28(a), when a monovalent receptor approaches a multivalent antigen, the receptor is in equilibrium with the ligand (circled in red), some of the time it is bound (binding is "on"), and some of the time it is not bound (binding is "off"). The ratio of time in the "on" state to the "off" state determines the affinity of the receptor-ligand interaction and is related to the strength of the sum of non-covalent interactions between the receptor and the ligand. In FIG. 28(b), a bivalent or multivalent bond helps ensure that the interaction between two molecules (or actually two cells) is not lost when one site momentarily releases its ligand, as

does a monovalent bond.

The term "avidity" is used to describe the overall strength of the collective binding interactions that occur during multivalent binding.

Due to the fluid nature of cell membranes, most membrane-bound antigen receptors function in a multivalent manner, even though the individual receptor molecules are essentially monovalent.

FIG. 29 is a conceptual diagram of a cell surface receptor binding to a multivalent antigen to form a cluster. (a) When receptors on the cell surface encounter a multivalent array of ligands, for example on the bacterial cell surface, (b) the receptors migrate in the plane of the membrane to form occupied receptor clusters. That is, FIG. 29 shows how multivalent antigens that interact with membrane-bound receptors in a reversible manner gradually stabilize increasingly larger receptor clusters in the cell membrane.

This is because once one or two stable connections are established, other receptors that diffuse randomly in the fluid environment of the cell membrane are caught in the receptor cluster and grow accordingly. These receptor clusters facilitate intramolecular interactions on the cytoplasmic side, thereby allowing cell activation signals to be transmitted to the nucleus.

In short, multivalent binding interactions have increased affinity compared to monovalent binding interactions, resulting in longer ligand occupancy time on the receptor. Monovalent receptors may also form multivalent clusters when they interact with membrane-bound multivalent ligands. Even if the epitope in a cell surface protein is monovalent, a multivalent cluster may be formed in the immunoconjugate of the present invention using a bivalent antibody.

When cells are activated, such as cancer cells, their receptor expression patterns may change, making them more or less responsive to certain signals. When considering the strength of the interaction between receptor and ligand, it is important to consider the anatomical environment in which this interaction occurs (e.g., pH of tumor tissue, solid tumor, cold tumor/hot tumor).

Immune antigen receptors may be transmembrane, cytoplasmic or secreted (e.g., antibodies).

For most biologists, the word "receptor" conjures up images of a transmembrane protein, dutifully waiting for a soluble ligand to diffuse into its surroundings such that it can respond to the ligand's signal.

In this regard, the affinity is low, and low concentrations of antibodies cannot bind to the antigen. Therefore, the anti-cancer immunotherapy agent of the present invention having an antibody or antigen-binding site-containing fragment (B-1) thereof having a dissociation constant $K_d$ value of $1*10^{-8}$ M or more to less than $1*10^{-6}$ M for an epitope of a cell surface protein; or an antibody or antigen-binding site-containing fragment (B-2) thereof that binds to a cell surface protein expressed in both normal cells and cancer cells, but has a higher binding affinity when binding divalently rather than monovalently to the epitope of the cell surface protein may penetrate deep into cancer tissue.

With regard to the antigen binding affinity of an antibody, the fold of an antibody-containing immunoglobulin consists of a pair of β sheets formed by β strands connected by loops that define the protein binding specificity.

In antibodies, each immunoglobulin domain forms a ring of amino acid domains with a characteristic spacing of disulfide bonds (about 67 amino acids separating two cysteine residues). Most immunoglobulin domains include approximately 110 amino acids, and each β-sheet consists of 3 to 6 strands. Pairs of β sheets within each domain are stabilized by intrachain disulfide bonds. Adjacent domains are linked to each other by relatively unstructured polypeptide chains.

Within each immunoglobulin (Ig) domain, several β strands are arranged in parallel to form a pair of β sheets. Along the amino acid sequence of each β strand, hydrophobic and hydrophilic amino acids are arranged alternately such that the hydrophobic amino acids of one sheet face the amino acids of the opposite sheet, and the hydrophilic residues interact with the environment. Therefore, the two β sheets form a very stable "hydrophobic sandwich", in which each domain is stabilized by hydrophobic interactions between the sheets and the entire protein is significantly soluble.

Immunoglobulin domains are composed of amino acid residues arranged in β sheets linked by variable loops. The two β-pleated sheets are indicated by two shades of blue. They are held together by hydrophobic interactions and conserved disulfide bonds (not shown). The three loops of each variable domain, shown in red, vary considerably in length and amino acid sequence and constitute the antigen-binding site. Therefore, this is called the complementarity determining region (CDR).

Antibody heavy chains include four or five domains, whereas light chains include only two. In each case, the antigen binding function is located within the amino (N)-terminal domain of the protein.

**[EGFR Targeting Antibody-Drug Conjugate]**

Epidermal growth factor receptor (EGFR), which is a member of the ErbB family of receptor tyrosine kinases, plays a central role in tumorigenesis in several types of solid tumors.

EGFR mutations and/or upregulation have been frequently identified in patients with non-small cell lung cancer (NSCLC), colorectal cancer (CRC), head and neck squamous cell carcinoma (HNSCC) and glioblastoma. Inhibiting EGFR activation with tyrosine kinase inhibitors (TKIs) or monoclonal antibodies (mAbs) has achieved great clinical

success in inhibiting tumor growth and improving patient survival.

However, even after the initial response, disease progression appears to be inevitable, which may be due to primary or acquired resistance mechanisms such as EGFR secondary or tertiary mutations, mutations in downstream mediators of EGFR signaling, or bypass of RTK signaling. Overcoming resistance to current EGFR-targeted therapies is a clinical challenge in cancer treatment and a major unmet medical need.

In this regard, the immunoconjugate of the present invention, in which a camptothecin-based drug (A) that degrades DDX5 protein is bound, through a linker (C), to an antibody or an antigen-binding site-containing fragment (B-1) thereof having a dissociation constant $K_d$ value of $1*10^{-8}$ M or more to less than $1*10^{-6}$ M with respect to an epitope of EGFR protein; or an antibody or antigen-binding site-containing fragment (B-2) thereof that has a higher binding affinity with respect to an epitope of EGFR protein when binding divalently than when binding monovalently, may provide an EGFR-targeted treatment that can solve difficult problems.

Antibody-drug conjugates (ADCs) are a new class of drugs that use antibodies to selectively deliver cytotoxic payloads to tumors expressing high levels of tumor-related antigens. By accumulating toxins locally in the tumor, it minimizes systemic toxicity and increases cancer cell death, thereby providing a higher therapeutic index than chemotherapy or maternal antibodies. Additionally, ADCs can bypass some resistance mechanisms by selectively delivering anti-mitotic or DNA damaging toxins to cancer cells to induce cell death, which is a mechanism independent of oncogenic signal inhibition. Recently, two ADCs targeting EGFR variant III (AMG-595 and ABT-414) demonstrated promising monotherapy efficacy in patients with relapsed glioblastoma, and these responses correlated with the presence of EGFR amplification or EGFRvIII, so as to provide early clinical validation of EGFR-targeted ADC in patients with EGFR amplified or mutant cancer.

Nimotuzumab (humanized IgG1, also known as hR3) is an EGFR-targeting antibody approved for the treatment of non-small cell lung cancer and glioma in many developing countries. The excellent selectivity of hR3 for EGFR-positive cancer cells provides a strong basis for designing hR3-based ADCs that can treat patients who have failed anti-EGFR treatment by enhancing cancer cell death while minimizing normal tissue toxicity.

Nimotuzumab is also in clinical trials for the treatment of a variety of solid tumors, including non-small cell lung cancer, CRC, gastric cancer and pancreatic cancer. Due to its unique binding kinetics with the EGFR receptor, hR3-related side effects in humans are only mild to moderate in severity. In particular, patients had a much lower incidence of severe acneiform rash and gastrointestinal mucosal toxicity, and these side effects were at levels that limited the toxicities typically observed with EGFR-targeted therapies.

Nimotuzumab is an antibody (B-1) with a dissociation constant $K_d$ value of $1*10^{-8}$ M or more to less than $1*10^{-6}$ M for an epitope of EGFR protein; or it corresponds to an antibody (B-2) that has a higher binding affinity when binding divalently than monovalently to the epitope of the EGFR protein.

Although inhibiting EGFR-mediated signaling has proven to be effective in treating certain types of cancer, mechanisms to restore EGFR signaling or activate alternative pathways that promote the proliferation and survival of malignant cells are rapidly evolving such that the efficacy and utility of approaches that inhibit EGFR function are limited. Considering the fact that the overexpression of EGFR is commonly observed in many cancers, EGFR-targeted antibody-drug conjugates (ADCs) can selectively kill cancer cells independent of blocking EGFR-mediated signaling.

**[Antibody or Antibody-Binding Site-Containing Fragment (B) Thereof With $K_d$ value of $1*10^{-8}$ M or More to Less Than $1*10^{-6}$ M, or Higher Binding Affinity When Binding Divalently Than Monovalently]**

The immunoconjugate of the present invention preferably uses an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

Non-limiting examples of the antibody or antigen-binding site-containing fragment (B-2) thereof that binds to a cell surface protein expressed in both normal and cancer cells, and has a higher binding affinity when binding divalently than binding monovalently to an epitope of the cell surface protein include an antibody that binds to cell surface proteins expressed in both normal and cancer cells, such as the growth factors illustrated in FIG. 2, especially in cancer cells.

Therefore, an antibody that has a higher binding affinity when binding divalently than when binding monovalently to an epitope of a cell surface protein may target cell surface proteins whose expression pattern changes when the cell is activated, such as cancer cells.

[An antibody that binds to a cell surface protein expressed in both normal and cancer cells and has a higher binding affinity when binding divalently to the epitope of the cell surface protein], like nimotuzumab, may be [an antibody that binds bivalently to EGFR epitopes] or may have a low binding affinity.

The reduced side effects of nimotuzumab (mAb) are related to its intrinsic binding affinity and density dependence. The low affinity between nimotuzumab and EGFR allows for an optimal drug dose that is below the toxic dose.

According to predictions from mathematical models, the binding affinity ($K_d$) for anti-EGFR mAb must be in the range of $10^{-8}$ M to $10^{-9}$ M to maximize tumor cell targeting while minimizing normal cell toxicity [Crombet T, Osorio M, Cruz T, et al. Use of the humanized anti-epidermal growth factor receptor monoclonal antibody h-R3 in combination with radiotherapy in the treatment of locally advanced head and neck cancer patients. J Clin Oncol 2004;22(9):1646-54]. Nimotuzumab falls within this range, but cetuximab and panitumumab have a binding affinity that is more than 10 times stronger. According to a recently proposed density model, the property of nimotuzumab to bind transiently monovalently and strongly bivalently to the EGFR epitope further explains the reduced side effects of nimotuzumab. EGFR expression in normal cells (e.g., skin epithelial cells) is too low to cause nimotuzumab bivalent binding, thereby limiting the efficacy of the mAb and avoiding unwanted toxicity. On the other hand, overexpressing tumor cells have sufficient receptor density for nimotuzumab to bind bivalently and strongly inhibit the receptor. This theory is supported by comparative dose binding curves of nimotuzumab and cetuximab to normal skin and kidney cells, where nimotuzumab shows much less binding than cetuximab [Garrido G, Rabasa A, Gracia E, et al. Binding properties of the anti-EGFR monoclonal antibody, nimotuzumab, limit its interaction with the EGFR in renal and epidermal cells. AACR 100th Annual Meeting, 2009 (abstract 2763). Available from: http://www.ymbiosciences.com/upload_files/nimo_poster_AACR2009.pdf [Last accessed 22 June 2009]].

**[Advantages of Immunotherapy Including Antibody or Antigen-Binding Site-Containing Fragment (B) Thereof Having $K_d$ value of $1*10^{-8}$ M or More to Less Than 1*10-6 M or Having Higher Binding Affinity When Binding Bivalently Than Monovalently]**

According to the present invention, anticancer immunotherapy agents having an antibody or antigen-binding site-containing fragment thereof (B) having a $K_d$ value of $1*10^{-8}$ M or more to less than $1*10^{-6}$ M or having a higher binding affinity when binding bivalently than when binding monovalently may relieve solid stress within the microenvironment of cancer and thereby increase accessibility to anticancer drugs and/or immunotherapy agents, and thus, it is possible to increase treatment effectiveness.

Traditionally, cancer has been considered a cytogenetic disease caused by mutations in genes that control cell division, differentiation and death. However, recently, the cancer microenvironment is also known to be involved in the occurrence, progression, evasion of immune surveillance and treatment response of cancer, and has received much attention. As cancer cells proliferate, they modify the structure and function of the surrounding environment biochemically and physically/mechanically. As a result, this physical degeneration affects both cancer cells and their microenvironment and can accelerate cancer progression or make it resistant to treatment.

The eight typical characteristics of cancer are: 1. continuous cell division signals, 2. evasion of growth inhibitory signals, 3. resistance to apoptosis, 4. indefinite cell division, 5. promotion of angiogenesis, 6. activation of tissue invasion and metastasis., 7. changes in energy metabolic activity, and 8. avoidance from immune destruction.

As cancer grows, it biochemically and physically transforms surrounding tissues, and these transformations cause physical deformities that can affect cancer cells and the surrounding microenvironment.

Four additional characteristics based on the physical deformity of the cancer are: (i) solid stress and elastic energy, (ii) interstitial fluid compression, (iii) rigidity (elasticity), and (iv) matrix structure.

Although these four characteristics are conceptually separate, they can interact synergistically with each other, and moreover, they worsen the eight existing biological characteristics of cancer such that it is possible to promote the division of cancer cells, metastasis, evasion of immune surveillance and treatment resistance.

With regard to solid stress and elastic energy, solid stresses, which are also known as "residual stresses," refer to mechanical forces such as compressive, tensile and shear, and they are internalized in, or transmitted between solid elastic elements of cells or the extracellular matrix. The magnitude of solid stress is expressed in Pascals (Pa) or millimeters of mercury (mmHg), and ranges from about 0.7 mmHg for glioblastoma to 75 mmHg for pancreatic ductal adenocarcinoma.

The various mechanisms that cause solid stress in cancer are the following.

**(1) Increase in tissue volume by cell division, cell infiltration and matrix deposition.**

This increased volume pushes out the viscoelastic structures that exist inside and outside the cancer tissue and causes solid stress on the cancer and surrounding tissues. Therefore, when the number of cancer cells decreases through chemotherapy, solid stress decreases, and the pressure on blood vessels around the cancer tissue also decreases.

**(2) Deformation of normal tissue surrounding cancer tissue**

Some cancers are well-controlled and grow as nodular masses, and these cancers coexist with surrounding normal tissues and push the surrounding normal tissues, thereby increasing mechanical stress. Meanwhile, some

cancers find and infiltrate vulnerable parts of surrounding normal tissue and grow by mixing with normal tissue, and in this case, weaker solid stress is created compared to the former case.

### (3) Swelling due to water absorption

Complex polysaccharides in the matrix, such as hyaluronic acid, increase water content due to osmotic pressure, and this increased moisture within cancer tissue causes a type of solid stress different from hydraulic pressure.

### (4) Cell contraction by Actomyosin

Fibroblasts, immune cells and cancer cells can move within cancerous tissue or contract the matrix as they attempt to repair damaged tissue. Cell contraction generates a tensile force that contracts the extracellular matrix and causes tension in some parts of the cancer tissue, which is soon balanced by compressing other parts.

It was first reported in 1997 that solid stress could inhibit the growth of cancer. This physical force is large enough to compress surrounding blood vessels or lymphatic vessels, and thus, when blood flow is reduced, it can cause hypoxia and promote metastasis of cancer cells or block access to anticancer drugs or immunotherapy drugs. Cancer cells and normal cells have a mechanical stimulus response system that can sense physical forces applied from the outside, and they may respond directly to cell-extracellular matrix or cell-cell forces, or also respond indirectly to changes in the extracellular matrix caused by solid stress.

By relieving solid stress in cancer animal models, pressure on blood vessels is reduced, which in turn increases accessibility to anticancer drugs, resulting in increased survival rates. This approach to relieving solid stress may also increase the effectiveness of other treatments, such as immunotherapy. Additionally, the payload released from the immunoconjugate of the present invention may effectively exert a bystander effect.

In most organs, blood that enters through the arteries drains into the veins and some remaining tissue fluid is discharged through the lymphatic vessels, thereby maintaining interstitial fluid homeostasis, and a normal organ has an interstitial fluid pressure close to zero. When a tumor develops, this balance is disrupted due to pressure on blood vessels or blood vessels/lymphatic vessels with increased permeability. Loose blood vessels and narrowed drainage ducts lead to an increase in interstitial fluid within the tissue, which in turn leads to an increase in interstitial fluid pressure. The interstitial fluid pressure, which was maintained at a certain level within the cancer tissue, decreases steeply in the area surrounding the tumor, which in turn creates a flow of tissue fluid from the center of the tumor toward the surrounding normal tissue where the lymph drainage ducts are located.

This flow of interstitial fluid applies shear stress to cells outside the blood vessels, and shear stress soon causes the following changes in cancer cells and surrounding cells: fibrocyte activation, regulation of blood vessel/lymphatic vessel formation, matrix metalloproteinases (MMP) activity regulation, cancer cell metastasis mobility regulation, and cell cycle inhibition. Meanwhile, since immune cells can also sense interstitial fluid flow, this flow of tissue fluid may also be involved in regulating immunity.

In addition to directly regulating mechanical signaling mechanisms, tissue fluid flow due to interstitial fluid pressure differences is also involved in cancer progression and responsiveness to anticancer treatment. High interstitial fluid pressure can inhibit the spread of anticancer drugs arriving through blood vessels to cancer tissues, and additionally, interstitial fluid flow from cancer tissues toward blood vessels/lymphatic vessels helps cancer cells spread to other tissues through blood vessels or lymphatic vessels.

Since the accumulated solid stress compresses blood vessels, thereby hindering blood flow and increasing interstitial fluid pressure, the method of relieving solid stress using the immunoconjugate according to the present invention also removes the pressure applied to blood vessels/lymphatic vessels and improves blood flow, and thus, it is possible to maintain a normal level of interstitial fluid pressure.

### [Linker (C)]

Among the elements that constitute an ADC, the linker is what combines an antibody and a cytotoxic drug.

When designing an ADC, it is important to enhance the specificity for target tumor cells, ensure stability in plasma, reduce toxicity to normal cells, and increase the tumor cell killing effect by selecting an efficient drug.

The linker must be stable in the bloodstream, prevent the drug from separating from the antibody, maintain in a prodrug state until it reaches the target, and minimize damage to normal tissues. The most ideal linker is one that is stable when the ADC circulates throughout the body, but is cleaved in the target cells to properly release the cytotoxic drug and safely deliver the drug to the target, thereby allowing the ADC to have both efficacy and safety.

When linking a drug to an antibody with a linker, the drug should not affect the structural stability, substrate binding characteristics and pharmacokinetics of the antibody.

In particular, the ADC must maintain the same affinity as the antibody before it is combined with the drug. In other

words, the drug bound to the antibody should not interfere with antibody-antigen binding.

Due to the high hydrophobicity of a camptothecin-based drug (A) that degrades DDX5 protein, drug absorption and payload release do not occur by depending on the drug target of the antibody, but non-selective uptake occurs in macrophages and the like, causing unexpected side effects. In order to reduce the side effects that appear, the linker (C) is preferably hydrophilic.

Linkers used in ADC are divided into non-cleavable and cleavable depending on their cleavage ability.

Non-cleavable linkers have relatively high plasma stability and are resistant to proteolysis. A thioether linker is a representative example of non-cleavable linkers.

Unlike cleavable linkers, non-cleavable linkers do not decompose the linker itself, and thus, the drug can be released only after the antibody is decomposed after introduction into cells in the form of ADC. For example, after being introduced into a cell, the antibody must be decomposed to release the drug in the form of a complex with the linker, and this complex has drug activity.

The drug released in this way is charged and is unlikely to spread to surrounding cells (bystander effect). Although there is no bystander effect that is toxic to surrounding cells, the effect appears only on target cells after target cell internalization, which means that it is relatively safe. It can be seen that ADCs manufactured with non-cleavable linkers are more dependent on biological mechanisms within target cells.

The cleavable linker is cleaved in response to specific environmental factors and releases the drug into the cytoplasm. There are two types of cleavage types: enzyme cleavage type and non-enzyme cleavage type.

Non-enzyme cleavage types include acid-labile linkers and oxidation-reduction reaction linkers.

The antibody-drug conjugate (ADC) according to one embodiment of the present invention used (i) an acid-sensitive linker or (ii) an enzyme-sensitive linker (FIGS. 21 to 23).

Since proteolytic enzyme activity is high in cancer cells, it is recommended to use an enzyme-sensitive linker.

The enzyme cleavage form is cleaved by enzymes such as cathepsin B, β-glucuronidase, phosphatase, pyrophosphatase and sulfatase.

Peptide linkers are decomposed by proteolytic enzymes, and protease inhibitors are present in plasma, resulting in high plasma stability. Cathepsin B is a representative proteolytic enzyme used in ADC. Cathepsin B is present at high levels in tumor tissue, thereby imparting tumor selectivity to the ADC.

The peptide linker consists of a dipeptide or tetrapeptide that is recognized and cleaved by proteolytic enzymes within the lysosome once the ADC is internalized. Tetrapeptide was used in the early stages of development and showed limitations such as relatively slow drug release and the possibility of aggregation when combined with hydrophobic drugs. This problem was solved with the development of dipeptide linkers such as Val-Cit, Phe-Lys, Val-Lys, and Val-Ala.

The β-glucuronide linker is degraded by β-glucuronidase, which is a glycolytic enzyme present in lysosomes. β-glucuronidase is overexpressed in some tumor cells, thereby imparting tumor specificity.

β-glucuronidase is abundantly present inside rhizosomes and is known to be overexpressed in some tumors. This enzyme is highly active at low pH, but drops to 10% at neutral pH. Due to this property, ADCs with a β-glucuronide linker have improved stability in plasma, thereby preventing off-target drug release. In order to confirm the plasma stability of the β-glucuronide linker, when an experiment was conducted in rat plasma with the Val-Cit linker, the β-glucuronide linker was found to be much more stable with 89% and less than 50% after 7 days, respectively, and the half-life was approximately measured to be 81 days and 6 days. In addition, ADC with a β-glucuronide linker showed high stability and efficacy despite combining a high dose of cytotoxic drugs (up to 8).

The enzyme-sensitive linker according to one embodiment of the present invention may be -S-Maleimide-Spacer-Enzymatic cleavable site-Self immolative spacer-(Payload) or -S-Dibromaleimide-Spacer-Enzymatic cleavable site-Self immolative spacer-(Payload). FIG. 23 analyzed the *in vivo* efficacy of the ADC (DAR 8 or DAR 4) of the present invention using an enzyme-sensitive linker.

Herein, a non-limiting example of the self immolative spacer is exemplified in FIG. 27.

*In vivo* transformation due to linker deconjugation may occur due to chemical dissociation or enzymatic cleavage.

In the case of ADC where a linker is connected to a lysine residue by an amide bond, it may be detached in the form of a linker-cytotoxic drug due to amide hydrolysis by enzymes, and in the case of ADCs where a linker including a maleimide group or a disulfide group is attached to a cysteine residue, the S of the cysteine residue is reduced and the linker-cytotoxic drug attached in an exchange manner may be detached. In the detached state, the cysteine residues of the monoclonal antibody may exist in a disulfide bonded state with other cysteine amino acids or endogenous or exogenous substances including S, such as glutathione (GSH). Therefore, ADCs lose the mechanism of action on the target due to cytotoxic drugs, while the detached linker-cytotoxic drug may form an adduct with another protein or enzyme, or be metabolized to become active, thereby causing toxicity.

A representative redox linker is a disulfide linker. Disulfide linkers are also a type of chemically unstable linker and are based on oxidation-reduction reactions. After internalization, the linker is degraded by disulfide exchange or reducing agents such as glutathione, thereby releasing cytotoxic drugs.

Glutathione is a low-molecular-weight thiol that regulates cell proliferation and death and is known as an

antioxidant that protects cells from inflammation and oxidative stress. Glutathione exists at a concentration of 0.5 to 10 mM within cells, but in tumors under hypoxic conditions, it exists at a concentration up to 1,000 times higher. It exists at a low concentration (2 to 20 $\mu$M) in plasma, and the disulfide linker has high plasma stability and reduces non-specific drug release, making it a relatively safe linker that reacts tumor-specifically.

In the present invention, the acid-sensitive linker is stable in the neutral environment of blood at pH 7.3 to 7.5, but it refers to a linker that releases the drug by hydrolysis in a weakly acidic environment such as the surrounding tumor cells (pH 6.5~7.2) or the endosome (pH 5.0~6.5) and lysosome (pH 4.5~5.0) where internalization occurs within the cell. Therefore, the acid-sensitive linker in the present invention has a hydrophilic molecular structure to create a hydrolytic environment. For this purpose, the acid-sensitive linker may include, for example, a polyethylene glycol (PEG) spacer.

However, acidic conditions are not limited to the tumor microenvironment and are often found extracellularly, which can lead to non-specific drug release. A representative linker is a hydrazone linker, and recently, silyl ether linkers are being studied. They have high stability in plasma, and the plasma half-life is over 7 days, which is a significant improvement over hydrazone, which is 2 to 3 days.

It is preferable that the camptothecin-based drug (A) and the acid-sensitive linker (C) are linked by a carbonate or ester bond such that it is decomposed in an acidic atmosphere (pH $\leq$ 7), and the free camptothecin-based drug is released when the acid-sensitive linker is decomposed.

The tetrapeptide linker showed a limit to the extent to which ADC aggregation could occur when combined with a hydrophobic drug.

CL2A, which is a linker used in Trodelvy that is an existing FDA-approved ADC, is a linker that satisfies all characteristics, such as (i) storage stability after manufacturing, (ii) stability in the blood upon administration (little exposure to free payload during plasma), and (iii) rapid release of payload in cancer tissue.

The Phe-Lys peptide inserted into the original CL2 derivative enables cleavage through cathepsin B. In an effort to simplify the synthesis process, phenylalanine was removed from CL2A, and accordingly, the cathepsin B cleavage site was removed. These changes had no effect on conjugate binding, stability or efficacy. This suggests that it was released from the conjugate primarily by cleavage of the pH-sensitive benzyl carbonate bond to the lactone ring of SN-38, rather than through the cathepsin B cleavage site in CL2.

The acid-sensitive linker used in the present invention may utilize a CL2A linker and may be designed as shown in Chemical Formula 7 below to selectively and efficiently deliver a camptothecin-based drug to cancer tissues. That is, in the present invention, the acid-sensitive linker may be derived from a compound of Chemical Formula 7 below:

[Chemical Formula 7]

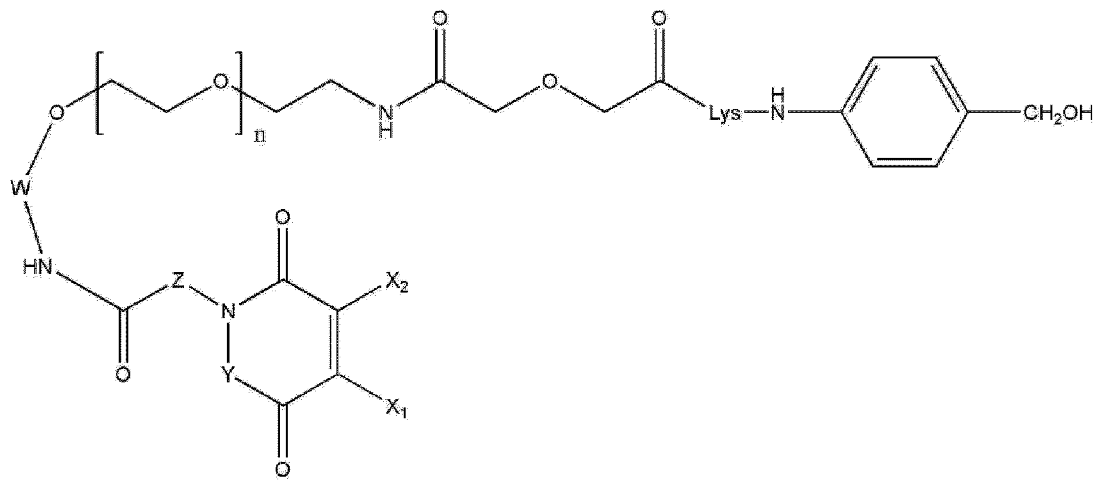

Herein, $X_1$ and $X_2$ are each independently -H or -halogen;

Y is -NH-, $-NR^A-$, or nothing (null);
Z is $-C_1-C_4$alkyl-, $-C_3-C_6$cycloalkyl-, $-(C_1-C_2$alkyl$)-(C_3-C_6$cycloalkyl$)-$, $-(C_3-C_6$cyclo alkyl$)-(C_1-C_2$alkyl$)-$, or $-(C_1-C_2$alkyl$)-(C_3-C_6$cycloalkyl$)-(C_1-C_2$alkyl$)-$;
W is $-R^B-$, -M- $-R^B-M-$, $-MR^B-$ or $-R^B-MR^C-$:
$R^A$ to $R^C$ are each independently $C_1-C_4$alkyl,
M is

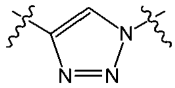

and;
n is an integer from 5 to 9.

Preferably in Chemical Formula 7,

$X_1$ and $X_2$ are each independently -H or -halogen;
Y is -$NR^A$-, or nothing (null);
Z is -$C_1$-$C_4$alkyl-, -($C_1$-$C_2$alkyl)-($C_3$-$C_6$cycloalkyl)-, or -($C_3$-$C_6$cycloalkyl)-($C_1$-$C_2$alkyl)-;
W is -$R^B$- or -$R^B$-$MR^C$-;
$R^A$ to $R^C$ are each independently $C_1$-$C_4$alkyl;
M is

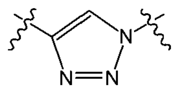

;

and
n may be an integer from 5 to 9.

In the present invention, the length of the linker, for example, n in Chemical Formula 7, may be an integer of 5 to 9, specifically n may be an integer of 6 to 8, and more specifically n may be 7, but is not limited thereto. Even in cases outside the above range, if there is no significant difference in effect due to change in linker length, all are naturally included within the equivalent scope of the present invention.

Since the water dispersibility of a drug may be improved by placing a polyethylene glycol (PEG) spacer between the drug and the antibody, the linker of Chemical Formula 7 includes a low molecular weight PEG moiety including a limited number (n = 5 to 9) of PEG monomers.

The acid-sensitive linker of Chemical Formula 7 is a customized linker that can be optimized according to the characteristics of a targeting target, payload and carrier.

Camptothecin-based drugs have various linkers and easy-to-attach sites for the production of antibody-drug conjugates. For example, the alcohol group portion of a camptothecin-based drug may be used as an attachment site to the linker.

Therefore, in the present invention, [camptothecin-based drug]-[acid-sensitive linker] may be one in which the alcohol moiety of the camptothecin-based drug is connected to the alcohol moiety of the acid-sensitive linker of Chemical Formula 7.

In order to develop a new ADC that can be selectively used for various solid cancers, it is essential to utilize a linker system that exceeds the drug delivery efficiency of existing ADC. In order to develop ADC targeting new antigens beyond already established drug targets such as Trop-2, Her2 and Folate Receptor, ADC for slow internalizing antigens such as CEACAM-5 and/or cancer-specific antigens such as NY-ESO-1/HLA Complex should be developed, but it is difficult to deliver a sufficient amount of drug with the limited drug delivery efficiency of the existing Val-Cit or MAC-glucuronide Linker system.

In particular, for efficient drug delivery to antigens such as CEACAM-5, the antibody-drug bond maintains stability in the blood and/or the surrounding environment of normal tissues, but requires the characteristic of rapidly releasing the drug in the surrounding environment of cancer cells, such as the tumor microenvironment.

If the drug can be released quickly even in the tumor microenvironment surrounding the cancer, it may be advantageously used to target various antigens (typically CEACAM-5, various Cancer Specific antigen-HLA Complexes, etc.) that have limitations in ADC uptake speed.

Therefore, the camptothecin-based drug that binds to and degrades DDX5 is a drug that has improved water dispersibility to prevent aggregation in body fluids such as blood and interstitial fluid compared to SN-38, and thus, the present invention is characterized by selecting an acid-sensitive linker that decomposes in the acidic environment ($pH \leq 7$) around the cancer tissue as a linker with a release profile that quickly and efficiently delivers the drug after reaching the cancer tissue, making full use of the hydrophobic small molecule's ability to penetrate deep into the cancer tissue and move into the cell by penetrating the cell membrane.

In addition, the linker is stable in the bloodstream and prevents the drug from separating from the antibody, and although it should be maintained in a prodrug state until it reaches the target and damage to normal tissues should be

minimized, the present invention may alleviate the problem of ADC aggregation occurring when combined with a hydrophobic drug by using an acid-sensitive linker having a hydrophilic molecular structure to create a hydrolysis environment.

Different metabolites are formed depending on the type of linker. In this regard, in the [camptothecin-based drug]-[acid-sensitive linker] of the present invention, the camptothecin-based drug and the acid-sensitive linker are preferably linked by a carbonate or ester bond such that the free camptothecin-based drug is released when the acid-sensitive linker is decomposed.

In general, carbamate bonds provide superior drug linker stability compared to ester and carbonate bonds with respect to hydrolysis. However, in the present invention, in order to design a camptothecin-based drug to be separable from the drug linker both outside and inside the cells surrounding cancer cells in an acidic atmosphere (pH) surrounding cancer cells, instead of carbamate bonds, it is characterized by using unstable ester or carbonate bonds.

The pH of blood is kept constant at 7.3 to 7.4. Therefore, the camptothecin-based drug is not cleaved from the acid-sensitive linker in the blood, and even if it is cleaved, the release rate of the camptothecin-based drug from the ADC at the neutral pH of serum is much reduced compared to that in tumor tissue in an acidic atmosphere.

[FL118 drug of Chemical Formula 1-1]-[Acid sensitive linker] may be derived from any one selected from the group consisting of compounds represented by Chemical Formulas 7-1 to 7-3 below.

[Chemical Formula 7-1]

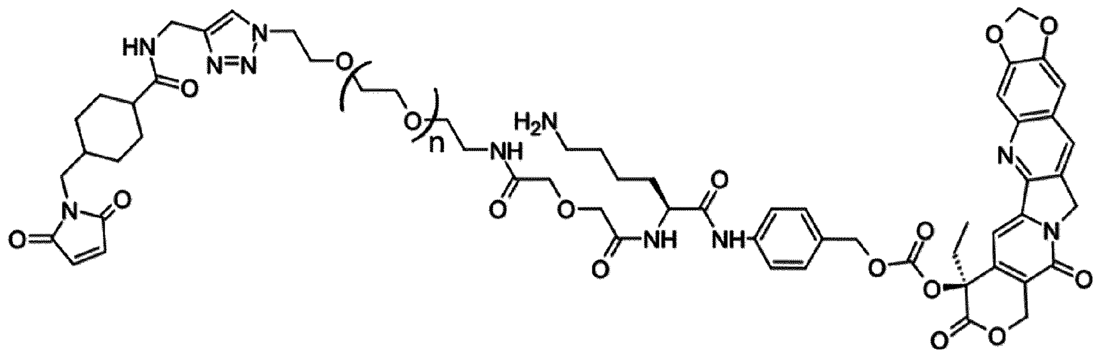

[Chemical Formula 7-2]

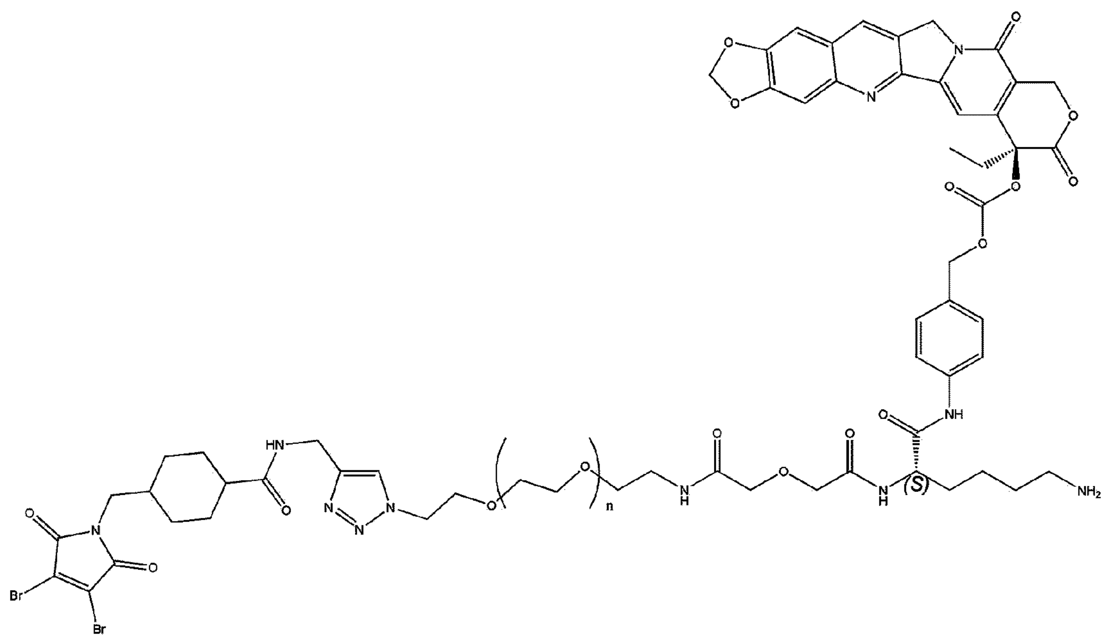

[Chemical Formula 7-3]

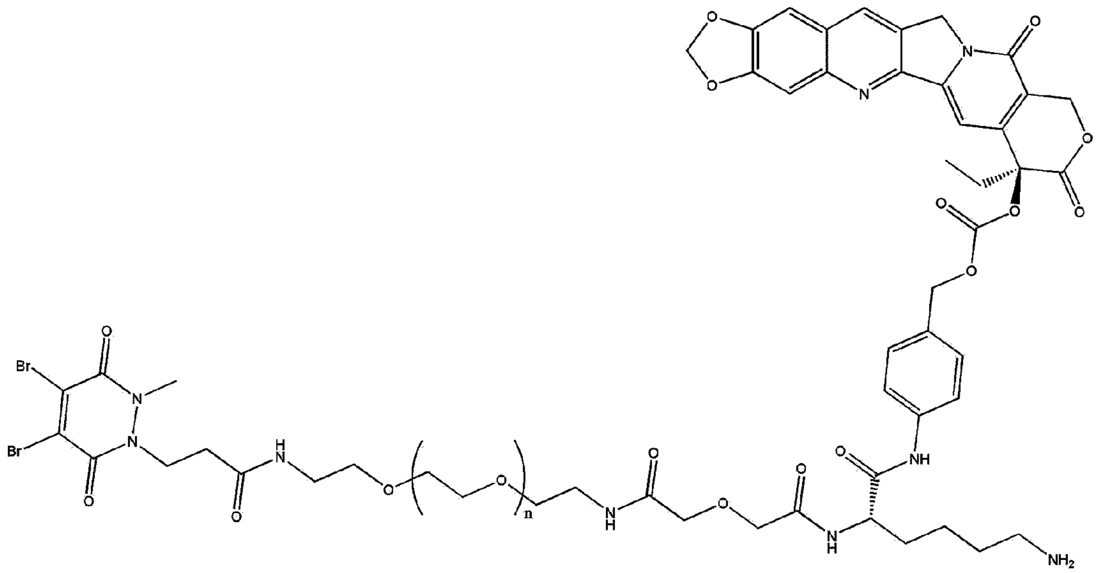

(where n is each independently an integer from 5 to 9).

In order for an ADC to operate efficiently, it is not enough to simply maximize powerful antigen-selective cytotoxicity against cells *in vitro,* but it must also be able to penetrate cancer tissue well and efficiently deliver the drug *in vivo* or in actual patients.

After the immunoconjugate of the present invention is targeted to cancer cells by an antigen-binding site that targets the antigen of cancer cells, the acid-sensitive linker is decomposed in an acidic atmosphere (pH ≤ 7) around the cancer, thereby freeing at least part of the camptothecin-based drug. The camptothecin-type drug is a hydrophobic small molecule, but compared to SN-38, it has improved water dispersibility to prevent aggregation in body fluids such as blood and interstitial fluid, and thus, it may penetrate deep into cancer tissue and penetrate the cell membrane to move into cells.

Therefore, the immunoconjugate of the present invention may rapidly release the drug in the tumor microenvironment surrounding the cancer by using an acid-sensitive linker that degrades in an acidic atmosphere (pH ≤ 7) surrounding the cancer, and the free camptothecin-based drug has a low molecular weight. Unlike antibodies, it has high cancer tissue penetration, and thus, it can solve the problems of existing ADCs, which have problems with antibodies that do not penetrate deep into cancer tissue.

Accordingly, the cells into which the free camptothecin-based drug moves may be the targeted cancer cells and/or surrounding cells thereof.

In the present invention, the linkage of [linker]-[antibody or antigen-binding site-containing fragment thereof] may be performed by linking a thiol group included in the antibody or antigen-binding fragment thereof to a maleimide group or maleic hydrazide group of the linker through the "click" reaction in Reaction Scheme 1.

[Reaction Scheme 1]

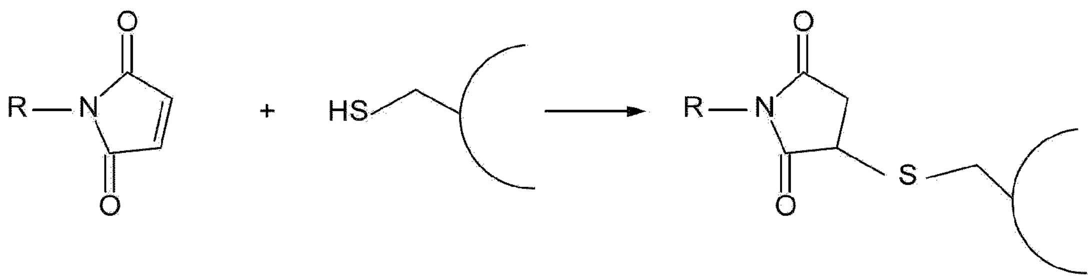

maleimide cysteine residue in protein modified cysteine

The synthesis of linkers is quite complex, and the efficient release of cytotoxic drugs is affected depending on which linker is used. In addition, even if the linker is attached to the same antibody amino acid sequence, it may affect biotransformation due to deconjugation of the linker in terms of the steric environment and electromagnetic environment depending on the type and length of the linker and the position of the antibody to which the linker is conjugated.

The development of the linker reflects a long half-life, which is an advantage of monoclonal antibodies (mAb) such that the mAb must be stable during systemic circulation, and it is important that the combination of the linker and the cytotoxic drug does not affect the stability and pharmacokinetics of the antibody.

In general, the catabolic reactions that may occur during systemic blood circulation related to linker-cytotoxic drugs are as follows, and there are various other catabolic reactions that have not been identified: hydrazone cleavage, protease-mediated dipeptide cleavage, esterase-mediated carbamate cleavage, hydrolysis of acetate ester, disulfide cleavage and succinimide ring opening.

In some cases, the catabolic reaction that occurs at a specific location of the linker-cytotoxic drug may maintain the cytotoxic effect and be active at the target, and it may cause toxicity in systemic blood circulation. Conversely, if the cytotoxic effect is lost, it may be difficult to expect a pharmacological effect even if the drug reaches the target.

A significant number of ADCs currently in clinical trials employ chemical linkers such as hydrazone, disulfide, peptide or thioether linkages. The process of drug release from the linker utilizes differences in specific pH or enzyme concentrations within cancer cells. Usually, hydrazone and disulfide linkers have limited stability in plasma. On the other hand, peptide-based linkers have excellent stability in plasma and are easy to control drug release.

The peptide-based valine-citrulline linker is cleaved by the cathepsin enzyme. Cleavable dipeptide linkers such as Valine-Alanine (Val-Ala) and Valine-Citrulline (Val-Cit) undergo rapid hydrolysis in the presence of lysosomal extracts or purified human cathepsin B, and thus, the cleavage principle of the linkers depends on the intracellular environment.

Some chemical linkers regulate the hydrophobicity balance of the antibody and drug to prevent aggregation of ADC in the bloodstream, which is a hydrophilic environment. In hydrophilic linkers and spacers, there are cyclodextrin, polyethylene glycol (PEG) or other polymers, and they play a role in stability in the bloodstream and pharmacokinetic properties.

Linkers containing hydrophilic spacers containing sulfonate- or PEG-, which exhibit high solubility in both organic solvents and aqueous solutions, solve many of the problems observed in hydrophobic linkers. PEG linkers have advantages such as water solubility, low toxicity, low immunogenicity and controlled linker chain length. In this regard, studies have reported that the use of PEG linkers significantly improves the *in vivo* pharmacokinetic profile and increases the half-life and plasma concentration, resulting in an increased plasma concentration-time curve (AUC).

Any linker used in the present invention may be used as long as it is previously known in the relevant field. Representative examples include CL-2A, MC-VC-PABC, MC-GGFG, MC-AAA, MC-VA-PABC, Mac-Glucuronide linker and the like.

**[Antibodies and Modification Thereof]**

Therapeutic antibodies are being used to treat a growing variety of diseases, including cancer, autoimmune and inflammatory diseases, infectious diseases and the like, and in some cases, they are revolutionizing the treatment of serious diseases. A variety of therapeutic antibodies are being used in three clinical situations: cancer, autoimmune and inflammatory diseases, and infectious diseases. Therapeutic antibodies used in cancer treatment target specific molecules expressed on tumor cells. Many studies have shown that this antibody kills tumor cells through two mechanisms. IgG1 antibodies bound to tumor cells are recognized by Fcγ receptors on macrophages and activate phagocytosis (in the cancer literature, this is called antibody-dependent cellular phagocytosis, ADCP). Additionally, FcγRIII of NK cells binds to tumor cell-binding antibodies and induces apoptosis of tumor cells by antibody-dependent cell-mediated cytotoxicity (ADCC). Trastuzumab (Herceptin), which is another therapeutic antibody with a specific target, reacts with the epidermal growth factor receptor (EGFR, also known as HER-2) found in some advanced breast cancers. EGF is required for tumor growth, and blocking the receptor causes cells to lack this essential activator. Herceptin has also been shown to provide protection by inducing both ADCP and ADCC.

Cancer treatment antibodies that react with two co-inhibitory receptors (CTLA-4 and PD-1) on tumor cells or with the co-inhibitory ligand PD-L1 work with the patient's immune system to eliminate the tumor. Antibody blockade against inhibitory proteins such as CTLA-4, PD-1 and PD-L1 allows existing tumor-specific T cells to destroy cancers that are very difficult to treat.

Monoclonal antibody treatments have many advantages over small molecule treatments. Since it has high target specificity, there are fewer side effects caused by acting on off-target areas, and since it has a large molecular weight and does not pass through the blood-brain barrier, there are fewer central nervous system side effects. In addition, since it is broken down into amino acids and metabolized by the reticuloendothelial system, it does not undergo hepatic metabolism or renal excretion, and thus, there is little drug interaction with other drugs, and it may be used relatively safely for patients with liver disease or kidney disease. It has the advantage of having a long half-life and only needing to be administered once every few weeks or months. On the other hand, since it is a protein, it has a large molecular weight and is easily destroyed in the gastrointestinal tract by proteolytic enzymes, and thus, it cannot be used as an oral medication. In addition, due to its long half-life, it has the disadvantage of not being able to be quickly removed non-invasively when unexpected side effects occur. Unlike small molecule drugs, the production of neutralizing antibodies and hypersensitivity to the

injection site may occur. Additionally, since monoclonal antibodies cross the placenta in the second half of pregnancy, there is a possibility that they may affect the growth of the fetus and placenta.

In order to reduce immunogenicity *in vivo,* chimeric, humanized or fully humanized monoclonal antibodies are being generated, the sequences of which are partially or fully human derived.

Therapeutic antibodies are being modified to optimize their *in vivo* functions, including complement activation, binding to Fc receptors to activate phagocytosis, ADCC, inhibitory signaling, and extending lifespan in the blood.

Meanwhile, human epidermal growth factor receptor (HER/EGFR/ERBB) includes EGFR, HER2, FolR, PSMA or Trop-2.

EGFR is the first growth factor receptor to be identified among receptor tyrosine kinases, and is known to be overexpressed in more than 70% of non-small cell lung cancer, and such EGFR overexpression is associated with poor prognosis. When EGFR binds to a ligand, receptor dimerization occurs, followed by autophosphrylation of the activation loop of the intracellular tyrosine kinase domain, and it activates key signaling pathways of oncogenes such as cell proliferation, cell survival, angiogenesis and metastasis through pathways such as mitogen activated protein kinase (MAPK) and Akt. Methods for blocking EGFR include two methods of using EGFR monoclonal antibodies that interfere with the binding of ligands to EFGR outside the cell, and using EGFR-TKI, which is a small molecule that competitively interferes with ATP during the phosphorylation process of the EGFR tyrosine kinase domain within cells.

It has been shown that the use of EGFR-TKI causes apoptosis and produces rapid effects.

Erlotinib and Gefitinib were developed as tyrosine kinase inhibitors and are used as treatments for non-small cell lung cancer, and EGFR mutations are known as biomarkers.

It is known that EGFR mutation selectively activates the Akt and STAT pathways, which promote cell promotion and anti-apoptosis, among the EGFR downstream signaling pathways, whereas it has no effect on the ERK pathway, which is related to cell proliferation.

In addition, Gefitinib and Erlotinib are used as drugs for EGFR mutation type late-stage non-small cell lung cancer. Panitumumab and Cetuxcimab have been developed as antibody drugs targeting EGFR and are used for the treatment of EGFR wild-type metastatic colorectal cancer. In particular, Cetuximab is an anti-EGFR antibody targeting the extracellular domain of EGFR and has been approved for the treatment of end-stage colon cancer and squamous cell carcinoma, and Panitumumab has been approved for the treatment of metastatic colon cancer. Anti-EGFR treatments are known to have KRAS mutations as a biomarker, and according to recent reports, they are known to show higher survival rates in p16-negative squamous cell carcinoma.

*In vitro,* cetuximab, which is a monoclonal antibody to EGFR, is active in cell lines that are resistant to EGFR-TKI. ABX-EGF has been developed, and trastuzumab targeting Her-2/neu (erbB2) has been developed.

Cetuximab (C225, Erbitux) is a murine/human chimeric monoclonal antibody that binds to the extracellular receptor domain III of EGFR and blocks the dimerization of EGFR. Cetuximab is known to be more effective in combination therapy with existing cytotoxic anticancer therapies than as single therapy. In a phase 1 study in combination with cisplatin, it was confirmed that there was no cumulative toxicity with cetuximab at a dose of 200 to 400 mg/$m^2$, and in a phase 2 study targeting patients with recurrent non-small cell lung cancer whose EGFR expression was +1 or higher by immunohistochemistry, combination therapy with docetaxel showed encouraging results, with a response rate of 25% and a disease control rate of over 60%. Based on this, a phase 3 study on combination therapy with docetaxel or pemetrexed has been conducted. Cetuximab is currently approved for colorectal cancer and is reported to have a synergistic effect as a combination therapy with radiotherapy for locally advanced head and neck cancer.

Unlike cetuximab, ABX-EGF is a fully humanized IgG2 monoclonal antibody and is known to have higher affinity for EGFR than cetuximab. Although skin toxicity was reported as the most common toxicity in phase 1 studies, it was not a serious problem, and a phase 3 study was conducted on combination therapy with paclitaxel/carboplatin.

Molecular-targeted anticancer drugs are expected to provide effective treatment because they focus on individual cancer-specific cells or genes.

HER2 (human epidermal growth factor receptor-2), which is known as a receptor tyrosine kinase, is known to be overexpressed in breast cancer, and for this reason, it is proposed as a biomarker expected to be effective in drugs targeting HER2. In fact, three drugs developed to target HER2, Trastuzumab, Pertuzumab and Trastuzubmab emtansine, have been approved for the treatment of HER-2-positive breast cancer.

Trastuzumab (Herceptin) is a chimeric monoclonal antibody against HER2/neu (erbB2) and is an FDA-approved drug for metastatic breast cancer. To date, no noteworthy results have been reported in non-small cell lung cancer, and it is because Her2 expression is not observed at a significant frequency in non-small cell lung cancer patients.

HER2 overexpression in breast cancer is known to play a role in promoting cell survival, proliferation, angiogenesis, invasion and metastasis. In addition, breast cancer has been shown to be resistant to traditional systemic chemotherapy and has been predicted to have a poor prognosis, thereby making it a target for oncology treatment. Therefore, before deciding on chemotherapy for metastatic breast cancer patients, a test for HER2 receptor must be performed, and HER2 overexpression is evaluated for overexpression by using immunohistochemical (IHC) stain (3+) or fluorescence in-situ hybridization (FISH) or silver in-situ hybridization (SISH) for HER2 IHC (2+).

Trastuzumab was developed in 1998 as a humanized monoclonal antibody targeting the extracellular domain of the HER2 protein and was first approved by the FDA.

The mechanism by which Trastuzumab acts on the HER2 protein is that, firstly, it inhibits cancer cell growth by reducing the amount of overexpressed HER2, and although this causes apoptosis in only a small number of cancer cells, it is known that apoptosis increases significantly when administered in combination with cytotoxic anticancer drugs. Secondly, it helps cancer-killing cells such as NK cells and macrophages to kill HER2-positive breast cancer cells.

Since the formation of new blood vessels is involved in tumor growth, tumor cells promote angiogenesis. The substance required for this process is vascular endothelial growth factor (VEGF), and the VEGF receptor is overexpressed in tumor blood vessels rather than normal blood vessels. Bevacizumab is a recombinant humanized murine monoclonal antibody that binds to VEGF and prevents it from binding to the receptor, thereby inhibiting neovascularization and exhibiting anti-cancer effects.

In order to provide an EGFR ADC using an antibody with a dissociation constant $K_d$ value of $1*10^{-8}$ M or more to less than $1*10^{-6}$ M according to the present invention, instead of Cetuximab which has a $K_d$ value of $1.8*10^{-9}$ M, Nimotuzumab which has a $K_d$ value of $1*10^{-8}$ M or more to less than $1*10^{-6}$ M, for example, $2.9*10^{-8}$M, may be used.

In order to provide a Trop-2 ADC using an antibody with a dissociation constant $K_d$ value of $1*10^{-8}$ M or more to less than $1*10^{-6}$ M according to the present invention, by changing the amino acids of the Fv sequence one by one from Sacituzumab which has a $K_d$ value of $1.7*10^{-9}$ M, an antibody (Sacituzumab-1) having a $K_d$ value of $1*10^{-8}$ M or more to less than $1*10^{-6}$ M, for example, $4*10^{-8}$ M, may be produced.

In order to provide Her2 ADC using an antibody with a dissociation constant $K_d$ value of $1*10^{-8}$ M or more to less than $1*10^{-6}$ M according to the present invention, by changing the amino acids of the Fv sequence one by one from Trastuzumab which has a $K_d$ value of $2.7*10^{-9}$ M, an antibody (Trastuzumab-1) having a $K_d$ value of $1*10^{-8}$ M or more to less than $1*10^{-6}$ M, for example, $3*10^{-8}$ M, may be produced.

Antibodies are essential components of the adaptive immune response. Immunoglobulin G (IgG) is the most common type of antibody found in the circulation and extracellular fluid. Although IgG alone can protect the body against infection directly through the activity of its antigen-binding domain, most IgG immune functions are mediated through proteins and receptors expressed by specialized cell subsets that bind to the fragment crystalline (Fc) region of IgG. Fc gamma (γ) receptors (FcγRs) belong to a broad protein family that now includes classical membrane-bound surface receptors as well as atypical intracellular receptors and cytoplasmic glycoproteins. Among atypical FcγRs, the neonatal Fc receptor (FcRn) is gaining increasing notoriety due to its intimate influence on IgG biology and its ability to also bind albumin. FcRn functions as a recycling or transcytosis receptor that is responsible for maintaining IgG and albumin in the circulation and transporting these two ligands bidirectionally across polarized cell barriers. More recently, FcRn has been evaluated to act as an immune receptor by interacting with peptides derived from IgG immune complexes (ICs) and promoting antigen presentation.

Antibodies bind to targets in the bloodstream and are taken up by cells. Once within the endosomal compartment, the antibody is protected from degradation by FcRn and recycled. In this way, the long half-life of the therapeutic antibody is preserved, thereby making it more effective even at substoichiometric levels.

Alteration of the hinge and Fc sequences by mutations is being performed to improve the desired functional properties of antibodies.

For example, the Fc region was engineered to produce antibodies with a 50-fold higher binding affinity for the C1q complement component, thereby allowing a single IgG antibody to activate complement-mediated tumor cell killing.

Different amino acid substitutions in the Fc region recognized by FcRn in vascular endothelial cells can increase the half-life of IgG in the circulation. For many therapeutic antibodies, extending their lifespan in the body may be of great help to patients in terms of antibody effectiveness, reduced number of injections, and reduced treatment costs.

Therapeutic immunoglobulin G (IgG) antibodies have a relatively long half-life because the neonatal Fc receptor (FcRn) binds to IgG Fc at the acidic pH of the endosome and protects IgG from degradation. In order to further extend the half-life, amino acid substitution antibodies with high affinity for FcRn are being developed, and one such therapeutic antibody (ravulizumab) has been approved.

As the use of monoclonal antibodies (mAbs) expands as a treatment for a variety of human diseases, including chronic inflammation, infection, cancer, autoimmune diseases, cardiovascular disease and transplant medicine, FcRn has emerged as a key regulator of mAb efficacy. This is directly related to the persistence of therapeutic antibodies in the bloodstream, which in turn may increase localization to the target site. In order to ensure the long circulating half-life of IgG, pH-dependent binding and FcRn-dependent recycling are important. Importantly, limited binding at neutral pH is required for the adequate release of IgG from cells and increased mAb affinity for FcRn at acidic pH correlates with prolonged half-life. Therefore, engineering of IgG Fc to optimize pH-dependent binding to FcRn was developed to tune pharmacokinetics and increase mAb half-life. For example, the MST mutation (Met252Tyr/Ser254Thr/Thr256Glu) increased IgG persistence by up to 5 folds in humans and monkeys. In phase 2 clinical trials, the IgG MST variant showed a half-life of 80 to 120 days. Similarly, the MN (Met428Leu/Asn434Ser) mutation adjacent to the critical FcRn binding site of the IgG Fc shows the potential to prolong the IgG half-life for therapeutic antibodies.

For example, by screening a library introducing random amino acid mutations into specific regions of the constant domain of human IgG molecules, mutations that increase the affinity of IgG molecules for FcRn are collected and characterized, and thus, it is possible to produce antibodies with extended serum half-life.

In order to provide an antibody or an antigen-binding site-containing fragment (B) thereof, which is used in the immunoconjugate of the present invention and has a $K_d$ value of $1*10^{-8}$ M or more to less than $1*10^{-6}$ M or has a higher binding affinity when binding bivalently than when binding monovalently, various antibodies and variously engineered antibodies described above may be utilized.

**[Pharmaceutically Acceptable Salt]**

In the present specification, pharmaceutically acceptable salts refer to salts that are commonly used in the pharmaceutical industry, and for example, there are salts of inorganic ions including sodium, potassium, calcium, magnesium, lithium, copper, manganese, zinc, iron and the like, and other than the above, there are salts of organic acids such as ascorbic acid, citric acid, tartaric acid, lactic acid, maleic acid, malonic acid, fumaric acid, glycolic acid, succinic acid, propionic acid, acetic acid, orotate acid, and acetylsalicylic acid, and salts of amino acids such as lysine, arginine and guanidine. In addition, there are also salts of organic ions such as tetramethyl ammonium, tetraethyl ammonium, tetrapropyl ammonium, tetrabutyl ammonium, benzyl trimethyl ammonium, benzethonium and the like that can be used in pharmaceutical reactions, purification and separation processes. However, the types of salts meant in the present invention are not limited to these salts listed above.

**[Various Carrier-Drug Conjugates]**

It is very important to easily deliver an ADC or other targeted-drug conjugate material into cancer tissue, and the [camptothecin-based drug]-[linker] conjugate according to the present invention may be applied to various types of drug carriers other than antibodies, and unlike antibodies, it may be utilized for a variety of applications by using a carrier that can penetrate deep into cancer tissue and has characteristics that facilitate CMC.

The carrier may be an antigen binding site of an antibody, a peptide, a repebody and/or an aptamer.

[Table 1]

| | **ADC** | **PDC** | **ApDC** | **Repebody** |
|---|---|---|---|---|
| Size | > 150 kDa | 2-5 kDa | > 20 kDa | 30~50 kDa |
| Advantage | • A next-generation anticancer drug that is gaining attention in the global market because it can simultaneously exhibit two functions of target selectivity of antibodies and cytotoxicity of drugs. | • High tissue permeability<br>• Low immunogenicity<br>• Precise control of DAR possible<br>• Introduction of modified amino acids such as non-natural amino acids possible | • High tissue permeability<br>• Low immunogenicity<br>• Precise control of DAR possible<br>• Easy to chemically modify | • Relatively low immunogenicity<br>• Relatively easy DAR regulation<br>• High structural stability at high temperature and pH |
| Disadvantage | • High immunogenicity<br>• Difficulty in regulating DAR<br>• Low tissue permeability | • Low *in vivo* stability | • Low *in vivo* stability | • In the early stages of development, related research and optimization technology are required. |

The Aptamer-Drug Conjugate (ApDC) is an aptamer introduced instead of an antibody in ADC. Aptamers are single-stranded nucleic acids with a three-dimensional structure. It is discovered through the 'SELEX' (Systematic Evolution of Ligands by Exponential enrichment) process. Selex is a technology that obtains functional nucleic acids that bind to target protein molecules in a compound library.

Since aptamers can bind to targets very strongly and selectively, they are also called chemical antibodies. Aptamers are about 20 kDa in size and are known to have excellent cell penetration and low immunogenicity compared to antibodies.

Since aptamers can be chemically synthesized, precise design of the conjugation location and number of drug conjugates is possible when producing aptamer-drug conjugates. The production cost is lower than that of ADC.

Since aptamers are generally made of natural nucleic acids, they are degraded by nucleic acid degrading enzymes in the body, making them less stable *in vivo*. However, by taking advantage of the ease of chemical modification of aptamers, the limitations on the stability of modified aptamers may be overcome.

The Peptide-Drug Conjugate (PDC) is a form of ADC in which peptides are introduced instead of antibodies. Peptides are composed of amino acids and have a size ranging from 500 to 5,000 Da (Dalton). This is a very small size compared to antibodies of 150 kDa (kilodaltons) or more. Therefore, peptide-based PDC has superior cell penetration ability compared to ADC, and the possibility of immunogenicity is very low. Additionally, peptides can be chemically synthesized. For this reason, PDC not only has a very low production cost, but also allows precise control of the conjugation position and ratio of peptide and drug.

In general, peptides are easily degraded by proteolytic enzymes and thus have a short biological half-life. In order to overcome these limitations of peptide-based drug conjugates, strategies using modified peptides such as cyclic peptides and introduction of non-natural amino acids are being proposed.

Repebody is a type of artificial antibody that does not have an antibody skeleton but has the function of recognizing antigens like an antibody. Repebody that is specific to target proteins may be discovered through phage display technology.

Phage display is a technology that expresses desired proteins on the surface of bacteriophage. Repebody is about 30kDa in size, which is 20% of an antibody drug. Therefore, it is known to have relatively low immunogenicity and improved cell penetration compared to antibodies. In addition, it is expected that structural stability can be improved by controlling the thermal and pH stability of repebody. Compared to antibodies, production costs are also assessed to be relatively low. Because of these advantages of repebody, interest in the development of repebody-drug conjugates (repebody-DC) as a strategy to replace antibodies with repebody is increasing.

Therefore, the method for producing the carrier-drug conjugate of the present invention is characterized by

using the [camptothecin drug]-[acid sensitive linker of Chemical Formula 2] conjugate or a pharmaceutically acceptable salt thereof according to the present invention, so as to link one or more camptothecin drugs to a carrier through the linker of Chemical Formula 2.

**[Pharmaceutical Composition for Preventing or Treating Cancer]**

The present invention provides a pharmaceutical composition for preventing or treating cancer, including the above-described immunoconjugate according to the present invention or a pharmaceutically acceptable salt thereof as an active ingredient.

According to one embodiment of the present invention, provided is a method for treating or preventing cancer, including administering a therapeutically effective amount of the immunoconjugate to a subject in need thereof. The subject may be a mammal, including humans.

The immunoconjugate of the present invention binds specifically to the antigen of cancer cells and exhibits cytotoxicity by releasing the drug inside and outside the cancer cells, and thus, it can be advantageously used in the treatment or prevention of cancer. The anticancer activity of the immunoconjugate of the present invention is as described above.

In the present invention, the cancer may be solid cancer or hematological cancer. For example, it may be at least one selected from the group consisting of pseudomyxoma, intrahepatic biliary tract cancer, hepatoblastoma, liver cancer, thyroid cancer, colon cancer, testicular cancer, myelodysplastic syndrome, glioblastoma, oral cancer, oral cavity cancer, mycosis fungoides, acute myeloid leukemia, acute lymphocytic leukemia, basal cell carcinoma, ovary epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, sinonasal cancer, non-small cell lung cancer, tongue cancer, astrocytoma, small cell lung cancer, pediatric brain cancer, pediatric lymphoma, childhood leukemia, small intestine cancer, meningioma, esophageal cancer, glioma, renal pelvis cancer, kidney cancer, heart cancer, duodenum cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, stomach cancer, gastric carcinoid, gastrointestinal stromal cancer, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleura cancer, blood cancer and thymic cancer, but is not limited thereto. Additionally, the cancer includes not only primary cancer

but also metastatic cancer.

As used herein, the term "therapeutically effective amount" refers to the amount of the immunoconjugate that is effective for treating or preventing cancer. Specifically, "therapeutically effective amount" means an amount that is sufficient to treat the disease with a reasonable benefit/risk ratio applicable to medical treatment, and the effective dose level may be determined based on factors including the type and severity of the subject, age, gender, type of disease, activity of the drug, sensitivity to the drug, time of administration, route of administration and excretion rate, duration of treatment, concurrent medications, and other factors well known in the medical field. The pharmaceutical composition of the present invention may be administered as an individual therapeutic agent or in combination with other therapeutic agents, and may be administered sequentially or simultaneously with commercially available therapeutic agents. In addition, it can be administered single or multiple times. Considering all of the above factors, it is important to administer the amount that can achieve the maximum effect with the minimum amount without side effects. Since the immunoconjugate of the present invention exhibits a dose-dependent effect, the administered dose may be easily determined by those skilled in the art depending on various factors such as the patient's condition, age, gender and complications. Since the active ingredient of the pharmaceutical composition of the present invention has excellent safety, it may be used at a dose exceeding the determined dosage.

In addition, according to one embodiment of the present invention, the present invention provides the use of the immunoconjugate for use in the manufacture of a medicament for use in the treatment or prevention of cancer. The immunoconjugate for the manufacture of a medicament may be mixed with acceptable auxiliaries, diluents, carriers and the like, and may be prepared as a complex preparation with other active agents to have a synergistic effect of the active ingredients.

Matters that are mentioned in the uses, compositions and treatment methods of the present invention apply equally unless they contradict each other.

## ADVANTAGEOUS EFFECTS

The present invention may provide an antibody-drug conjugate (ADC) that uses an antibody with a $K_d$ value of $1*10^{-8}$ M or more to less than $1*10^{-6}$ M, which indicates the degree to which the antibody binds to a drug target, and a camptothecin-series Topoisomerase I inhibitor that degrades DDX5 protein as a payload, thereby demonstrating stronger efficacy and ensuring safety compared to existing ADCs.

[An antibody or antigen-binding site-containing fragment thereof that has a higher binding affinity to an epitope of a cell surface protein when binding divalently than when binding monovalently] used in the present invention can solve the problem of on-target toxicity by reversely using the binding affinity for antigens, which allows for greater internalization as the binding force to the epitope of the antigen is stronger, due to the characteristic of antibodies that internalize via receptors, when designed to target [cell surface proteins expressed on both normal and cancer cells], and can also increase the therapeutic effect through a mechanism called apoptosis through combined use with [a camptothecin-based drug that degrades DDX5 protein].

According to the present invention, the immunoconjugates that use an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently can maximize target treatment effects by improving target tissue penetration.

## DESCRIPTION OF DRAWINGS

FIG. 1 shows the synthetic design concept of a novel active camptothecin derivative (a) with a dual mechanism of action (MoA) that degrades the oncoprotein DDX5 along with the ability to inhibit type I topoisomerase through improved structure of FL118.

FIG. 2 shows the mechanism of action of cell membrane receptors (HER2, EGFR, and other receptor tyrosine kinases) to which growth factors bind.

FIG. 3 is a mimetic diagram showing the efficient drug release mechanism and anticancer action mechanism in an ADC (e.g., PBX-001) using a FL118-acid sensitive linker.

FIG. 4 shows the analysis results confirming that DAR=8 of FL118-CL2A linker-sacituzumab (PBX-001).

FIG. 5 shows results showing that the drug FL118 and Exatecan have better efficacy against *in vitro* cytotoxicity compared to SN-38.

FIG. 6 is a Western blot result showing the degree of inhibition of anti-apoptotic proteins in HCT-8 and FaDu cell lines,

showing that the FL118 drug and the exatecan drug have better efficacy in downregulating tumor proteins compared to the SN-38 drug.

FIGS. 7 and 8 are Western blot results showing whether/the degree of degradation of DDX5 and p-DDX5 proteins of various camptothecin drugs (FL118 drug, SN-38 drug, Exatecan drug, PBX-7011, PBX-7014, PBX-7016) in A549 cell line, and showing the degree of inhibition of various anti-apoptotic proteins. FIG. 8 is the results of the western blot experiment conducted on the A549 cell line (FIG. 7), which graphically quantifies the concentration.

FIG. 9 shows the results of comparative evaluation of *in vitro* cell viability for various camptothecin-based drugs in FaDu cell line or A549 cell line.

FIG. 10 shows the results of comparative evaluation of *in vitro* cell viability for various camptothecin-based drugs in MDA-MB-453 (HER2++) cell line and FaDu (HER2+) cell line.

FIG. 11 is a result showing the mechanism by which FL118-CL2A linker-sacituzumab (PBX-001) overcomes the ABCG2 efflux pump. That is, cells with an ABCG2 efflux pump are resistant to Trodelvy but are not affected by PBX-001.

FIGS. 12 and 13 show evaluation results of FL118-CL2A linker-sacituzumab (PBX-001, DAR 8) in the FaDu Xenograft model, showing that it exerts excellent anticancer efficacy without weight loss in the *in vivo* Xenograft model.

FIG. 14 shows the results confirming the ADC production of Cetumximab-CL2A-FL118 (DAR 8) using LC-MS.

FIG. 15 shows the results of confirming the ADC production of nimotuzumab-CL2A-FL118 (DAR 8) using LC-MS.

FIG. 16 shows the process of selecting cell lines for *in vitro* and *in vivo* experiments on EGFR targets by confirming EGFR expression in various cell lines.

FIG. 17 shows the results of comparing the binding affinity to EGFR of nimotuzumab and Nimotuzumab (YTE), which is modified to extend its serum half-life, and ADC which is conjugated with the FL118 drug-CL2A linker or the PBX-7016 drug-GGFG linker.

FIG. 18 shows the results of performing *in vitro* assays of various Nimotuzumab ADCs in EGFR positive and negative cells.

FIG. 19 shows the results of performing *in vitro* assay of Nimotuzumab ADC and Cetuximab ADC in EGFR positive and negative cells.

FIG. 20 shows the results of in vitro assay of Nimotuzumab ADC and Nimotuzumab (YTE) ADC in EGFR positive and negative cells.

FIG. 21 shows the *in vivo* evaluation results of Cetuximab-CL2A-FL118, which is an EGFR target ADC, in the MDA-MB-468 model.

FIG. 22 shows the *in vivo* evaluation results of Cetuximab-CL2A-FL118 and Nimotuzumab-CL2A-FL118, which are EGFR target ADCs in the MDA-MB-468 model.

FIG. 23 shows the *in vivo* evaluation results of Nimotuzumab-GGFG-FL118/PBX-7016 and Nimotuzumab (YTE)-GGFG-FL118/PBX-7016, which are EGFR target ADCs produced in Example 5 in the MDA-MB-468 model.

FIG. 24 shows the results of carboxylate to lactone form conversion in pH 7.4.

FIG. 25 shows the results of carboxylate to lactone form conversion in pH 6.0.

FIG. 26 shows the mechanism of ADC toxicity (Source: Cancers 2023, 15 (3), 713; https://doi.org/10.3390/cancers15030713).

FIG. 27 exemplifies the operating mechanism of various self-immolative spacers.

FIG. 28 is a conceptual diagram of monovalent and divalent or multivalent bonds.

FIG. 29 is a conceptual diagram of a cell surface receptor binding to a multivalent antigen to form a cluster.

## MODES OF THE INVENTION

Hereinafter, the present invention will be described in more detail through examples. However, the following examples are only intended to clearly illustrate the technical features of the present invention and do not limit the scope of protection of the present invention.

### Preparation Example 1: Synthesis of active camptothecin derivatives (PBX-7011 and PBX-7012) of Chemical Formula 3 or Chemical Formula 3-1

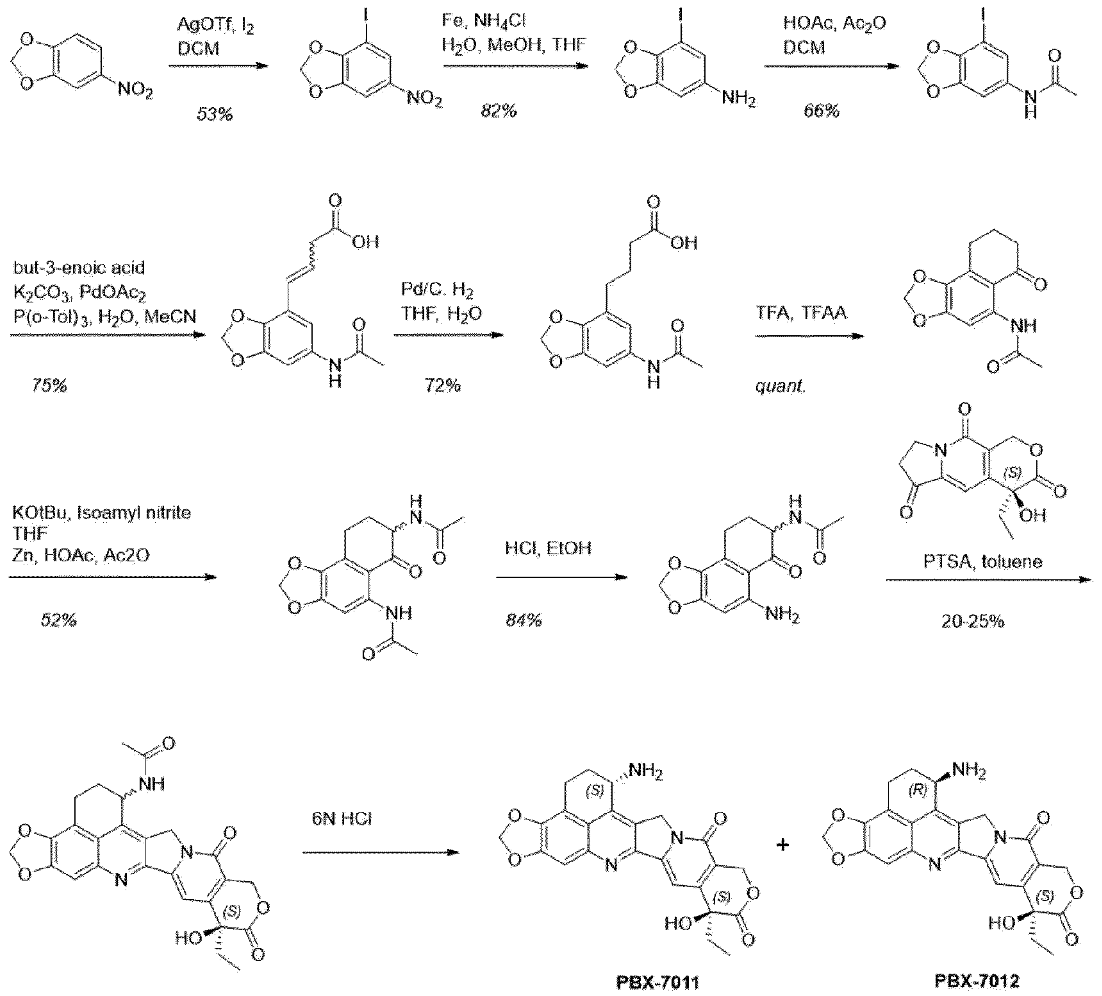


PBX-7011 PBX-7012

**1-1. Synthesis of Compound 2**

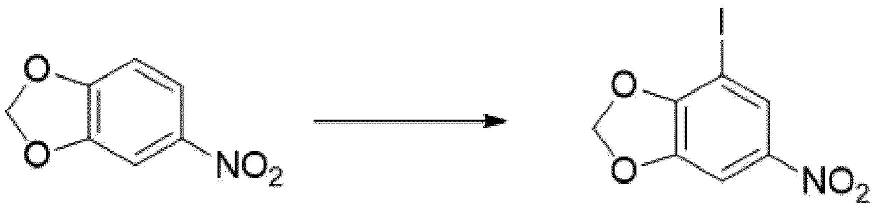

1 2

Silver triflate (57.7 g, 224 mmol) and iodine (57.0 g, 224 mmol) were added to a solution of 5-nitrobenzo[d][1,3]dioxole (25 g, 150 mmol) in a dichloromethane (748 mL) flask. This solution was stirred in the dark at room temperature under $N_2$ atmosphere.

AgI was removed by filtration, and the solid was washed with dichloromethane (100 mL). The solvent was removed under reduced pressure, and the residue was partitioned between EtOAc (250 mL) and 5% (v/v) $NH_4OH/H_2O$ solution (200 mL). The organic layer was separated, washed with 1M $Na_2SO_3$(5 x 200 mL) and brine (200 mL), dried over $Na_2SO_4$, treated with activated carbon, and filtered through Celite. The solvent was evaporated under reduced pressure to provide the crude as a brown solid. This was triturated in ice-cold EtOH (400 mL), filtered, and the solid was washed with ice-cold EtOH (100 mL) to provide the product as a light brown-gray solid (14.3 g). The solvent was removed from the filtrate, and the crude was triturated a second time in ice-cold EtOH (300 mL). The solid was collected by filtration and

washed with EtOH (50 mL) to provide the additional product (10.2 g) as a dark brown-gray solid. Both batches were used as is without further purification.
SC_ACID: m/z 294.2 $[M+H]^+$

**1-2. Synthesis of Compound 3**

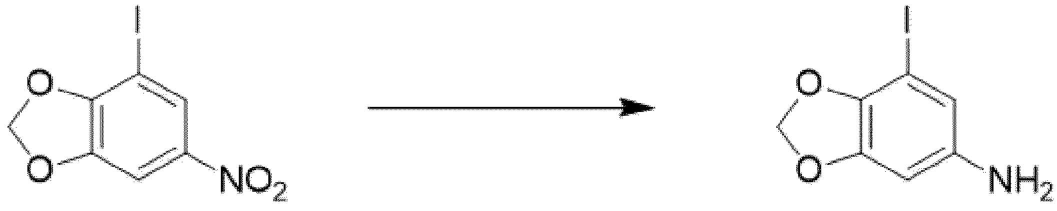

2 3

Iron powder (6.67 g, 119 mmol) and ammonium chloride (6.39 g, 119 mmol) were added to a suspension of 4-iodo-6-nitrobenzo[d][1,3]dioxole (8.75 g, 29.9 mmol) in a mixture of water (80 mL) methanol (40.0 mL) and tetrahydrofuran (40.0 mL). The suspension was heated to 75°C and stirred for 16.5 hours. The reaction mixture was cooled to room temperature and concentrated under reduced pressure. The resulting black solid was suspended in ethyl acetate (100 mL), and the solution was decanted. The residue was washed with EtOAc (3 x 50 mL). The mixture was transferred to a separatory funnel, water was added, and the aqueous layer was removed. The organic layer was washed with saturated aqueous $NaHCO_3$ solution (150 mL) and brine (150 mL). The organic layer was dried over $Na_2SO_4$, and the solvent was removed under reduced pressure to obtain a brown solid (4.75 g, 60% yield). The iron residue was washed thoroughly with ethyl acetate to recover the additional product. Subsequently, the organic fraction was washed with saturated aqueous $NaHCO_3$ solution (150 mL), brine (150 mL) and dried over $Na_2SO_4$. The solvent was removed under reduced pressure to obtain a brown solid (1.74 g, 22% yield).
SC_ACID: m/z 264.0 $[M+H]^+$

**1-3. Synthesis of Compound 4**

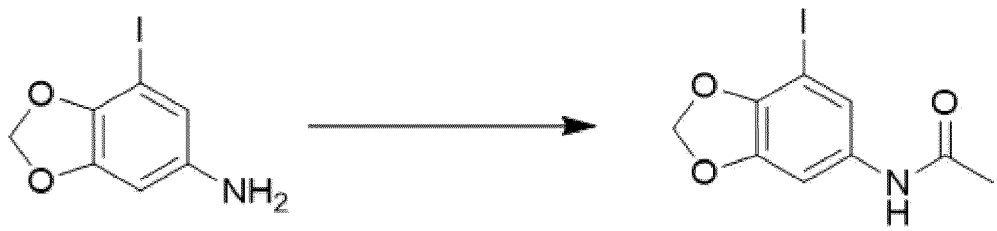

3 4

Acetic anhydride (2.75 mL, 29.2 mmol) and triethylamine (4.06 mL, 29.2 mmol) were added to a solution of 7-iodobenzo[d][1,3]dioxol-5-amine (6.39 g, 24.29 mmol) in dichloromethane (49 mL). The reaction mixture was stirred at room temperature overnight. After a few hours, additional DCM (10 ml) was added. The suspension was filtered through a sintered funnel and washed with ice-cold DCM (10 mL). The product was further dried under reduced pressure to obtain a light gray solid (4.46 g). The filtrate was concentrated under reduced pressure, and the residue was dissolved in EtOAc, washed with water and brine, dried over $Na_2SO_4$ and concentrated under reduced pressure to obtain a brown solid (~3 g). The brown solid was purified by flash column chromatography (80 g Si, 0-100% ethyl acetate in heptane). All batches of product were triturated in ice-cold EtOAc (5-10 mL). The two batches were combined and further dried to obtain a grey-white solid.

Total yield: 5.0 g, 66%

SC_ACID: m/z 306.0 $[M+H]^+$
$^1$HNMR (400 MHz, DMSO-d6) δ 9.88 (s, 1H), 7.39 (d, *J* = 1.9 Hz, 1H), 7.17 (d, *J* = 1.9 Hz, 1H), 6.04 (s, 2H), 1.99 (s, 3H).

**1-4. Synthesis of Compound 5**

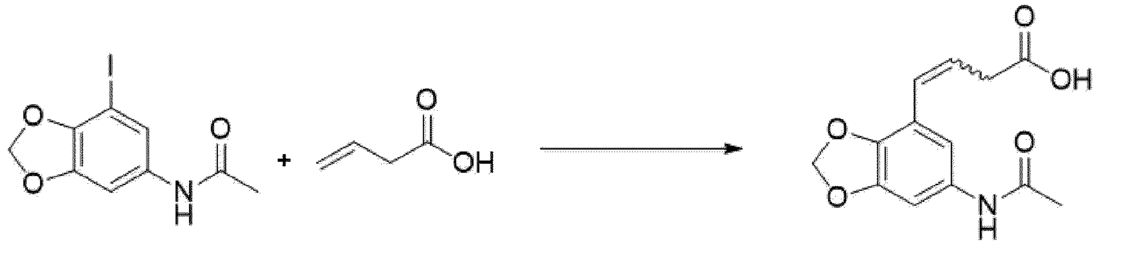

4 5

A slurry of N-(7-iodobenzo[d][1,3]dioxol-5-yl)acetamide (5.06 g, 16.59 mmol), but-3-enoic acid (1.69 mL, 19.90 mmol) and potassium carbonate (2.98 g, 21.56 mmol) in acetonitrile (40 mL) was cooled to 0 to 5 °C in a 3-neck flask equipped with a condenser. Water (13.33 mL) was slowly added to generate gas. Once gas generation ceased, the mixture was degassed with Ar for 30 minutes. Tri-o-tolylphosphine (0.505 g, 1.659 mmol) and palladium acetate (0.186 g, 0.829 mmol) were added, and the mixture was degassed again for 30 minutes and then heated to reflux under Ar. The reaction product was cooled to room temperature and filtered through Celite. The filter cake was washed with $H_2O$ and EtOAc. The organic solvent was removed from the filtrate under vacuum, and the aqueous material was acidified to a concentrated solution. HCl until pH = 1 to 2. The aqueous layer was extracted with EtOAc, and the combined organic phases were washed with brine, dried over $Na_2SO_4$, filtered and concentrated under reduced pressure to obtain a crude product. The crude material was triturated in ice-cold EtOAc (30 mL), and the solid was collected by filtration to product the product as a brown solid. The mother liquor was concentrated under reduced pressure and purified via flash chromatography (80 g Si, 0 - 100% EtOAc in heptane). The product fractions were concentrated under reduced pressure to obtain a light brown foam. Total yield: 3.46 g, 75%. A mixture of E/Z isomers was obtained.
SC_ACID: m/z 264.4 $[M+H]^+$

**1-5. Synthesis of Compound 6**

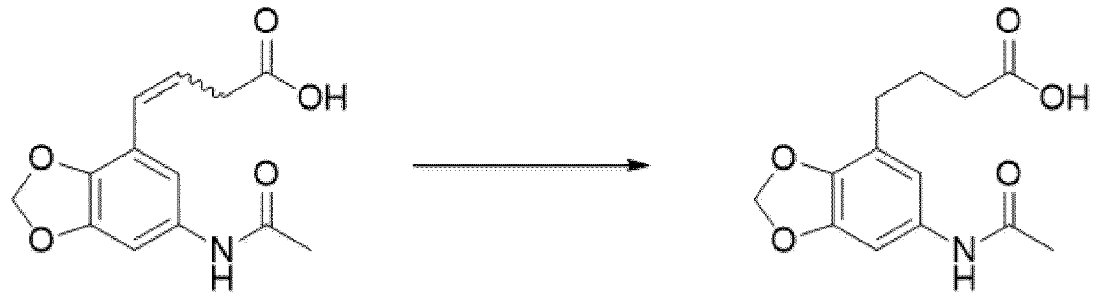

5 6

A suspension of 4-(6-acetamidobenzo[d][1,3]dioxol-4-yl)but-3-enoic acid (3.47 g, 13.18 mmol) in tetrahydrofuran (50 mL)/water (50 mL) was degassed with $N_2$ for 15 minutes. Pd/C (2.97 g, 1.397 mmol) was added, and the suspension was degassed with $H_2$ for 5 minutes before heating to 40°C under $H_2$ (balloon). After 24 hours, the reaction mixture was added with nitrogen and additional Pd/C (2.97 g, 1.397 mmol). The reaction mixture was bubbled with hydrogen for 10 minutes and stirred under hydrogen at 45°C overnight. The reaction was filtered through Celite. The filter cake was washed with $H_2O$ (50 mL) and EtOAc (50 mL), and the organic solvents were removed from the filtrate under vacuum. The aqueous solution was acidified with concentrated HCl until pH = 1, and the dark brown/green precipitate was collected by filtration

through a sintered funnel. The aqueous filtrate was extracted. The combined organic phases were washed with brine, dried over $Na_2SO_4$ and filtered, and the solvent was removed under vacuum to obtain a brown oil. Since product recovery was low, the filter cake was mixed with EtOAc (50 ml), water (200 ml) and MeOH (400 mL). A water/EtOAc flush was added to the flask containing the solids from the MeOH flush. The aqueous layer was acidified to concentrated consistency. HCl and a grey-white precipitate formed until pH = 1, which was collected by filtration through a sintered funnel. After extracting the aqueous filtrate with EtOAc (3x200 mL), LCMS showed that all product was removed from the aqueous material. The combined organic phases were washed with brine, dried over $Na_2SO_4$ and filtered, and the solvent was removed under vacuum to obtain a light brown solid. All product batches were combined and purified via flash column chromatography (40 g Si, 0-10% MeOH in DCM). The product fractions were concentrated to obtain a light brown solid. Yield: 2.52 g, 72%.

SC_ACID: 266.2 $[M+H]^+$
$^1H$ NMR (400 MHz, DMSO-d6) δ 12.07 (s, 1H), 9.78 (s, 1H), 7.14 (d, *J* = 2.0 Hz, 1H), 6.80 (d, *J* = 2.2 Hz, 1H), 5.95 (s, 2H), 2.50 - 2.46 (m, 2H), 2.23 (t, *J* = 7.4 Hz, 2H), 1.98 (s, 3H), 1.84 - 1.70 (m, 2H).

**1-6. Synthesis of Compound 7**

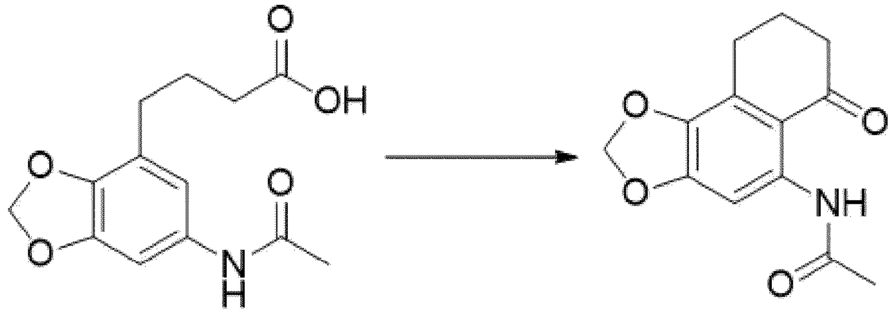

6 7

A suspension of 4-(6-acetamidobenzo[d][1,3]dioxol-4-yl)butanoic acid (150 mg, 0.565 mmol) in TFA (433 μL, 5.65 mmol) was cooled on ice. TFAA (157 μL, 1.131 mmol) was added. The mixture was stirred at 0 to 5°C for 1 hour and over time turned into a dark solution. The reaction mixture was added dropwise to ice-cold saturated aqueous $NaHCO_3$ solution (10 mL), and the aqueous solution was extracted with ethyl acetate (3 x 25 mL). The combined organic layers were dried over sat. $NaHCO_3$ and brine with $Na_2SO_4$ and filtered, and the solvent was removed in vacuum to obtain a salmon pink solid. Yield: 140mg, 100%

SC_ACID: m/z 248.2 $[M+H]^+$
$^1H$ NMR (400 MHz, DMSO-d6) δ 12.34 (s, 1H), 8.11 (s, 1H), 6.13 (s, 2H), 2.80 (t, *J* = 6.2 Hz, 2H), 2.66 - 2.58 (m, 2H), 2.12 (s, 3H), 2.01 - 1.91 (m, 2H).

**1-7. Synthesis of Compound 8**

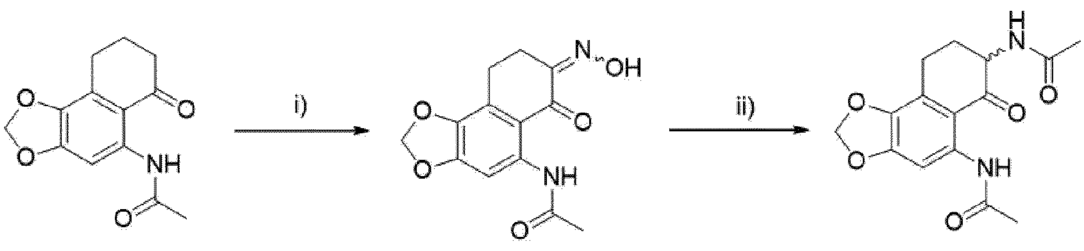


7 8

A suspension of N-(6-oxo-6,7,8,9-tetrahydronaphtho[1,2-d][1,3]dioxol-5-yl)acetamide (50 mg, 0.202 mmol) in tetrahydrofuran (1.2 mL) was cooled to 0°C, and potassium tert-butoxide (27.2 mg, 0.243 mmol) and isoamyl nitrite (35.0 μL, 0.263 mmol) were added. The dark green mixture was stirred on ice (<5°C) for 1.5 hours. Acetic acid (170 μL, 2.94 mmol), acetic anhydride (170 μL, 1.810 mmol) and zinc dust (66.1 mg, 1.011 mmol) were added to the reaction mixture. The suspension was stirred at 0°C for 2 hours. The reaction mixture was filtered over Celite and flushed with DCM. The filtrate was concentrated under reduced pressure to obtain a black oily substance. The crude product was purified via flash column chromatography (4 g Si, 0-4% MeOH in DCM). The product fractions were concentrated to obtain a gray solid (32 mg, 52%) with 80 to 90% purity. By purifying with purification MPLC, samples of higher purity can be obtained.

SC_ACID: m/z 305.4 $[M+H]^+$
$^1$H NMR (400 MHz, DMSO-d6) δ 12.10 (s, 1H), 8.21 (d, J = 8.0 Hz, 1H), 8.12 (s, 1H), 6.15 (d, J = 9.3 Hz, 2H), 4.66 - 4.56 (m, 1H), 2.93 (dd, J = 8.9, 4.0 Hz, 2H), 2.14 (s, 3H), 2.13 - 1.93 (m, 2H), 1.91 (s, 3H).

**1-8. Synthesis of Compound 9**

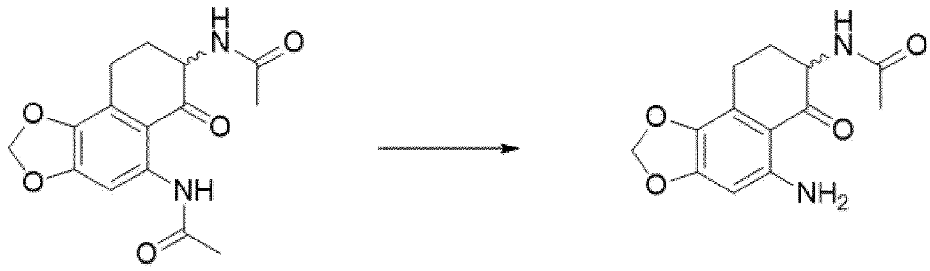

8 9

N,N'-(6-oxo-6,7,8,9-tetrahydronaphtho[1,2-d][1,3]dioxole-5,7-diyl)diacetamide (244 mg, 0.802 mmol) was suspended in 2M hydrochloric acid (4.69 mL, 9.38 mmol) in ethanol/water (5/1). The mixture was heated to 55°C for 4 hours. The black mixture was cooled to 0 to 5°C. Triethylamine (1.4 mL, 10.04 mmol) was added dropwise while stirring. Next, the mixture was diluted with EtOH and evaporated to dryness. The residue was partitioned between water and DCM. The layers were separated, and the aqueous layer was extracted once with DCM. The combined organic layers were dried over $Na_2SO_4$ and concentrated to obtain the product as a brown solid (178 mg, 73%) with 86% purity.

SC_ACID: m/z 263.0 $[M+H]^+$
$^1$H NMR (400 MHz, DMSO) δ 8.04 (d, J = 8.0 Hz, 1H), 6.20 (s, 1H), 5.94 (d, J = 6.0 Hz, 2H), 4.48 - 4.41 (m, 1H), 3.08 (s, 2H), 2.88 - 2.69 (m, 2H), 2.15 - 2.03 (m, 1H), 1.88 (s, 3H), 1.86 - 1.75 (m, 1H).

**1-9. Synthesis of Compound 10**

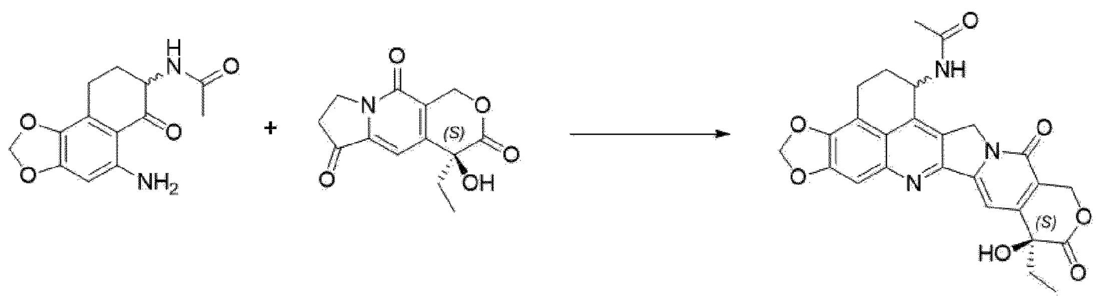

9 10

(4S)-4-ethyl-7,8-dihydro-4-hydroxy-1H-pyrano[3,4-f]indolizine-3,6,10(4H)-trione (146 mg, 0.555 mmol) and N-(5-amino-6-oxo-6,7,8,9-tetrahydronaphtho[1,2-d][1,3]dioxol-7-yl)acetamide (112 mg, 0.427 mmol) were added to dry toluene (4.5 ml) and PPTS (21 mg, 0.085 mmol), and the reaction mixture was stirred at 115°C for 40 hours.

The reaction mixture was cooled to room temperature. The suspension was diluted with 2 mL DCM and filtered. A black residue (210 mg) was obtained.

The crude product was purified by column chromatography (12 g Si, 0-7% methanol in DCM) to obtain the product (45 mg, 21%) as a brown solid.

LCMS analysis showed two diastereomers.

SC_ACID: m/z 490.2 $[M+H]^+$

$^1H$ NMR (400 MHz, DMSO-d6) δ 8.47 (t, *J* = 9.3 Hz, 1H), 7.42 (s, 1H), 7.24 (s, 1H), 6.49 (s, 1H), 6.29 (d, *J* = 5.2 Hz, 2H), 5.57 - 5.49 (m, 1H), 5.41 (s, 2H), 5.23 - 5.07 (m, 2H), 3.09 - 3.00 (m, 2H), 2.11 - 2.01 (m, 2H), 1.91 (s, 3H), 1.89 - 1.79 (m, 2H), 0.87 (t, *J* = 7.1 Hz, 3H).

**1-10. Synthesis of PBX-7011 and PBX-7012**

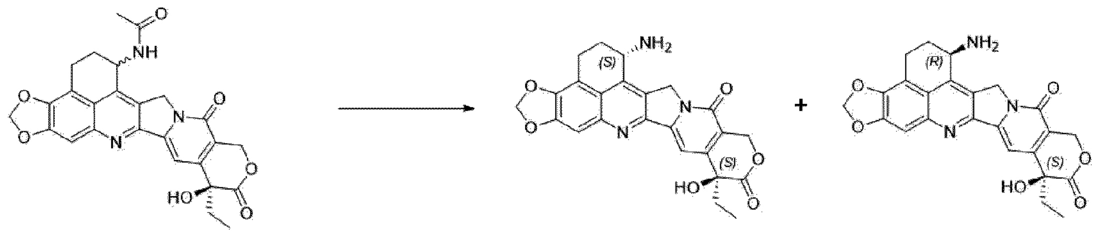

10 PBX-7011 PBX-7012

N-((10S)-10-ethyl-10-hydroxy-11,14-dioxo-2,3,10,11,14,16-hexahydro-1H,13H-benzo[de][1,3]dioxolo[4,5-g]pyrano[3',4':6,7]indolizino[1,2-b]quinolin-1-yl)acetamide (73.5 mg, 0.150 mmol) was dissolved in 1.0 mL 6N HCl and stirred at 90°C for 8 hours and then at room temperature overnight. The reaction mixture was concentrated under reduced pressure and purified by two runs through acidic preparative MPLC (Luna2-30). The fraction containing the separated diastereomers was acidified with 5 drops of 3N HCl and lyophilized to yield the product as a yellow solid.

1st eluting isomer: PBX-7011, 23 mg, 34% yield

U_AN_ACID: m/z 448.4 $[M+H]^+$

$^1H$ NMR (400 MHz, DMSO-d6) δ 8.54 (s, 3H), 7.50 (s, 1H), 7.27 (s, 1H), 6.52 (s, 1H), 6.34 (d, *J* = 13.3 Hz, 2H), 5.77 (d, *J* = 19.3 Hz, 1H), 5.44 (s, 2H), 5.37 (d, *J* = 19.3 Hz, 1H), 5.05 (s, 1H), 3.19 - 3.02 (m, 2H), 2.46 (s, 1H), 2.20 - 2.03 (m, 1H), 1.94 - 1.81 (m, 2H), 0.88 (t, *J* = 7.3 Hz, 3H).

2nd eluting isomer: PBX-7012, 28 mg, 41% yield

U_AN_ACID: m/z 448.2 $[M+H]^+$

$^1H$ NMR (400 MHz, DMSO-d6) δ 8.57 (d, *J* = 4.7 Hz, 3H), 7.51 (s, 1H), 7.27 (s, 1H), 6.52 (s, 1H), 6.34 (d, *J* = 12.2 Hz, 2H), 5.76 (d, *J* = 19.4 Hz, 1H), 5.44 (s, 2H), 5.37 (d, *J* = 19.4 Hz, 1H), 5.08 (s, 1H), 3.16 - 2.98 (m, 2H), 2.46 (s, 1H), 2.18 - 2.06 (m, 1H), 1.94 - 1.80 (m, 2H), 0.87 (t, *J* = 7.3 Hz, 3H).

**Preparation Example 2: Synthesis of active camptothecin derivatives (PBX-7014 and PBX-7015) of Chemical Formula 4 or Chemical Formula 4-1**

This example describes the synthesis of compounds PBX-7014 and PBX-7015 starting from two separated diastereomers of the Exatecan-hybrid compounds (PBX-7011 and PBX-7012).

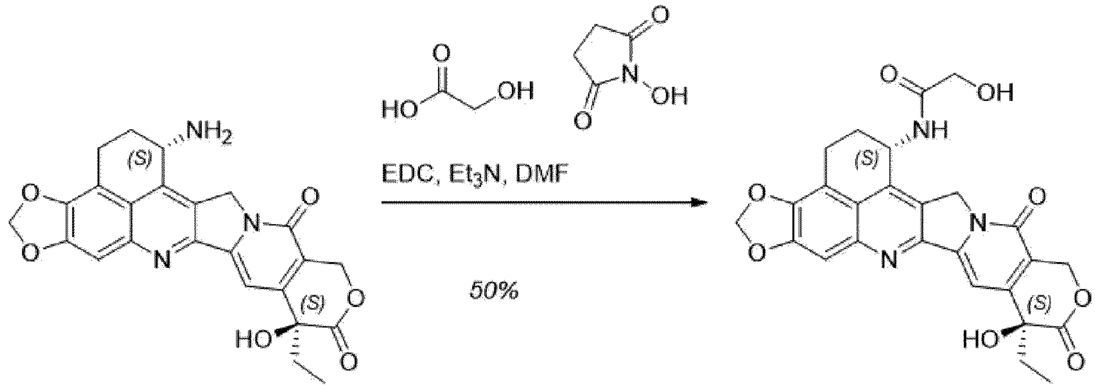


PBX-7011 PBX-7014

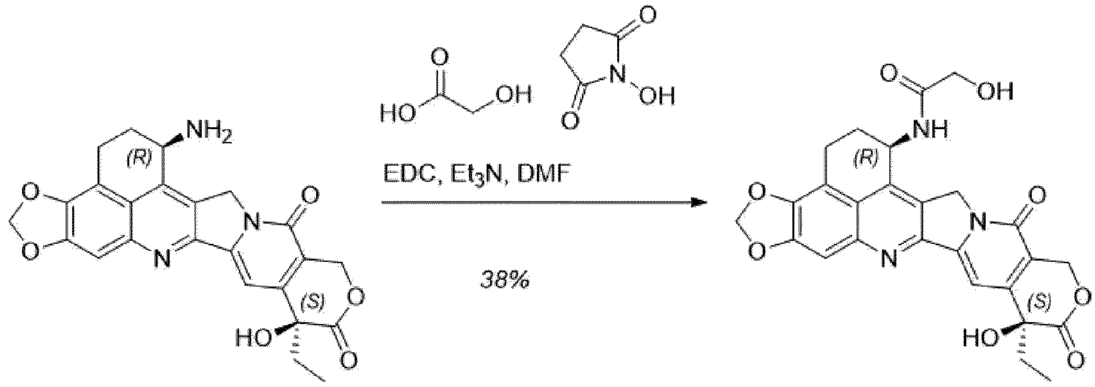


PBX-7012 PBX-7015

**2-1: Synthesis of PBX-7014**

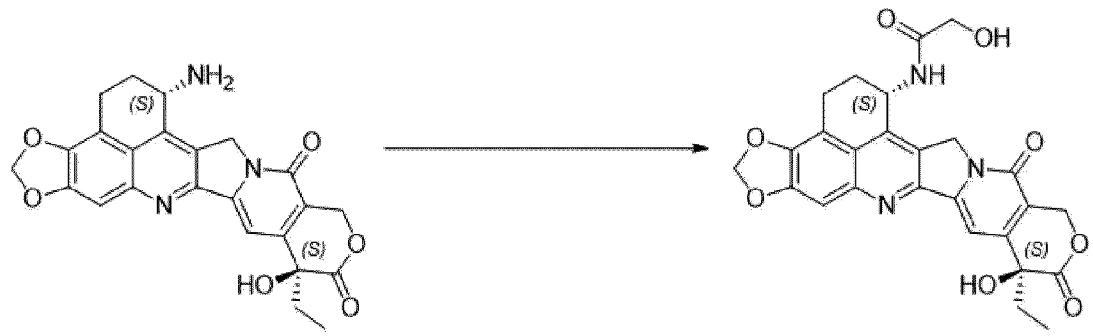

PBX-7011 PBX-7014

A stock solution of activated glycolic acid was prepared according to the following procedure: Glycolic acid (17 mg, 0.224 mmol) was dissolved in 1 mL of N,N-dimethylformamide. HOSu (25.7 mg, 0.223 mmol) and EDC (42.8 mg, 0.223 mmol) were added. The reaction mixture was stirred at room temperature for 1 hour.

Subsequently, 0.4 mL of the activated acid solution was added to a suspension of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (40 mg, 0.089 mmol) and triethylamine (0.025 mL, 0.179 mmol) in N,N-dimethylformamide (2.5 mL). The mixture was stirred at room temperature for 3 hours. 0.05 mL of freshly prepared activated acid solution was added. Next,

the reaction mixture was stirred at room temperature for additional 2 hours. The reaction mixture was evaporated to dryness. The crude product was purified by column chromatography (0-8% methanol in chloroform). This gave a yellow solid containing the PBX-7014 product and residual succinimide. The product was further purified by acidic preparative MPLC (Luna5-40) to obtain a light yellow solid after lyophilization of the product fractions. Yield: 20 mg, 50%

U_AN_ACID: m/z 506.2 $[M+H]^+$
$^1H$ NMR (400 MHz, DMSO-d6) δ 8.40 (d, *J* = 8.9 Hz, 1H), 7.40 (s, 1H), 7.23 (s, 1H), 6.49 (s, 1H), 6.28 (d, *J* = 4.6 Hz, 2H), 5.61 - 5.45 (m, 2H), 5.45 - 5.35 (m, 2H), 5.19 - 5.06 (m, 2H), 3.95 (s, 2H), 3.13 - 2.96 (m, 2H), 2.21 - 2.02 (m, 2H), 1.94 - 1.77 (m, 2H), 0.87 (t, *J* = 7.3 Hz, 3H).

**2-2: Synthesis of PBX-7015**

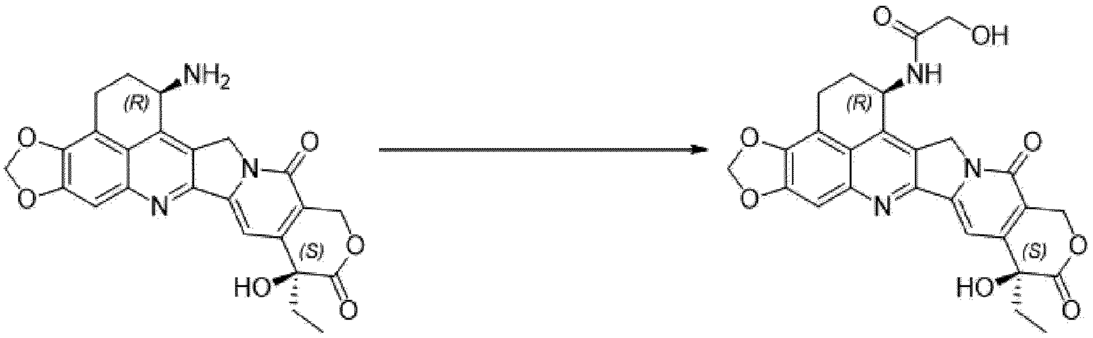

PBX-7012 PBX-7015

A stock solution of activated acid was prepared according to the following procedure: Glycolic acid (17.00 mg, 0.223 mmol) was dissolved in 1 mL of N,N-dimethylformamide. HOSu (25.7 mg, 0.223 mmol) and EDC (42.8 mg, 0.223 mmol) were added. The reaction mixture was stirred at room temperature for 1 hour.
Subsequently, 0.25 mL of activated acid solution was added to a suspension of (1R,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (25 mg, 0.056 mmol) and triethylamine (0.016 mL, 0.112 mmol) in N,N-dimethylformamide (2.5 mL). The mixture was stirred at room temperature overnight. 0.03 mL of freshly prepared activated acid solution was added. Next, the reaction mixture was stirred at room temperature for 2 hours. The reaction mixture was combined with the previous smaller batch and evaporated to dryness. The crude product was purified by column chromatography. This provided a yellow solid containing PBX-7015 product and residual succinimide. The product was purified by acidic preparative MPLC (Luna5-40). Product fractions were lyophilized to yield a light yellow solid. Yield: 15 mg, 38%

U_AN_ACID: 506.2 $[M+H]^+$
$^1H$ NMR (400 MHz, DMSO) δ 8.44 (d, *J* = 9.0 Hz, 1H), 7.41 (s, 1H), 7.24 (s, 1H), 6.48 (s, 1H), 6.29 (d, *J* = 2.3 Hz, 2H), 5.61 - 5.44 (m, 2H), 5.44 - 5.36 (m, 2H), 5.20 - 5.07 (m, 2H), 3.96 (s, 2H), 3.10 - 2.96 (m, 2H), 2.20 - 2.06 (m, 2H), 1.95 - 1.79 (m, *J* = 7.3 Hz, 2H), 0.87 (t, *J* = 7.3 Hz, 3H).

**Preparation Example 3: Synthesis of active camptothecin derivative of Formula 5 (PBX-7016)**

This example describes the synthesis of compound PBX-7016 starting from the Exatecan-hybrid compound (PBX-7011).

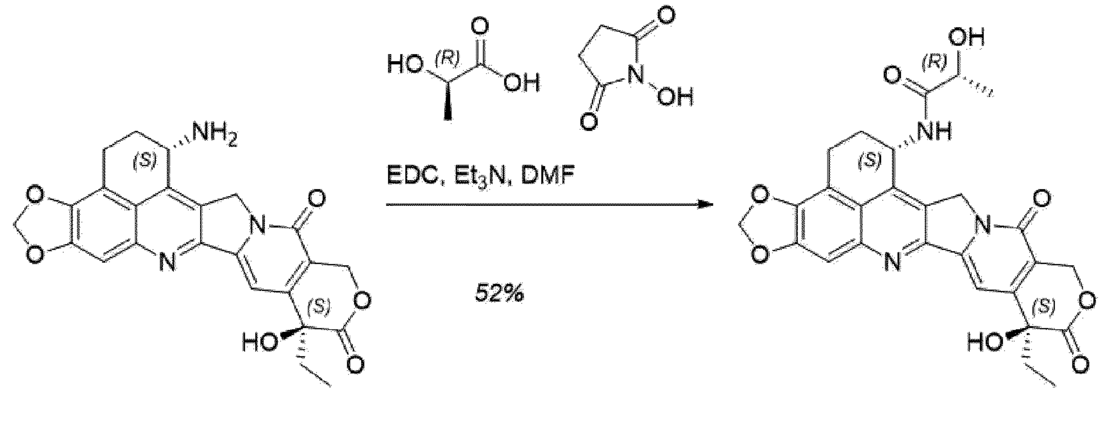


PBX-7011 PBX-7016

A stock solution of activated D-lactic acid was prepared according to the following procedure.

D-lactic acid (22 mg, 0.244 mmol) was dissolved in 1 mL of N,N-dimethylformamide. HOSu (27 mg, 0.235 mmol) and EDC (38.6 mg, 0.201 mmol) were added. The reaction mixture was stirred at room temperature for 2 hours.

Subsequently, 0.3 mL of activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (36 mg, 0.080 mmol) and triethylamine (0.022 ml, 0.161 mmol) in N,N-dimethylformamide (2.5 ml). The mixture was stirred at room temperature for 6 hours. 0.05 mL of the prepared activated acid solution was added. Next, the reaction mixture was stirred at room temperature overnight. The reaction mixture was directly purified by acidic preparative MPLC (Luna10-50) to obtain a light yellow solid after lyophilization of the product fraction.

Yield: 22mg, 52%
U_AN_ACID: m/z 520.2 $[M+H]^+$
$^1H$ NMR (400 MHz, DMSO) $\delta$ 8.43 (d, J = 9.1 Hz, 1H), 7.41 (s, 1H), 7.23 (s, 1H), 6.50 (s, 1H), 6.29 (d, J = 2.1 Hz), 2H), 5.62 - 5.58 (m, 1H), 5.58 - 5.51 (m, 1H), 5.41 (s, 2H), 5.21 - 5.01 (m, 2H), 4.17 - 4.07 (m, 1H), 3.14 - 2.95 (m, 2H), 2.19 - 2.04 (m, 2H), 1.92 - 1.78 (m, 2H), 1.39 (d, J = 6.8 Hz, 3H), 0.87 (t, J = 7.3 Hz, 3H).

As described above, PBX-7016 of Chemical Formula 5 may be synthesized from PBX-7011 of Chemical Formula 3, and therefore, PBX-7017 of Chemical Formula 5-1 may be synthesized from PBX-7012 of Chemical Formula 3-1 by the same method.

**Preparation Example 4: Synthesis of active camptothecin derivative of Formula 9 (PBX-7024)**

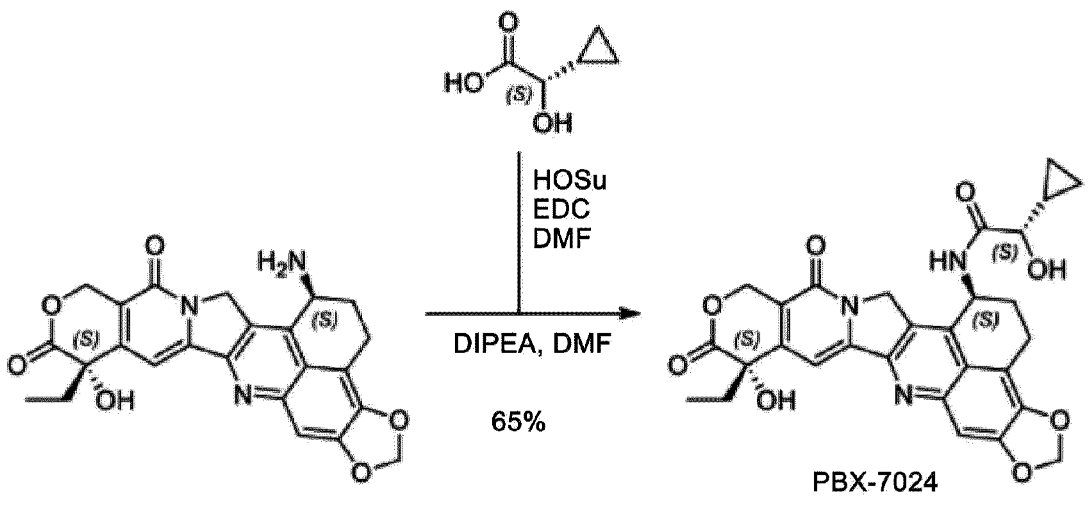


PBX-7024

PBX-7011

20 mg shipped

PBX-7024 was prepared in high yield by combining (2S)-2-cyclopropyl-2-hydroxyacetic acid with the PBX-7011 compound.

(2S)-2-cyclopropyl-2-hydroxyacetic acid (26 mg, 0.244 mmol) was dissolved in 1 mL of N,N-dimethylformamide. HOSu (26 mg, 0.226 mmol) and EDC (42 mg, 0.219 mmol) were added. The reaction mixture was stirred at room temperature for 2 hours. Next, 0.6 mL of activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinoline-11,14-dione (40 mg, 0.089 mmol) and DIPEA (0.047 ml, 0.268 mmol) in N,N-dimethylformamide (2.5 ml). The mixture was stirred at room temperature overnight. The reaction mixture was directly purified by acidic preparative MPLC (Luna10-50) to obtain a bright white solid after lyophilization of the product fraction.

Yield: 32 mg, 65%
U_AN_ACID: m/z 520.2 $[M+H]^+$
$^1H$ NMR (400 MHz, DMSO-d6) δ 8.33 (d, *J* = 8.7 Hz, 1H), 7.39 (s, 1H), 7.23 (s, 1H), 6.48 (s, 1H), 6.28 (d, *J* = 4.9 Hz, 2H), 5.50 (q, *J* = 6.7 Hz, 1H), 5.40 (s, 3H), 5.23 - 5.06 (m, 2H), 3.62 (d, *J* = 6.3 Hz, 1H), 3.03 (q, *J* = 6.2 Hz, 2H), 2.21 - 2.02 (m, 2H), 1.92 - 1.79 (m, 2H), 1.18 - 1.08 (m, 1H), 0.87 (t, *J* = 7.3 Hz, 3H), 0.47 - 0.30 (m, 4H).

**Example 1**

For FL118, Exatecan, SN-38, Dxd, PBX-7011, PBX-7014, PBX-7016 and PBX-7024, the expression inhibition analysis of anti-apoptotic proteins and *in vitro* Cell viability assay were performed as follows.

**1-1: Confirmation of suppressive activity of expression of cancer-related survival genes of DDXS, survivin, Mcl-1, XIAP and cIAP2 through Western blot**

(1) Protein extraction

200,000 FaDu cell lines were seeded per well in a 6-well plate and incubated at constant temperature (37°C, 5% $CO_2$). After 24 hours, drugs (FL118 drug, SN-38 drug, exatecan drug, PBX-7011, PBX-7014, PBX-7016) were treated in each well at concentrations of 0 nM, 10 nM and 100 nM. It was incubated at constant temperature (37°C, 5% $CO_2$) for 24 hours. The well was treated with 100 uL of RIPA buffer dissolved in protease inhibitor cocktail. The plate was placed on ice and incubated for 2 hours on an orbital shaker. RIPA buffer including lysed cells was transferred to an ep tube and centrifuged (16,000 rcf, 20 min, 4°C). Afterwards, only the supernatant was transferred to a new EP tube. Protein concentration was determined through protein assay.

(2) Protein separation through electrophoresis

The protein sample and 4x SDS-PAGE Loading Buffer were mixed in a 3:1 ratio, boiled at 95°C for 10 minutes and cooled. Samples were loaded into gel wells such that the amount of protein was the same. Electrophoresis was performed on the gel at 60V.

(3) Transfer of protein from gel to membrane

7 sheets of activated filter paper, PVDF membrane, gel and 7 sheets of filter paper were placed in order in the cassettes of the Trans-Blot® Turbo™ Transfer System, and the lid was closed, and after being inserted into the machine, the protocol started.

(4) Antibody incubation

The transferred membrane was immersed in blocking buffer and incubated at constant temperature (RT, 1h). Next, it was incubated at 4°C overnight in primary antibody solution. It was rinsed with TBST buffer for 3 minutes (repeated 3 times). Incubation was performed (RT, 1h) in a secondary antibody solution conjugated with HRP. It was rinsed with TBST buffer for 3 minutes (repeated 3 times).

(5) Imaging and result analysis

After soaking the membrane in the ECL substrate for 3 to 5 minutes, the signal was determined by using the ChemiDoc™ MP Imaging System. HRP bound to the secondary antibody oxidized the Luminol of ECL, and the emitted light was detected and displayed as an image. Since the thickness of the band is proportional to the amount of protein, the

amount of protein can be compared through the thickness of the band.

In the same way, A549, which overexpresses ABCG2, was seeded instead of the FaDu cell line, which does not express ABCG2, and 4 ug of protein was loaded, and Western blot was performed.

As shown in FIGS. 6 to 8, it was confirmed how the protein expression level changed by treatment with drugs (FL118 drug, SN-38 drug, exatecan drug, PBX-7011, PBX-7014, PBX-7016). GAPDH is an enzyme involved in glycolysis, which is an essential metabolic process in cells. It is a gene that is always expressed in cells and its expression level does not change easily, and it is an indicator that shows whether the same amount of protein is loaded on the gel in the samples.

**Consideration: Evaluation of the PBX-7011, PBX-7014 and PBX-7016**

As a result of confirming the degree of inhibition of the expression of anti-apoptotic proteins in the FaDu cell line through Western blot, PBX-7011, PBX-7014 and PBX-7016 all showed a decrease in the expression of Survivin, cIAP2, Mcl-1 and XIAP as the concentration increased, and compared with the existing drugs FL118, SN-38 and Exatecan, the levels were similar to those of FL118 and Exatecan, and better than SN-38.

In Example 2-1, it was confirmed that it exhibited a dual inhibition effect of inhibiting Topoisomerase 1 and degrading DDX5, and has a heterogeneous characteristic of DDX5 degradation and a corresponding anticancer effect.

Specifically, two cell lines (FaDu, A549) were treated with a certain concentration (0, 10, 100 nM) of the drug, and the expression level of each protein (DDX, Survivin, Mcl-1, XIAP) was confirmed by Western blot. As a result, DDX5 degradation by FL1 18 was superior to other substances, followed by 7011, 7016 and 7014.

The trends of not only DDX5 but also other anti-apoptotic proteins in its downstream were similar to the above.

In short, PBX-7014 and PBX-7016 not only inhibit type I topoisomerase, but also show a dual MoA that acts as a degrader of DDX5 (p68), which is an oncoprotein that regulates Survivin, Mcl-1, XIAP and the like.

As a result of the evaluation, among PBX-7014 and PBX-7016, PBX-7016 inhibits DDX5 in a concentration-dependent manner, and as a result, it was observed to inhibit Survivin, Mcl-1, and thus, in addition to being a topoisomerase I inhibitor, PBX-7016 also showed a role as a DDX5 degrader of FL118, and it was confirmed that PBX-7016 worked better than PBX-7014 (FIGS. 7 and 8).

**1-2: Cell viability assay for FaDu cell line / A549 cell line**

In a 96-well plate, 3,000 FaDu cell lines (cancer cells that do not express ABCG2) or A549 cell lines (cancer cells that overexpress ABCG2) per well were seeded and incubated at temperature (37°C, 5% $CO_2$). After 24 hours, 100 uL of the drug at 9 concentrations (serial dilution of 1/5 from 1,000 nM) was treated with the cells. In this case, Dxd drugs, PBX-7014, PBX-7016 and PBX-7024 were treated. In this case, a control group that was not treated with the drug (drug concentration 0) was also prepared. It was cultured at constant temperature (37°C, 5% $CO_2$) for 3 or 6 days. 100 uL of CellTiter-Glo reagent (using CellTiter-Glo® Luminescent Cell Viability Assay kit (Promega, G7571)) was added to each well and pipetted. After incubation at constant temperature (RT) for 10 minutes, luminescence was measured. When considering the luminescence value when the drug concentration was 0 as 100%, the concentration that exhibited a luminescence value of 50% is the $IC_{50}$ value.

Two types of cell lines (FaDu, A549) were treated with two compounds (PBX-7016, PBX-7024) and reference compounds (Dxd, SN-38, exatecan, FL118), and cell viability was observed through incubation for 3 days.

As shown in FIG. 9, PBX-7024 shows a strong apoptosis effect not only in the FaDu cell line that does not express ABCG2, but also in A549, which is a cancer cell line that overexpresses ABCG2. In other words, it was confirmed that it has an $IC_{50}$ of an equal or higher level compared to Dxd, which is a payload used in the existing Enhertu.

In A549 that is a cancer cell line overexpressing ABCG2, as shown in FIG. 9, it can be confirmed that camptothecin compounds including Dxd showed increased $IC_{50}$ values, whereas PBX-7016 and PBX-7024 still maintained strong efficacy.

In other words, when using the novel camptothecin compounds PBX-7016 and PBX-7024 according to the present invention, unlike ADCs using existing camptothecin compounds such as SN-38 and DXd, it has an effect of overcoming the resistance mechanism caused by the overexpression of ABCG2.

**1-3: New PBX-series compounds as candidate for ADC payload: *in vitro* cell viability test**

Cell viability assay was performed on MDA-MB-453 (HER2++) cell line and FaDu (HER2+) cell line in the same manner as Example 1-2.

Two cell lines (MDA-MB-453, FaDu) were treated with two compounds (PBX-7014, PBX-7016, PBX-7018, PBX-7020, PBX-7022) and one reference compound (Dxd), and cell viability was observed through 3 days of incubation. The results are shown in FIG. 10.

As shown in FIG. 10, as a result of the evaluation, it was confirmed that PBX-7016, PBX-7018, PBX-7020 and

PBX-7022 had $IC_{50}$ of the same or higher level than Dxd. In addition, it was confirmed that the FaDu cell line, which does not express ABCG2, had an $IC_{50}$ value equal to or higher than that of Dxd.

**Example 2 PBX-7016 IV and IP Pharmacokinetic Profile in NOD SCID Mice**

The experimental method is shown in Table 2 below. The IV Plasma concentration-time data of PBX-7016 are shown in Table 3, and the IP Plasma concentration-time data of PBX-7016 are shown in Table 4.

[Table 2]

| | | | **In-life** | | |
|---|---|---|---|---|---|
| Standard PK | | | | **Species/strain:** | NOD SCID Mice |
| 3 | | | | **Sex:** | Female |
| IV | 1 | mg/kg | | | |
| IP | 1 | mg/kg | | | |
| IV | 5% DMSO and 95%(0.1% (w/v) hydroxypropyl-beta-cyclodextrin in saline) adjust PH to 8~9 | | | | |
| IP | 5% DMSO and 95%(0.1% (w/v) hydroxypropyl-beta-cyclodextrin in saline) adjust PH to 8~9 | | | | |
| IV | 0.5 | mg/mL | | | |
| IP | 0.2 | mg/mL | | | |
| IV | 0.167, 1, 4, 12, 24 and 48 hr post dose. | | | | |
| IP | 0.167, 1, 4, 12, 24 and 48 hr post dose. | | | | |

[Table 3]

| **IV Dose** | **1** | **mg/kg** | | | | |
|---|---|---|---|---|---|---|
| **Time (h)** | **Concentration (ng/mL)** | | | **Mean (ng/mL)** | **SD (ng/mL)** | **CV (%)** |
| | **Mouse 1** | **Mouse 2** | **Mouse 3** | | | |
| 0.167 | 76.2 | 54.8 | 46.8 | 59.3 | 15.2 | 25.6 |
| 1 | 10.6 | 6.85 | 3.35 | 6.9 | 3.6 | 52.3 |
| 4 | BLOQ | BLOQ | BLOQ | NA | NA | NA |
| 12 | BLOQ | BLOQ | BLOQ | NA | NA | NA |
| 24 | BLOQ | BLOQ | BLOQ | NA | NA | NA |
| 48 | BLOQ | BLOQ | BLOQ | NA | NA | NA |

[Table 4]

| **IP Dose** | **1** | **mg/kg** | | | | |
|---|---|---|---|---|---|---|
| **Time (h)** | **Concentration (ng/mL)** | | | **Mean (ng/mL)** | **SD (ng/mL)** | **CV (%)** |
| | **Mouse 4** | **Mouse 5** | **Mouse 6** | | | |
| 0.167 | 158 | 186 | 149 | 164 | 19 | 11.7 |
| 1 | 10.5 | 23.9 | 14.0 | 16.1 | 7.0 | 43.1 |
| 4 | BLOQ | BLOQ | BLOQ | NA | NA | NA |
| 12 | BLOQ | BLOQ | BLOQ | NA | NA | NA |
| 24 | BLOQ | BLOQ | BLOQ | NA | NA | NA |
| 48 | BLOQ | BLOQ | BLOQ | NA | NA | NA |

Through the results in Tables 3 and 4, it was confirmed that PBX-7016 compound was cleared from the blood very quickly, when it as administered intravascularly or intraperitoneally to mice, and as a result, it is expected that PBX-7016 compound alone, not in the form of a prodrug or ADC, will exhibit high safety when administered *in vivo.* When the

PBX-7016 compound is administered IV, it is released out of the body within a very short period of time, but enters the body and can act as a safety device that can reduce systemic exposure to the drug even when the drug is released prematurely.

In addition, this experiment is the PK of the PBX-7016 compound used as a payload alone, suggesting that it is a safe payload that allows clearance of the PBX-7016 drug in the blood within a short period of time. In other words, this suggests that after the PBX-7016 compound exerts a bystander suicide effect at the tumor site, it is safely released outside the body within a very short period of time even when circulating in the blood.

**Example 3. Carboxylate to Lactone conversion rate of PBX-7016 at pH 6.0**

Example 3 (FIGS. 24 and 25) is an experiment related to the activation of the lactone moiety of camptothecin, which is known as an activator of Top1 inhibitor.

**3-1. Carboxylate to lactone form conversion in pH 6.0**

PBX-7016 powder was prepared by dissolving in DMSO at 5 mM. Since PBX-7016 dissolved in DMSO exists in lactone form, it was converted to carboxylate form by diluting the same 1/10 with 0.1 N NaOH and incubating in a 25°C water bath for 30 minutes. The carboxylate form of PBX-7016 was diluted to 3 μM using 4°C PBS (pH 6.0) including 10% DMSO. In this case, the same amount of HCl as the 0.1 N NaOH used was added to minimize the pH change. After dilution, centrifugation was performed at 4°C, 2 minutes and 13,000 RCF conditions, and the supernatant was used as a sample.

When PBX-7016 lactone form was generated, two solvents, Solvent A: 100mM Acetate buffer pH 5.5 and Solvent B: ACN, were used to separate the same by RP-HPLC. The column used was Cortecs C18 2.7μm, 4.6×50mm Column, and analysis was performed under the following conditions: 1) 20 minutes: 5% B, 2) sample injection, 3) 2 minutes: 5% B, 4) 10 minutes: 5% B → 50% B, 5) 1 minute: 50% B.

In order to measure changes over time, the sample chamber was maintained at 37°C, and the sample was set to be injected every measurement time. The measurement times were 0, 20, 40, 60, 90, 120, 180, 240, 300, 360, 480, 600, 720, 840, 960, 1,200 and 1,440 minutes.

The content was calculated from the peak area of each lactone and carboxylate. Since the absorption coefficients of lactone and carboxylate are different, the lactone area was obtained by multiplying the carboxylate area by the equation below.

$$\text{lactone } EC_{390} / \text{carboxylate } EC_{390} = 0.918$$

The same experiment as Example 3-1 was performed, except that FL118, SN38, DxD, exatecan, PBX-7014 and PBX-7024 were used instead of PBX-7016.

The results of the conversion experiment from carboxylate to lactone form at pH 6.0 are shown in FIG. 25. As shown in FIG. 25, it was confirmed that various camptothecin-based compounds change form from carboxylate to lactone, which is an active form that inhibits topoisomerase I, at a similar rate and in an equivalent amount at pH 6.0, and through this, it is generally predicted that the same level of activity will be observed in the tumor microenvironment at a pH of 6.0.

**3-2. Lactone to carboxylate form conversion in pH 7.4**

PBX-7016 powder was prepared by dissolving in DMSO at 5 mM, and diluted with 4°C PBS (pH 7.4) including 10% DMSO to prepare 3 μM PBX-7016. Afterwards, it was centrifuged at 4°C, 2 minutes, and 13,000 RCF conditions, and the supernatant was used as a sample. PBX-7016 dissolved in DMSO existed in a lactone form.

When PBX-7016 carboxylate form was generated, two solvents, Solvent A: 100 mM Acetate buffer pH 5.5 and Solvent B: ACN, were used to separate the same by RP-HPLC. The column used was Cortecs C18 2.7μm, 4.6×50mm Column, and analysis was conducted under the following conditions. 1) 20 minutes: 5% B, 2) sample injection, 3) 2 minutes: 5% B, 4) 10 minutes: 5% B → 50% B, 5) 1 minute: 50% B.

In order to measure changes over time, the sample chamber was maintained at 37°C, and the sample was set to be injected every measurement time. The measurement times were 0, 20, 40, 60, 90, 120, 180, 240, 300, 360, 480, 600 and 720 minutes.

The content was calculated from the peak area of each lactone and carboxylate. Since the absorption coefficients of lactone and carboxylate are different, the lactone area was obtained by multiplying the carboxylate area by the equation below.

$$\text{lactone } EC_{390} / \text{carboxylate } EC_{390} = 0.918$$

The same experiment as Example 3-2 was performed, except that FL118, SN38, DxD, exatecan, PBX-7014 and PBX-7024 were used instead of PBX-7016.

As shown in FIG. 24, which is the results of the conversion experiment from lactone form to carboxylate at pH 7.4, at pH 7.4, which is the pH of blood or extracellular fluid, it was confirmed that compounds PBX-7014, PBX-7016 and PBX-7024 represented by Chemical Formula 1 were converted from the lactone form (active form) to the carboxylate form (inactive form) which is inactive as TOP1 detoxifiers faster and at a higher rate than other reference compounds (exatecan, DXd, SN-38, FL118).

In particular, the degree of formation (63 to 86%) and rate (0.4 to 0.8%/minute) of carboxylate form, which is inactive as a TOP1 inhibitor at pH 7.4 in the blood, varies greatly depending on the type of camptothecin derivative. PBX-7014 and PBX-7016 formed more than 86% of the carboxylate form, and both converted from the lactone form to the carboxylate form relatively quickly at the level of 0.8% per minute. DXd, which is a competing drug, formed a carboxylate form at a level of 76.5%, and was converted from the lactone form to the carboxylate form at a level of 0.6% per minute.

Through this, it is expected that the compounds PBX-7014 and PBX-7016 will show high safety in normal cells because they exist in the inactive form of carboxylate rather than the active form of lactone, which exhibits cytotoxicity in the blood relatively quickly and at a high rate compared to the reference compounds.

**Preparation Example 5: Synthesis of CL2A-FL118**

CL2A-FL118 conjugate (Chemical Formula 7-1) was synthesized according to the method described in Example 1 of WO2022-015110 A1.

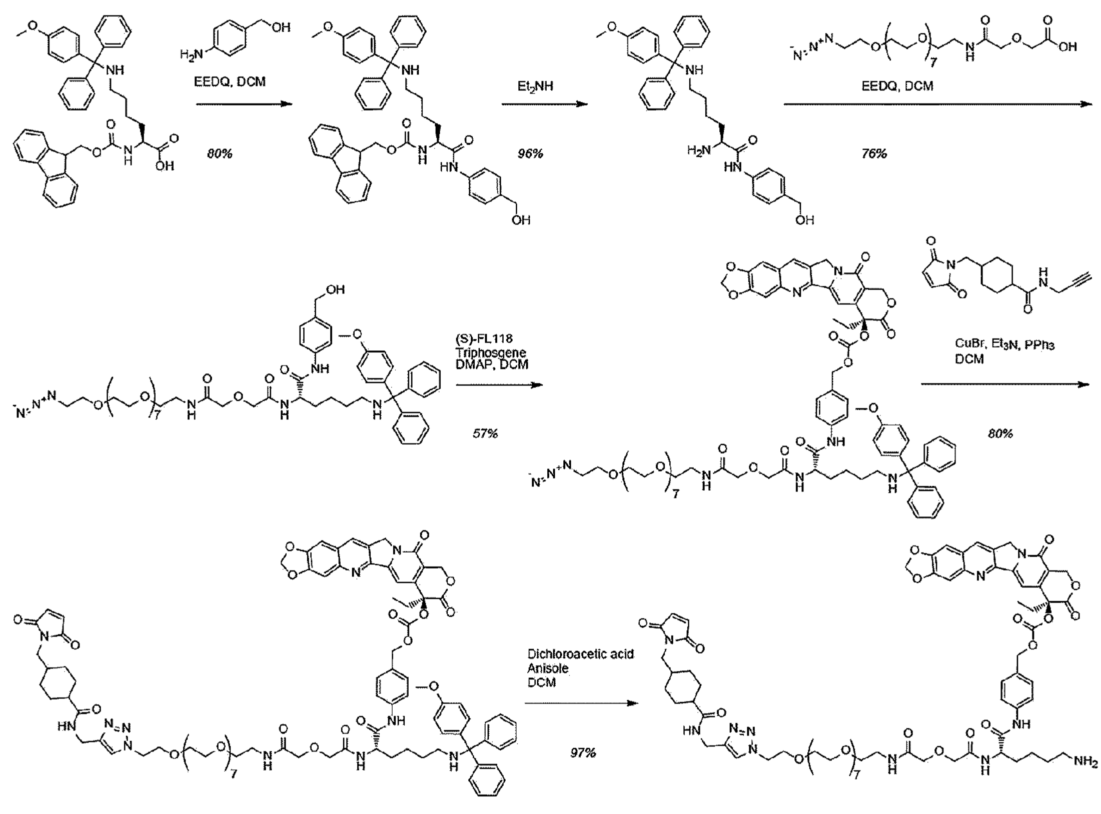


**Preparation Example 6: Synthesis of 25-4 and 25-6 from PBX-7014 and PBX-7016**

Linker-payloads 25-4 (Chemical Formula 9) and 25-6 (Chemical Formula 10) including two compounds (PBX-7014, PBX-7016) and introducing GGFG, which is an enzymatically cleavable linker system, were synthesized.

[Chemical Formula 9]

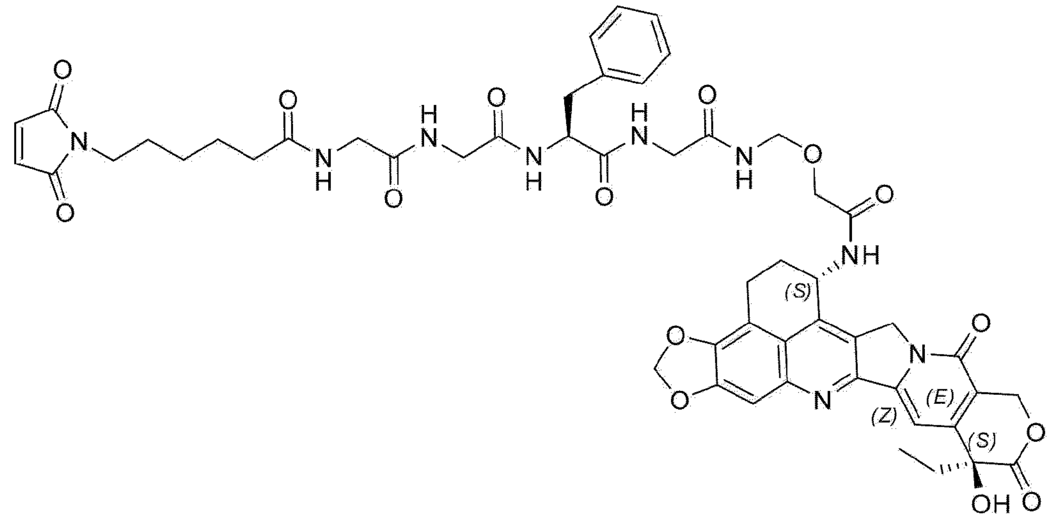

[Chemical Formula 10]

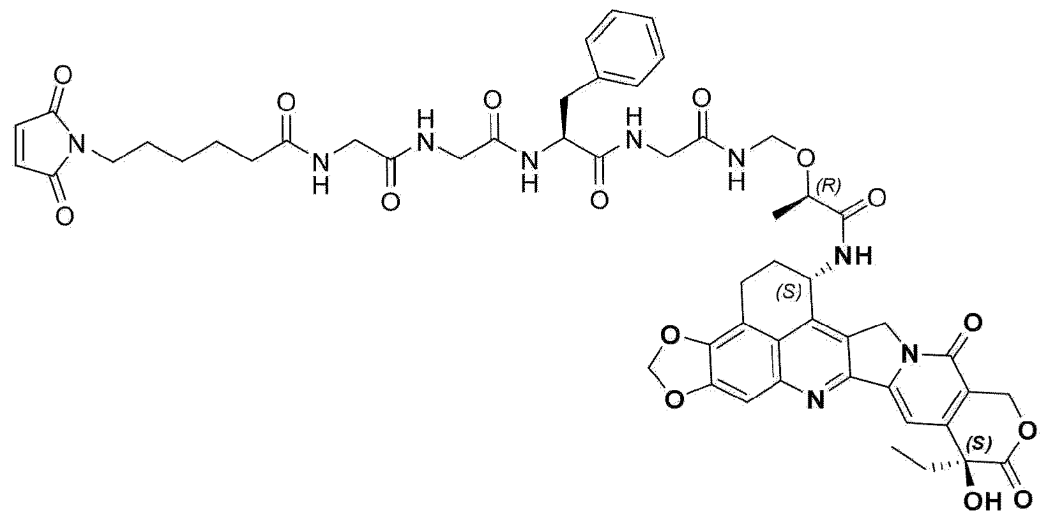

The molecular structure of 25-4 is GGFG-PBX-7014, and the molecular structure of 25-6 is GGFG-PBX-7016. That is, each uses the same GGFG linker as Enhertu®.

**Comparative Example 1: Preparation of Cetuximab-CL2A-FL118 immunoconjugate (DAR 8)**

Cetuximab-CL2A-FL118 immunoconjugate (DAR 8) was prepared by the method described in Example 8 of WO2022-015110 A1.

Anti-EGFR antibody (cetuximab) was reduced with 5 mM TCEP in original buffer (20 mM histidine, 150 mM NaCl, pH 6.0), and the reaction vial was placed in an incubator shaker at 22°C at 60 rpm rotation speed for 3 hours. After reduction, TCEP was removed through a spin desalting column (40 K, 2 mL x 2).

DMSO and CL2A-FL118 compound solutions (10 mg/mL stock in DMSO, 12 eq. for antibody) were added to the reduced antibody solution to a final DMSO concentration of 10%. The reaction mixture was mixed properly, and the reaction vial was left at 4°C for 2 hours.

After conjugation, the reaction mixture was placed in exchange buffer with 18 mM MES (pH 6.5) through a spin desalting column (40 K, 10 mL). The product was then sterile filtered through a 0.2 μm PVDF disposable filter. The resulting immunoconjugate was characterized, and the resulting immunoconjugate was freeze-dried after the addition of 0.07 mM PS80 and 20 mM trehalose dehydrate.

The DAR of the Cetuximab-CL2A-FL118 immunoconjugate of Example 1 was determined by using LCMS, and

the average MS-DAR value was confirmed to be 7.99 (FIG. 14).

**Example 4: Preparation of nimotuzumab-CL2A-FL118 immunoconjugate (DAR 8)**

A nimotuzumab-CL2A-FL118 immunoconjugate (DAR 8) was prepared in a similar manner to Comparative Example 1, except that nimotuzumab was used instead of Cetuximab.

The DAR of the nimotuzumab-CL2A-FL118 immunoconjugate of Example 4 was determined by using LCMS, and the average MS-DAR value was confirmed to be 7.94 (FIG. 15).

**Example 5: Preparation of ADC with 19-1 (CL2A-FL118, DAR 8), 25-6 (GGFG-PBX-7016, DAR 8) and 25-6-a (GGFG-PBX-7016, DAR 4)**

**5-1. Preparation method of Nimotuzumab (YTE)**

Nimotuzumab (YTE) is an antibody that improves the half-life of Nimotuzumab. Nimotuzumab (YTE) is a modified IgG that is constituted by including a human IgG normal domain or an FcRn-binding fragment thereof, including substitutions at amino acid residues 252, 254 and 256 based on Kabat's EU numbering for the wild-type human IgG normal domain. The above formulated IgG has an increased half-life compared to the half-life of an IgG having a wild-type human IgG normal domain, and in Nimotuzumab, the substitution at amino acid residue 252 is a substitution with tyrosine, the substitution at amino acid residue 254 is a substitution with threonine, and the substitution at amino acid residue 256 is a substitution with glutamic acid.

**5-2. Preparation method of Nimotuzumab(YTE)-19-1**

After buffer exchange of Nimotuzumab (YTE) antibody with reduction buffer (150 mM NaCl, 50 mM Histidine pH 6.0) using PD-10 desalting column, the disulfide bond of antibody was reduced by treating 27.5 uM antibody with 825 uM TCEP for 2 hours at 25°C.

Afterwards, excess TCEP was removed by using a PD-10 desalting column, and the conjugation reaction was performed by reacting 165 uM 19-1 linker payload and 13.8 uM reduced antibody in Reaction buffer (25mM Histidine pH6.0) including 10% DMSO at 25°C for 1 hour. After the conjugation reaction, excess Linker payload was removed by using PD-10, and the final material was obtained.

**5-3. Preparation method of Nimotuzumab-25-6 & Nimotuzumab(YTE)-25-6 (DAR 8)**

Nimotuzumab and Nimotuzumab (YTE) antibodies were each buffer exchanged with reduction buffer (150 mM NaCl, 50 mM Histidine pH 6.0) using a PD-10 desalting column, and then, 27.5uM of the antibody was treated with 825 uM TCEP for 2 hours at 25°C to reduce the disulfide bond of the antibody.

Afterwards, excess TCEP was removed by using a PD-10 desalting column, the conjugation reaction was performed by reacting 165 uM 25-6 linker payload (DAR 8) and 13.8 uM reduced antibody in a reaction buffer (25mM Histidine pH6.0) including 15% DMSO at 25 for 1 hour. After the conjugation reaction, the excess linker payload was removed by using PD-10 to obtain the final material.

**5-4. Preparation method of Nimotuzumab-25-6-a & Nimotuzumab(YTE)-25-6-a (DAR 4)**

Nimotuzumab and Nimotuzumab (YTE) antibodies were each buffer exchanged with reduction buffer (150 mM NaCl, 50 mM Histidine pH 6.0) using a PD-10 desalting column, and then, 27.5 uM of the antibody was treated with 825 uM TCEP for 2 hours at 25°C to reduce the disulfide bond of the antibody.

Afterwards, excess TCEP was removed by using a PD-10 desalting column, and the conjugation reaction was performed by reacting 82.5 uM 25-6-a linker payload (DAR 4) and 13.8 uM reduced antibody in a reaction buffer (25mM Histidine pH6.0) including 10% DMSO at 25 for 1 hour. After the conjugation reaction, excess linker payload was removed by using PD-10 to obtain the final material.

**Example 6. Anti EGFR ADCs with PBX-payloads: binding affinity analysis / Nimotuzumab ADC - EGFR binding**

FIG. 16 shows the background of cell line selection for *in vitro* and *in vivo* experiments of EGFR targets. The subsequent experiment was performed by evaluating MDA-MD-468, which overexpressed EGFR, as a positive cell line, and evaluating SW620, which did not express (underexpressed) EGFR, as a negative cell line.

Nimotuzumab ADC - EGFR binding affinity analysis was performed by modifying the method described in

"Example 6. Anti HER2 ADCs with PBX-payloads: binding affinity analysis" in PCT/KR2023/009854 (FIG. 17).

An evaluation was performed by synthesizing ADCs using a total of 6 types of Nimotuzumab and Nimotuzumab (YTE), which improved the half-life of Nimotuzumab, as an antibody. When each created ADC was analyzed by ELISA, it was confirmed that the binding strength was not significantly different from those of antibodies alone (Nimotuzumab and Nimotuzumab (YTE)).

BC50: binding concentration at 50% maximum
19-1: CL2A-FL118, 25-6: GGFG-PBX-7016 (DAR 8), 25-6-a: GGFG-PBX-7016 (DAR 4)

### Example 7: *In vitro* assay of Nimotuzumab ADC and Cetuximab ADC

Various evaluations were performed on EGFR positive and negative cells, and the results are shown in FIGS. 18, 19 and 20.

In FIG. 18, except for the ADC of 19-1 (CL2A-FL118) which allows extracellular payload release, ADC of 25-6 (GGFG-PBX-7016) using an enzyme-cleavable linker showed no cytotoxic effect in all cell lines. Through this, Nimotuzumab and Nimotuzumab (YTE) ADC, which has a binding affinity (Kd) of 1/10 that of cetuximab, showed a very high $IC_{50}$.

In FIG. 19, Cetuximab, which has a binding strength to EGFR about 10 times greater than Nimotuzumab, not only showed a very excellent $IC_{50}$ value, but also distinguished between positive and negative cell lines very well. Through *in vitro* experiments, Cetuximab ADC showed very excellent ADC results, but Nimotuzumab ADC did not show performance as an ADC.

In FIG. 20, except for the ADC of 19-1 (CL2A-FL118) which allows extracellular payload release, all enzyme-cleaved ADCs showed very high $IC_{50}$ in both positive and negative cell lines.

### Example 8: *In vivo* efficacy study in MDA-MB-468 model

#### 8-1. Animal model and material preparation

All procedures related to animal handling, care and treatment in this study were performed in accordance with the guidelines of the Association for Assessment and Accreditation of Laboratory Animal Care (AAALAC) and approved by the Institutional Animal Care and Use Committee (IACUC) of Shanghai Model Organisms Center, Inc. 21 female mice were housed in cages (200 $mm^3$), three per cage, maintained at a constant temperature (18 to 26°C) and humidity (40 to 70%), and given free access to sterilized dry granule feed and sterilized drinking water in individually ventilated cages.

Specific methods for evaluating drug efficacy *in vivo* are shown in Table 5 below.

[Table 5]

| Group | No. of animal | Animal | MDA-MB-468 cell numbers | Treatment | Dosage (mg/kg) | Dosing Route | Dosing Schedule | Dosing Times |
|---|---|---|---|---|---|---|---|---|
| 1 | 3 | SCID-beige | 1 X $10^7$ | saline | NA | I.V. | q1w | 2 |
| 2 | 3 | SCID-beige | 1 X $10^7$ | Cetuximab-FL118(DAR8) | 1 | I.V. | q1w | 2 |
| 3 | 3 | SCID-beige | 1 X $10^7$ | Cetuximab-FL118(DAR8) | 5 | I.V. | q1w | 2 |
| 4 | 3 | SCID-beige | 1 X $10^7$ | Cetuximab-FL118(DAR8) | 10 | I.V. | q1w | 2 |
| 5 | 3 | SCID-beige | 1 X $10^7$ | Nimotuzumab-FL118(DAR8) | 1 | I.V. | q1w | 2 |
| 6 | 3 | SCID-beige | 1 X $10^7$ | Nimotuzumab-FL118(DAR8) | 5 | I.V. | q1w | 2 |
| 7 | 3 | SCID-beige | 1 X $10^7$ | Nimotuzumab-FL118(DAR8) | 10 | I.V. | q1w | 2 |

### 8-2. Cell culture

MDA-MB-468 breast cancer cells were cultured in RPMI containing 10% FBS, 100 U/mL penicillin and 100 μg/mL streptomycin under conditions of 5% $CO_2$ and 37°C, and subculture was routinely performed twice a week by trypsin-EDTA treatment. Cells growing in exponential growth phase were harvested and counted for tumor inoculation.

### 8-3. Tumor inoculation

MDA-MB-468 cells (1 x $10^7$) in 0.2 mL of DPBS with Matrigel were inoculated subcutaneously into the right anterior flank of each mouse. When the average tumor volume reached approximately 215 $mm^3$, animals were randomly grouped, and then, treatment for drug efficacy studies was initiated.

### 8-4. Observation

During routine monitoring, normal behavior such as mobility, food and water consumption (observation only), body weight changes (weighed twice weekly), eye/hair matting, tumor growth and effectiveness of treatment were checked in animals every day. Deaths and observed clinical signs were recorded based on the number of animals in each subset.

### 8-5. Tumor measurements and endpoints

The primary endpoint was determined as a time point when tumor growth could be delayed or the mice could be cured. The two-dimensional size of the tumor was measured twice weekly using a caliper, and the volume was expressed in $mm^3$ using the equation ($V = 0.5\ a \times b^2$, where a and b are the long and short diameters of the tumor, respectively).

Tumor growth inhibition (TGI) was calculated for each group.

$$TGI (\%) = [1 - (T_i / T_0) / (V_i / V_0)] \times 100$$

($T_i$ is the average tumor volume of the treatment group on a given day, $T_0$ is the average tumor volume of the treatment group on the first day of treatment, $V_i$ is the average tumor volume of the vehicle control group on the same day as $T_i$, and $V_0$ is the average tumor volume of the vehicle group on the first day of treatment volume).

Animals with a body weight loss of more than 15% or a tumor volume of more than 3,000 $mm^3$ were euthanized as humane endpoints.

### 8-6. Statistical analysis

The results are expressed as mean and standard error (mean ± SEM). Data were analyzed by using two-way RM ANOVA Dunnett's multiple comparison test using Graphpad Prism 6.0 software, and $p < 0.05$ was considered statistically significant.

### 8-7. Body weight of MDA-MB-468 tumor model

The body weight of MDA-MB-468 tumor model mice was monitored regularly. As shown in FIG. 13, no obvious weight loss was observed in all treatment groups.

Unlike humans, mouse plasma has a high level of esterase activity and can easily cleave acid-sensitive bonds such as ester or carbonate functional groups. Therefore, when the immunoconjugate of the present invention is administered to tumor model mice, it was predicted that side effects such as weight loss would occur, but surprisingly, these side effects were not observed at all. From the above results, it was found that the immunoconjugate of the present invention does not show toxicity to normal cells even if FL118 is separated and released in the blood.

### 8-8. Tumor growth inhibition assay in MDA-MB-468 tumor model mice (FIGS. 21 to 23)

FIGS. 21 to 23 show the results of a total of three *in vivo* experiments on EGFR target ADC. The average tumor volume of MDA-MB-468 tumor model mice (n = 3) in each group is shown. Next, the TGI values of MDA-MB-468 tumor model mice (n = 3) were calculated based on tumor size (n = 3) on day 21 after treatment.

In the case of FIG. 21, Cetuximab-CL2A-FL118 ADC showed tumor regression in MDA-MB-468 tumor model mice.

In the case of FIG. 22, Nimotuzumab ADC with a large $K_d$ value (i.e., small binding affinity) was shown to be a relatively excellent tumor regression, that is, an excellent ADC, compared to Cetuximab ADC with a small $K_d$ value (i.e., large binding affinity).

FIG. 22 demonstrates that an immunoconjugate using an antibody having a dissociation constant $K_d$ value of $1*10^{-8}$ M or more to less than $1*10^{-6}$ M according to the present invention has a more superior effect than cetuximab, which is already known to exhibit excellent antitumor effect on its own. Although Nimotuzumab-CL2A-FL118 was designed to solve the toxicity problem, surprisingly, it was found that Nimotuzumab-CL2A-FL118 showed better efficacy compared to Cetuximab-CL2A-FL118, which was the reference. It was expected that the two ADCs would show similar efficacy, or Cetuximab-CL2A-FL118 would show slightly better efficacy, but the results exceeded expectations.

In FIG. 23, the following results were obtained.

(1) The ADC of Nimotuzumab-25-6 shows superior tumor regression than Nimotuzumab-19-1.
(2) In the case of comparison of Nimotuzumab and Nimotuzumab (YTE), that is, in the case of ADC that changed the half-life of the antibody, there was no significant difference. From this, it can be inferred that the ADC of the present invention penetrates cancer tissue well and exerts a cytotoxic effect at the initial stage of ADC administration.
(3) Comparison by administration dose (5 mpk, 10 mpk) also showed sufficient tumor regression in the 5 mpk administration group.
(4) Comparison of DAR4 and DAR8 ADCs also showed sufficient tumor regression with DAR4 alone.
(5) Only in the case of Nim(YTE) 5mpk and DAR4, there is exceptionally little tumor regression, and it is unclear whether this is due to the antibody (Nim(YTE)), the administered dose (5mpk), DAR(4) or the sum of all factors.

Through FIGS. 21 to 23, in the case of ADCs using an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently, antibodies that indirectly constitute ADCs bind to a drug target on the surface of cancer cells with strong affinity. In this case, it was demonstrated that this method can solve the problem of the administered ADC becoming useless because the drug target around the blood vessel is first saturated, and then, additional antibodies (ADC) cannot penetrate into the cancer tissue after a certain amount of antibodies have bound thereto, and the payload released from the ADC may be evenly distributed throughout the cancer tissue.

It was confirmed that the immunoconjugate of the present invention exhibits significantly excellent anticancer activity at all administered doses and does not exhibit side effects such as weight loss. In addition, since the immunoconjugates of the present invention have all been confirmed to exhibit a dose-dependent response, they may be administered in a patient-tailored regimen/dosage to achieve maximum efficacy and minimum toxicity for each patient.

Therefore, the immunoconjugate of the present invention may be very advantageously used for the prevention or treatment of cancer, and the FL118-linker conjugate of the present invention may be very advantageously used for the purpose of producing the immunoconjugate.

### Example 9

Antibodies that can be used in the ADC of the present invention are those that have a $K_d$ value of $1*10^{-8}$ M or more to less than $1*10^{-6}$ M among antibodies that can target drug targets for general solid cancer ADC development. $K_d$ value may be measured by using the SPR method or through quantitative Western blot analysis.

Drug targets for ADC that are the subject of the present invention include various examples such as Her2, EGFR, Trop2, c-Met, CEACAM-5, B7H3, B7H4, Muc1, CDH6 and CLDN18.2, but are specified to be overexpressed in cancer cells. Any surface antigen that can be used as a drug target for an ADC may be a suitable drug target for the ADC of the present invention.

Methods for obtaining the antibody of the present invention include (1) a method of obtaining an antibody with a $K_d$ value in a range of $1*10^{-8}$ M or more to less than $1*10^{-6}$ M from the process of producing an initial antibody against the antigen, and (2) it may be obtained by performing affinity maturation by changing the amino acid of the Fv region of an existing known antibody (e.g., Trastuzumab, Sacituzumab, etc.), but not in the direction of increasing affinity, but by lowering the affinity to a range where the $K_d$ value is $1*10^{-8}$ M or more to less than $1*10^{-6}$ M.

The target antibodies of this example obtained by the method of (1) include Nimotuzumab and variants of the Fc region obtained to improve the PK profile of Nimotuzumab. In this example, novel target antibodies such as Trastuzumab-1 and Sacituzumab-1 were produced by the method of (2).

Camptothecin series compounds that can be used in the ADC of the present invention include, representatively, camptothecin, topotecan, exatecan, various exatecan derivatives including SN-38 already used in Trodelvy, Deruxtecan (DXd) used by Daiichi-Sankyo, 10, 11-methylenedioxy-camptothecin (FL118) and derivatives thereof (PBX-7011) and

various derivatives thereof (PBX-7016, etc.). In the examples of the present invention, FL118 and PBX-7016 were used as payloads. However, as anyone of ordinary skill in the art would understand, it is not necessary to use FL118 and PBX-7016 to practice the present invention, and the same effects as in the examples of the present invention may be obtained by using an appropriate camptothecin compound.

PBX-7011 is a camptothecin derivative that was filed as a patent by the inventors of the present invention under Korean Patent Application No. 10-2022-0049158 on April 20, 2022. In addition to its strong Topoisomerase I inhibitory effect, it may play a role in inhibiting the expression of various anti-apoptotic proteins that cause cancer cell resistance to existing anticancer drugs. PBX-7016 is described in Korean Patent Application No. 10-2022-0103591 filed by the inventors of the present invention on August 18, 2022.

The most representative examples of the linker-payload used in the present invention include the following compounds 19-1 (CL2A-FL118) and 25-6 (MC-GGFG-NHCH2-PBX7016), and additionally, CL2A-SN38 used in the development of Trodelvy, CL2A-Belotecan used in the development of SKB264, MC-GGFG-NHCH2-DXd used in Enhertu, and various derivatives may be used as needed.

Additionally, in order to adjust the DAR to a lower value other than 8, the antibody conjugate of the linker compounds may be replaced with dibromomaleimide or a bridge compound that plays an equivalent role instead of maleimide, and specific examples of this are provided in 25-6-a below.

[Chemical Formula 11]

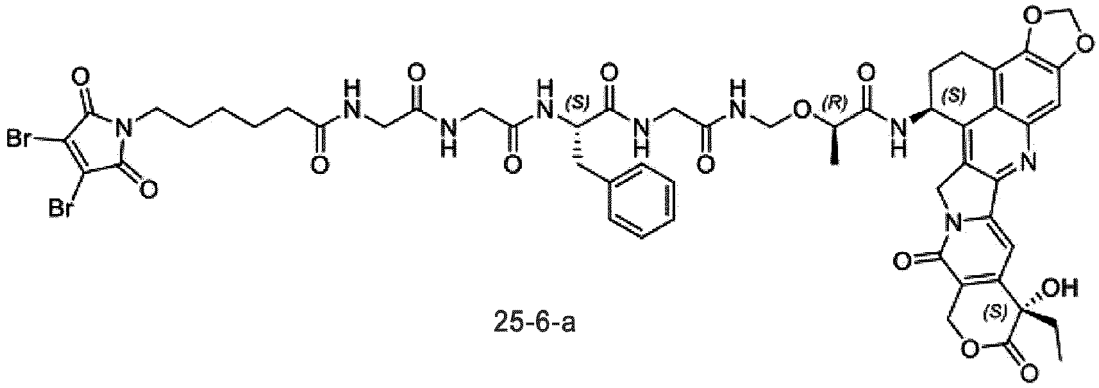

25-6-a

In the case of 19-1 (CL2A-FL118), n = 7 in Chemical Formula 7-1.

[Chemical Formula 7-1]

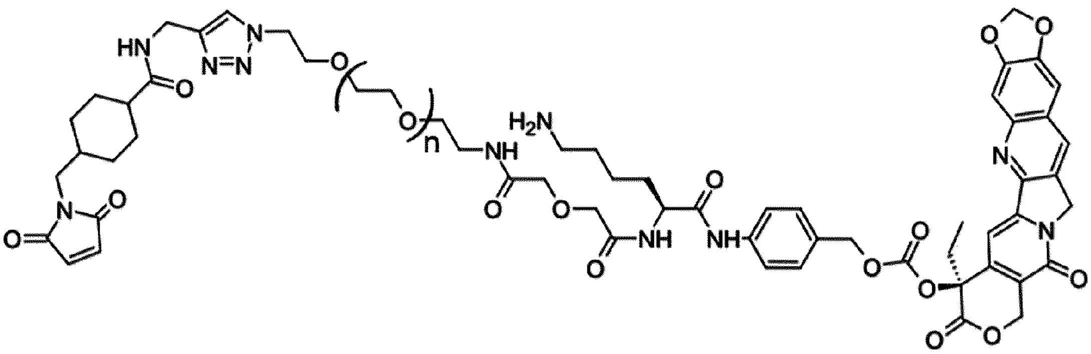

[Chemical Formula 10]

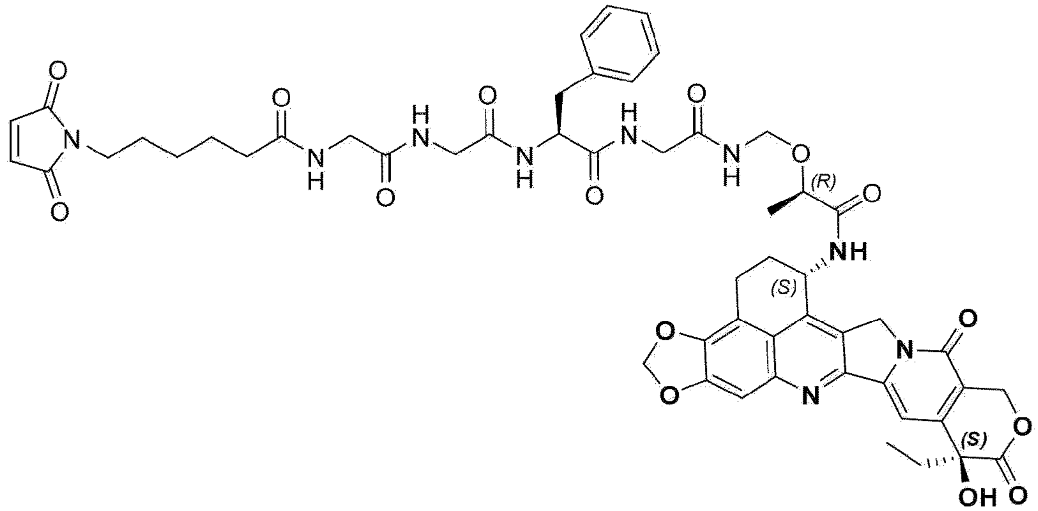

The ADC of this example may be synthesized by using the method of the examples described in Korean Patent No. 10-2349925 for the appropriate antibody and linker-payload compound of the present invention. That is, in the present invention, all interchain disulfide bonds are cleaved by using a reducing agent in the antibody to expose free -SH groups, and then, an excess amount of linker-payload compound is added to perform a conjugation reaction. Afterwards, the obtained antibody-drug conjugate may be purified by an appropriate method to prepare the ADC compound of the present invention.

### 9-1. Production of EGFR ADC

Cetuximab with a $K_d$ value of 1.8*10$^{-9}$ M was used as a control antibody, and Nimotuzumab with a $K_d$ value of 2.9*10$^{-8}$ M was used as an antibody that is suitable for use in the present invention.

As exemplified in Examples 4 and 5, first of all, ADCs of each DAR 8 (Nimotuzumab/19-1, Cetuxumab/19-1) were prepared by using the 19-1 compound, and at the same time, the 25-6 compound was used to prepare ADC of DAR 8 (Nimotuzumab/25-6, Cetuximab/25-6).

### 9-2. Production of Trop-2 ADC

Sacituzumab with a $K_d$ value of 1.7*10$^{-9}$ M was used as a control antibody, and in order to produce an antibody that is suitable for use in the present invention, amino acids in the Fv sequence were changed from Sacituzumab one by one to produce an antibody (Sacituzumab-1) with a $K_d$ value of 4*10$^{-8}$ M.

Sacituzumab-1/19-1 (DAR 8), Sacituzumab-1/25-6 (DAR 8), Sacituzumab/19-1 (DAR 8) and Sacituzumab/25-6 (DAR 8) were produced by the method described in Example 9-1.

### 9-3. Production of Her2 ADC

Trastuzumab with a $K_d$ value of 2.7*10$^{-9}$ M was used as a control antibody, and in order to produce an antibody that is suitable for use in the present invention, amino acids in the Fv sequence were changed from Trastuzumab one by one to produce an antibody (Trastuzumab-1) with a $K_d$ value of 3*10$^{-8}$ M.

Trastuzumab-1/19-1 (DAR 8), Trastuzumab-1/25-6 (DAR 8), Trastuzumab/19-1 (DAR 8) and Trastuzumab/25-6 (DAR 8) were produced by the method described in Example 9-1.

### *9-4. In vivo* evaluation of EGFR ADC

In the xenograft model of the MDA-MB-468 cell line, which is a TNBC model overexpressing EGFR, after the tumor volume reached 200 mm$^3$, Nimotuzumab/19-1 and Cetuximab/19-1 were administered at 1 mpk, 5 mpk, and 10 mpk, respectively, and as shown in FIG. 22, it was confirmed that the Nimotuzumab-based ADC administration group showed better efficacy.

A similar experiment was conducted by using Nimotuzumab/25-6 and Cetuximab/25-6 in the same animal model, and as shown in FIG. 22, in the Nimotuzumab/25-6 administration group, a TGI improvement effect of approximately 25 to 40% was confirmed compared to the Cetuximab/25-6 administration group.

### 9-5. *In vivo* evaluation of Trop-2 ADC

In the xenograft model of the MDA-MB-468 cell line, which is a TNBC model overexpressing Trop-2, after the cancer volume reached 200 $mm^3$, two types of Sacituzumab-1 and Sacituzumab-based ADCs were administered at 5 mpk, and it was confirmed that Sacituzumab-1-based ADCs showed TGI improved by more than 30% compared to the original Sacituzumab-based ADC.

### 9-6. *In vivo* evaluation of Her2 ADC

After the cancer volume reached 200 $mm^3$ in the xenograft model of the JIMT-1 cell line, which is a breast cancer model overexpressing Her2, after the cancer volume reached 200 $mm^3$, two types of Trastuzumab-1 and Trastuzumab-based ADCs were administered at 5 mpk, and as shown in the figure below, it was confirmed that Trastuzumab-1-based ADCs showed a TGI that was improved by approximately 20% compared to the original Trastuzumab-based ADC.

From the above description, those skilled in the art will understand that the present invention can be implemented in other specific forms without changing its technical idea or essential features. In this regard, the embodiments described above should be understood in all respects as illustrative and not restrictive. The scope of the present invention should be construed as including the meaning and scope of the patent claims described below rather than the detailed description above, and all changes or modified forms derived from the equivalent concept thereof are included in the scope of the present invention.

## Claims

1. An immunoconjugate or a pharmaceutically acceptable salt thereof, wherein a camptothecin-based drug (A) is bound, through a linker (C), to: an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

2. The immunoconjugate or pharmaceutically acceptable salt thereof of claim 1, wherein the camptothecin-based drug is an active camptothecin derivative represented by Chemical Formula 1, Chemical Formula 2 or Chemical Formula 1-1 below that is designed to bind to DDX5 protein and E3 ligase:

[Chemical Formula 1]

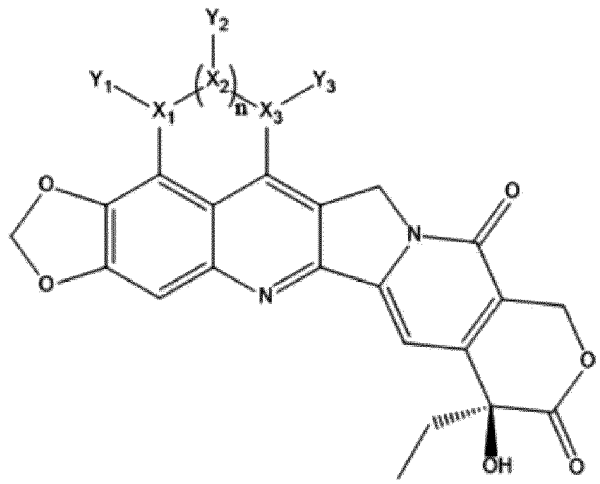

[Chemical Formula 2]

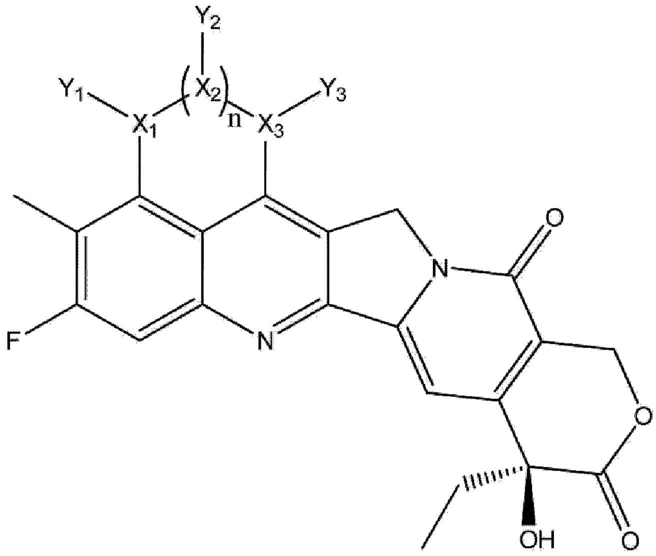

wherein $X_1$ and $X_3$ are each independently carbon, oxygen, nitrogen or sulfur, and $X_1$ and $X_3$ are the same or different,
wherein $X_2$ is carbon, oxygen, nitrogen, sulfur, a single bond or a double bond,
wherein $X_1$, $(X_2)n$ and $X_3$ form a 5-membered, 6-membered or 7-membered ring (n= value of 0 to 2), and
wherein $Y_1$, $Y_2$ and $Y_3$ are each independently hydrogen, or a functional group comprising oxygen, nitrogen, phosphorus or sulfur.

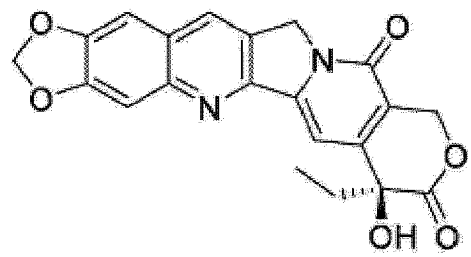

FL118

**3.** The immunoconjugate or pharmaceutically acceptable salt thereof of claim 1, wherein the immunoconjugate and/or payload released from the immunoconjugate penetrates into cancer tissue rather than just accumulating around vessels within cancer by an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody antigen-binding site-containing fragment (B-2) thereof that binds to a cell surface protein expressed in both normal cells and cancer cells and has a higher binding affinity with respect to an epitope of the cell surface protein when binding divalently than when binding monovalently.

**4.** The immunoconjugate or pharmaceutically acceptable salt thereof of claim 1, wherein the immunoconjugate increases a therapeutic effect by weakening cell internalization through receptor-mediated endocytosis by an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

**5.** The immunoconjugate or pharmaceutically acceptable salt thereof of claim 1, wherein the therapeutic effect of the immunoconjugate that has penetrated into the cancer tissue is to relieve a solid stress in the cancer microenvironment formed as the cancer cells proliferate, and optionally increase the access of an anticancer drug and/or immunotherapeutic agent by an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

**6.** The immunoconjugate or pharmaceutically acceptable salt thereof of claim 1, wherein an on-target toxicity problem of the immunoconjugate is solved to exert an anticancer effect by an antibody or an antigen-binding site-containing fragment (B-1) thereof having a dissociation constant $K_d$ value of $1*10^{-8}$ M or more to less than $1*10^{-6}$ M with respect to an epitope of a cell surface protein; or an antibody or antigen-binding site-containing fragment (B-2) thereof that binds to a cell surface protein expressed in both normal cells and cancer cells and has a higher binding affinity with respect to an epitope of the cell surface protein when binding divalently than when binding monovalently.

**7.** The immunoconjugate or pharmaceutically acceptable salt thereof of claim 1, wherein the dosage of the immunoconjugate or pharmaceutically acceptable salt thereof is at a level of 4.8 mpk or less, and preferably, at a level of 2 mpk to 4.5 mpk.

**8.** The immunoconjugate or pharmaceutically acceptable salt thereof of claim 1, wherein the immunoconjugate or pharmaceutically acceptable salt thereof has a DAR of 4 to 8 that indicates the number of drugs attached per antibody with respect to the antibody, or a DAR of 1 to 4 with respect to the antigen-binding site-containing fragment.

**9.** The immunoconjugate or pharmaceutically acceptable salt thereof of claim 1, wherein the linker (C) is (i) an acid-sensitive linker or (ii) an enzyme-sensitive linker.

**10.** The immunoconjugate or pharmaceutically acceptable salt thereof of claim 1, wherein the antibody or antigen-binding site-containing fragment thereof (B) targets an EGFR antigen that is distributed on a cancer surface and/or an EGFR antigen that is distributed in small quantities in normal tissues.

**11.** The immunoconjugate or pharmaceutically acceptable salt thereof of claim 1, wherein the antibody or antigen-binding site-containing fragment thereof (B) is nimotuzumab, a modified antibody having an antigen-binding site of nimotuzumab or a antigen-binding site-containing fragment of nimotuzumab.

**12.** The immunoconjugate or pharmaceutically acceptable salt thereof of claim 1, wherein an antibody or an antigen-binding site-containing fragment thereof (B) that binds to a cell surface protein selected from the group consisting of Her2, EGFR, Trop2, c-Met, CEACAM-5, B7H3, B7H4, Muc1, CDH6, CLDN18.2, FolR and PSMA is used.

**13.** The immunoconjugate or pharmaceutically acceptable salt thereof of claim 9, wherein after being targeted to cancer cells by an antigen-binding site that targets an EGFR epitope of cancer cells, an acid-sensitive linker is decomposed in the acidic environment ($pH \leq 7$) around a cancer cell, thereby releasing at least a portion of the camptothecin-based drug (A) that degrades the DDX5 protein, and a free camptothecin-based drug (A) penetrates deep into the cancer tissue and moves into cells by penetrating the cell membrane.

**14.** The immunoconjugate or pharmaceutically acceptable salt thereof of claim 9, wherein an acid-sensitive linker is stable in the neutral environment of the bloodstream (pH 7.3 to 7.5), but is hydrolyzed around tumor cells (pH 6.5 to 7.2), in endosomes (pH 5.0 to 6.5) or lysosomes (pH 4.5 to 5.0) after internalization into cells, thereby releasing an active drug.

**15.** The immunoconjugate or pharmaceutically acceptable salt thereof of claim 9, wherein after being targeted to cancer cells by an antigen-binding site that targets an antigen of cancer cells, an acid-sensitive linker is decomposed in the acidic environment ($pH \leq 7$) around cancer cells such that at least a portion of the camptothecin-based drug is released, thereby releasing a drug both inside and outside cells.

**16.** The immunoconjugate or pharmaceutically acceptable salt thereof of claim 9, wherein when an acid-sensitive linker is decomposed, the camptothecin-based drug and the acid-sensitive linker are linked by a carbonate or ester bond, so as to release a free camptothecin-based drug.

**17.** The immunoconjugate or pharmaceutically acceptable salt thereof of claim 9, wherein the acid-sensitive linker is derived from Chemical Formula 7 below:

[Chemical Formula 7]

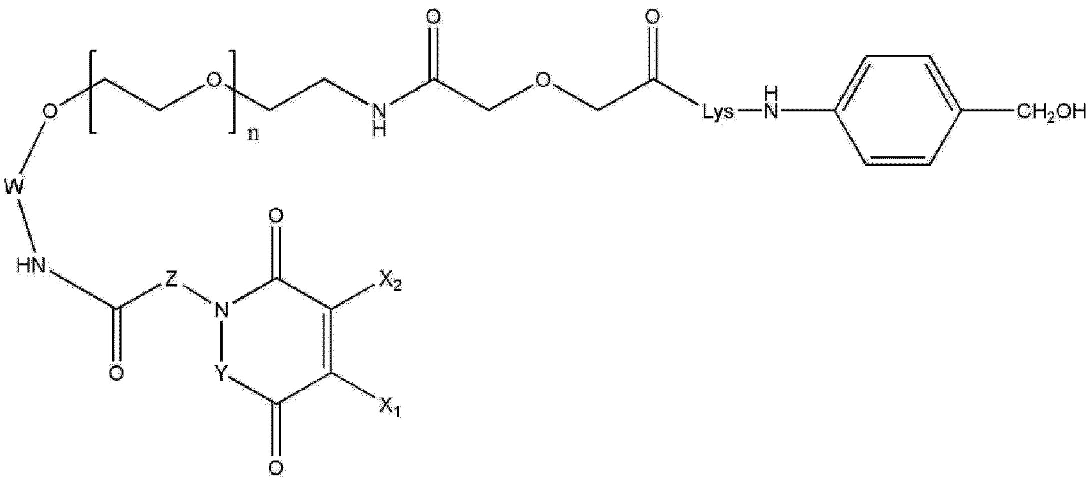

wherein $X_1$ and $X_2$ are each independently -H or -halogen;
wherein Y is -NH-, -NRA- or none (null);
wherein Z is $-C_1-C_4$alkyl-, $-C_3-C_6$cycloalkyl-, $-(C_1-C_2$alkyl)$-(C_3-C_6$cycloalkyl)-, $-(C_3-C_6$cycloalkyl)$-(C_1-C_2$alkyl)- or $-(C_1-C_2$alkyl)$-(C_3-C_6$cycloalkyl)$-(C_1-C_2$alkyl)-;
wherein W is $-R^B-$, -M- $-R^B-M-$, $-M-R^B-$, or $-R^B-M-R^C-$;
wherein $R^A$ to $R^C$ are each independently $C_1-C_4$alkyl,
Mis

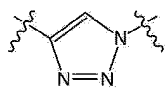

;

and
wherein n is an integer from 5 to 9.

**18.** The immunoconjugate or pharmaceutically acceptable salt thereof of claim 1, wherein the camptothecin drug (A) that degrades DDX5 protein kills target cells, then passes through the cell membrane to kill surrounding cells, and/or penetrates deep into cancer tissues and/or aggregates with each other and is absorbed by macrophages and removed from the body.

**19.** A pharmaceutical composition for preventing or treating cancer, comprising the immunoconjugate or pharmaceutically acceptable salt thereof according to any one of claims 1 to 18 as an active ingredient.

[FIG. 1]

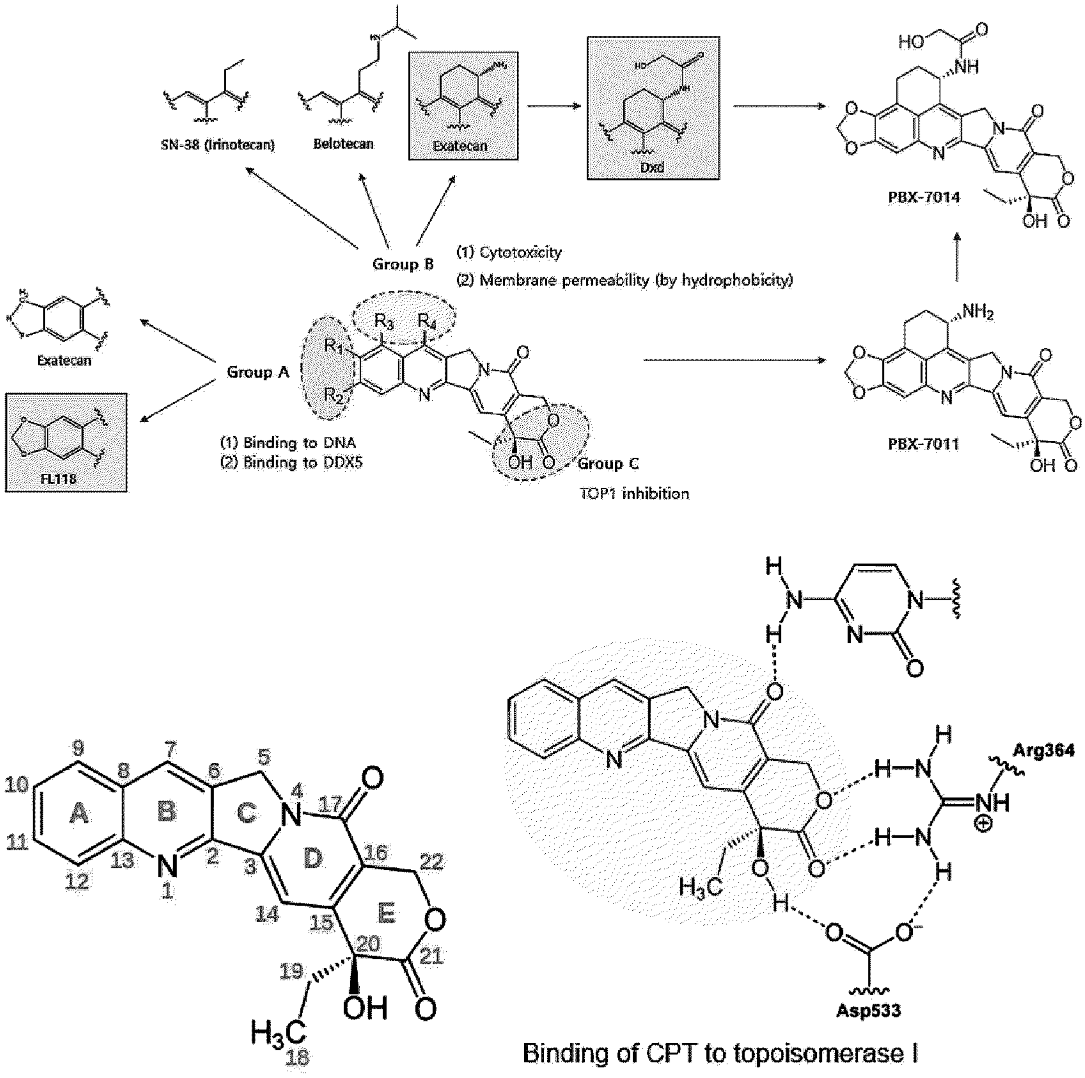
SN-38 (Irinotecan)
Belotecan
Exatecan
Dxd
PBX-7014
Group B
(1) Cytotoxicity
(2) Membrane permeability (by hydrophobicity)
Exatecan
FL118
Group A
(1) Binding to DNA
(2) Binding to DDX5
Group C
TOP1 inhibition
PBX-7011
Arg364
Asp533
Binding of CPT to topoisomerase I

[FIG. 2]

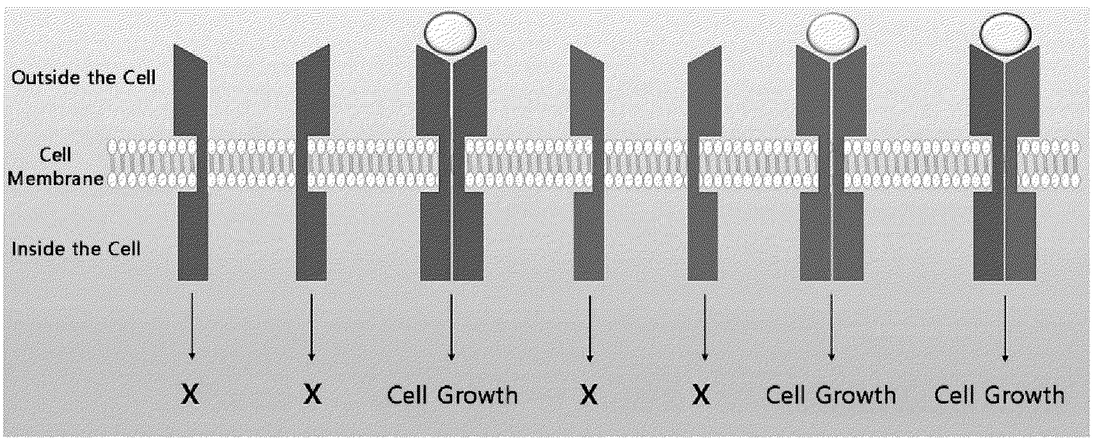
Outside the Cell
Cell Membrane
Inside the Cell
X
X
Cell Growth
X
X
Cell Growth
Cell Growth

[FIG. 3]

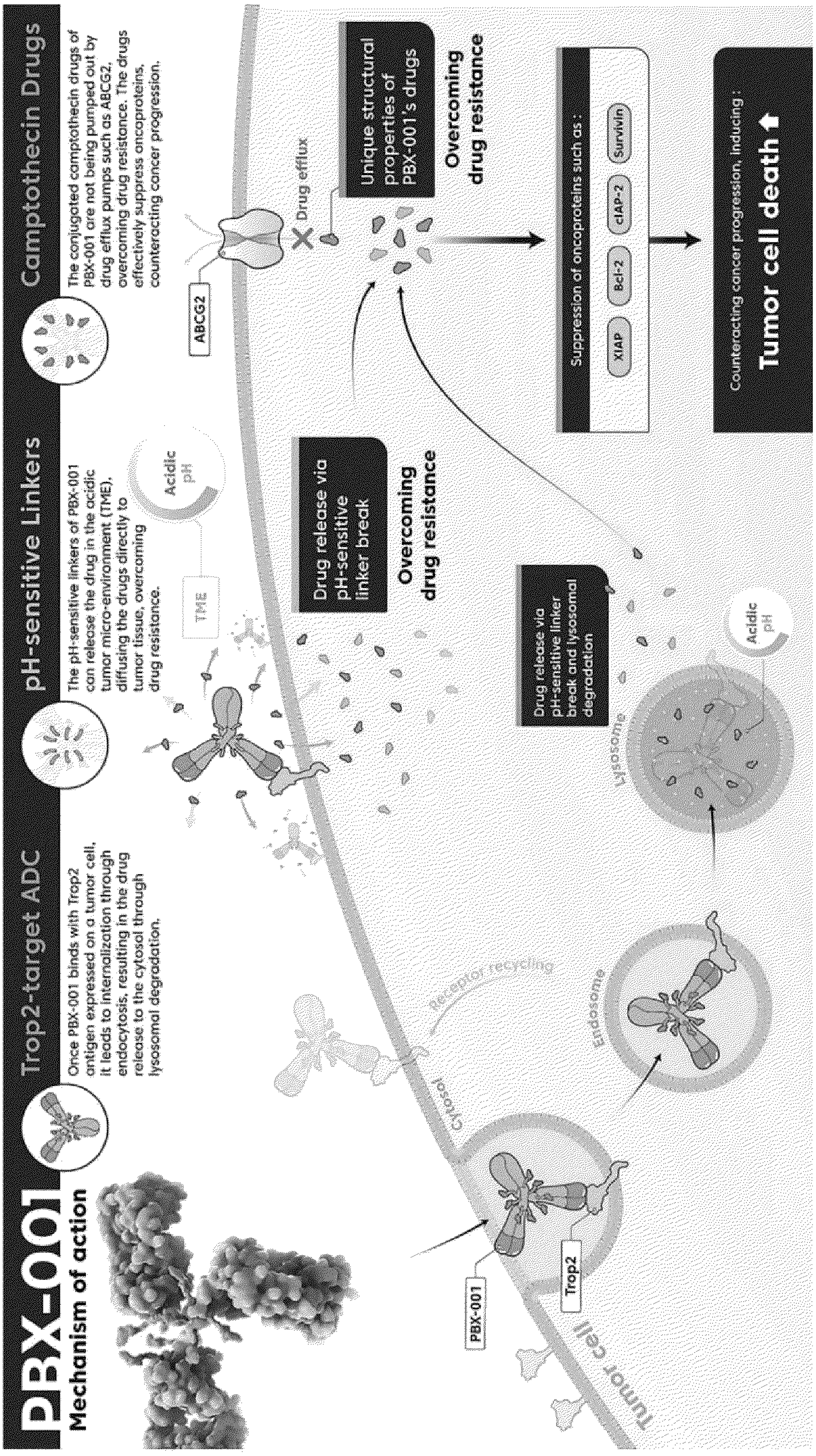
PBX-001
Mechanism of action
Trop2-target ADC
Once PBX-001 binds with Trop2 antigen expressed on a tumor cell, it leads to internalization through endocytosis, resulting in the drug release to the cytosol through lysosomal degradation.
pH-sensitive Linkers
The pH-sensitive linkers of PBX-001 can release the drug in the acidic tumor micro-environment (TME), diffusing the drugs directly to tumor tissue, overcoming drug resistance.
Camptothecin Drugs
The conjugated camptothecin drugs of PBX-001 are not being pumped out by drug efflux pumps such as ABCG2, overcoming drug resistance. The drugs effectively suppress oncoproteins, counteracting cancer progression.
TME
Acidic pH
ABCG2
Drug efflux
Drug release via pH-sensitive linker break
Overcoming drug resistance
Unique structural properties of PBX-001's drugs
Overcoming drug resistance
Suppression of oncoproteins such as :
XIAP
Bcl-2
cIAP-2
Survivin
Counteracting cancer progression, inducing :
Tumor cell death
Drug release via pH-sensitive linker break and lysosomal degradation
Acidic pH
Lysosome
Endosome
receptor recycling
Cytosol
PBX-001
Trop2
Tumor cell

[FIG. 4]

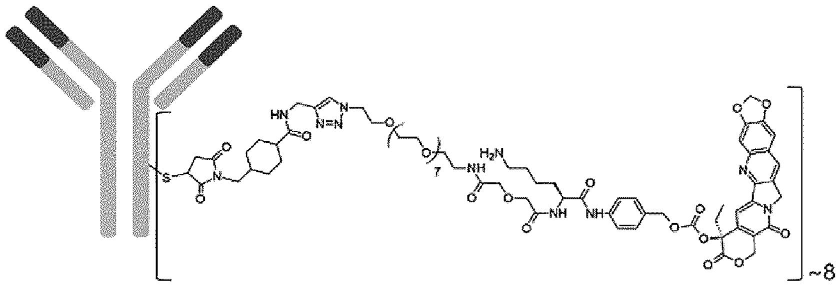

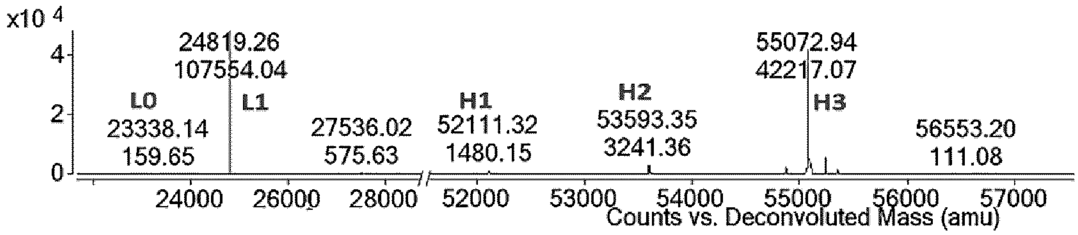


| Drug Distribution | L0 | L1 | L2 | H0 | H1 | H2 | H3 | H4 | MS-DAR |
|---|---|---|---|---|---|---|---|---|---|
| Mass | 23337 | 24818 | 26299 | 50629 | 52106 | 53588 | 55069 | 56551 | 7.74 |
| Peak Area | 0% | 100% | 0% | 0% | 3% | 7% | 90% | 0% | |

[FIG. 5]

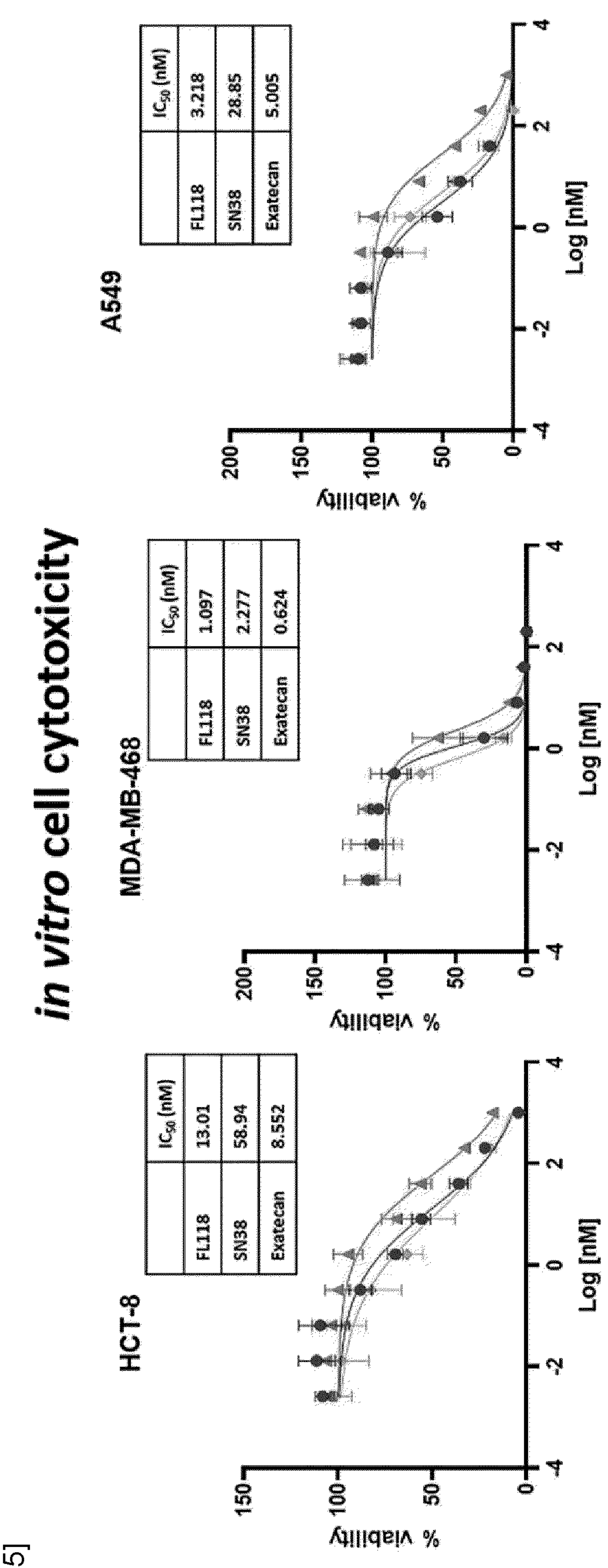
in vitro cell cytotoxicity
HCT-8
MDA-MB-468
A549
% viability
Log [nM]
IC50 (nM)
FL118 13.01
SN38 58.94
Exatecan 8.552
FL118 1.097
SN38 2.277
Exatecan 0.624
FL118 3.218
SN38 28.85
Exatecan 5.005

[FIG. 6]

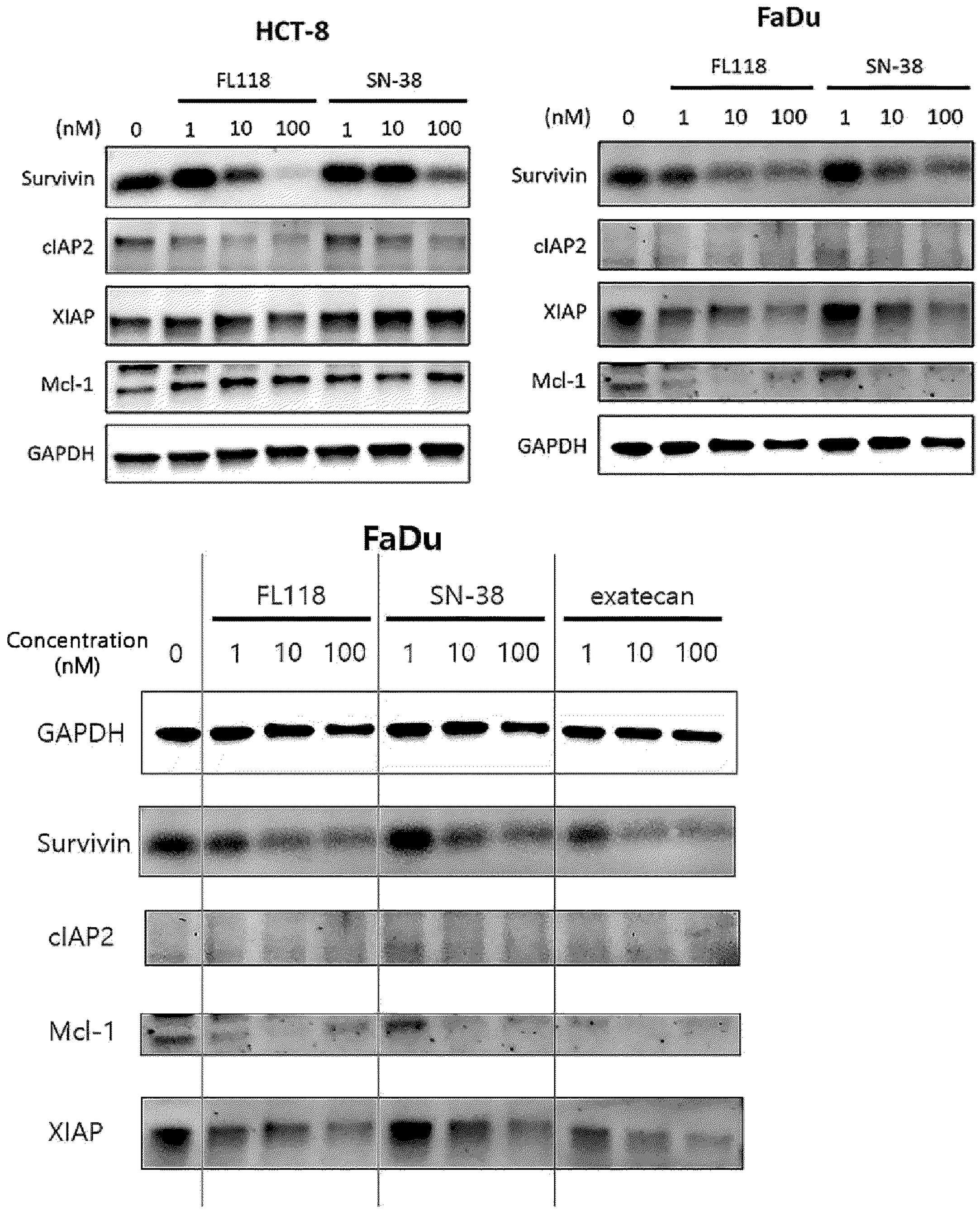
HCT-8
FL118
SN-38
(nM) 0 1 10 100 1 10 100
Survivin
cIAP2
XIAP
Mcl-1
GAPDH
FaDu
FL118
SN-38
(nM) 0 1 10 100 1 10 100
Survivin
cIAP2
XIAP
Mcl-1
GAPDH
FaDu
FL118
SN-38
exatecan
Concentration (nM) 0 1 10 100 1 10 100 1 10 100
GAPDH
Survivin
cIAP2
Mcl-1
XIAP

[FIG. 7]

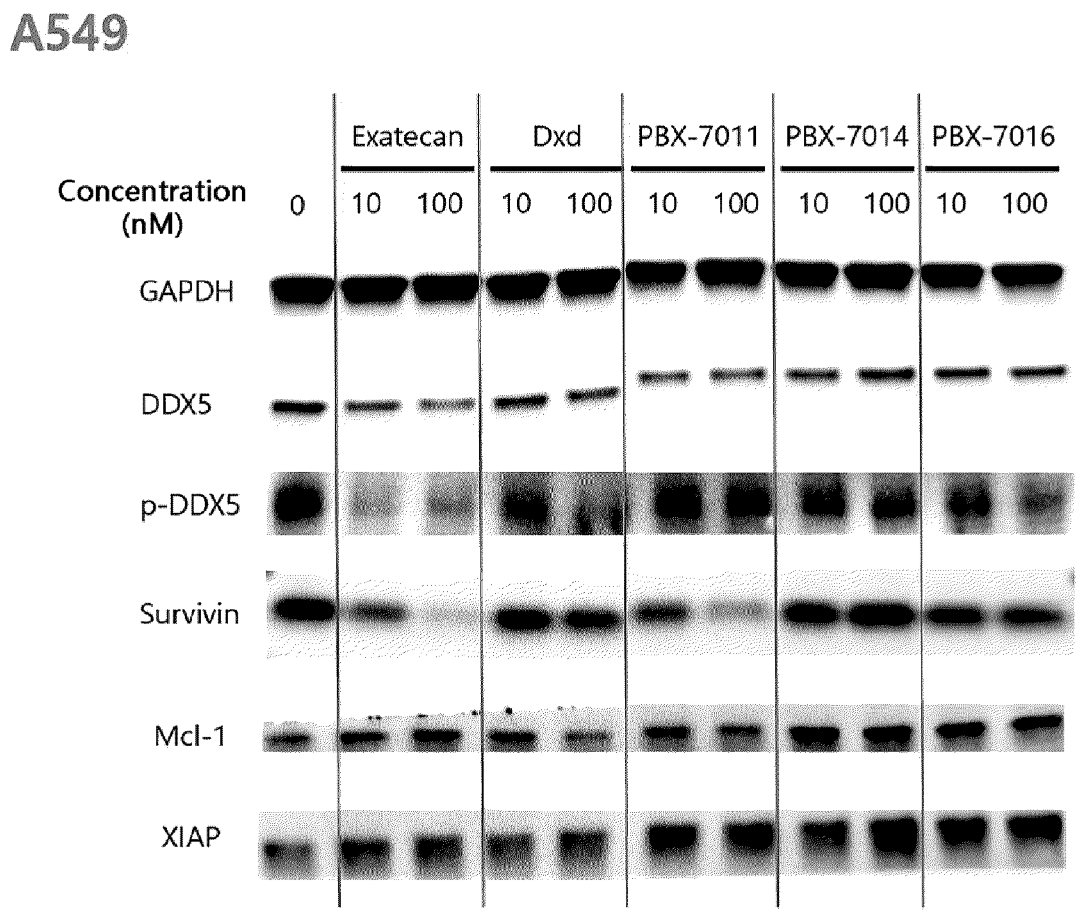
A549
Exatecan
Dxd
PBX-7011
PBX-7014
PBX-7016
Concentration (nM)
0
10 100
10 100
10 100
10 100
10 100
GAPDH
DDX5
p-DDX5
Survivin
Mcl-1
XIAP

[FIG. 8]

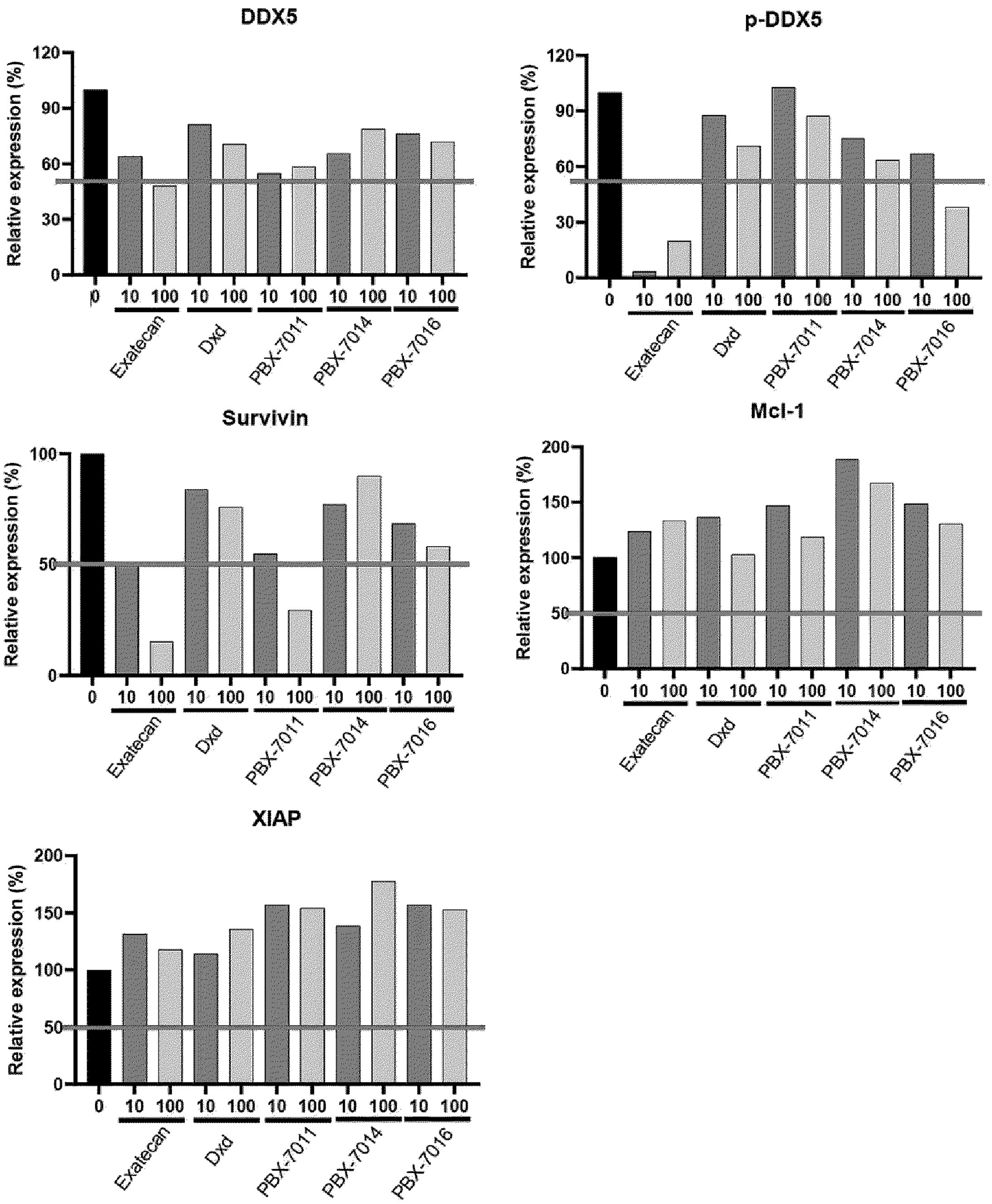
DDX5
p-DDX5
Survivin
Mcl-1
XIAP
Relative expression (%)
120
90
60
30
0
200
150
100
50
10 100
Exatecan
Dxd
PBX-7011
PBX-7014
PBX-7016

[FIG. 9]

FaDu (3days)

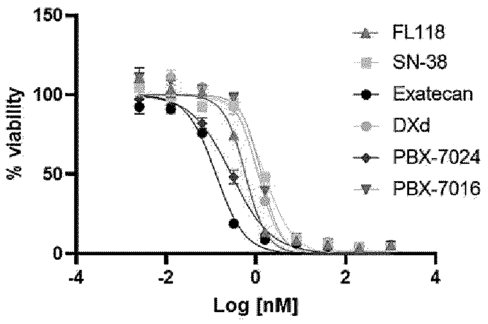


| Drug | $IC_{50}$ (nM) |
|---|---|
| FL118 | 0.5844 |
| SN-38 | 1.555 |
| Exatecan | 0.1298 |
| DXd | 1.130 |
| PBX-7016 | 1.331 |
| PBX-7024 | 0.2841 |

A549 (3days)

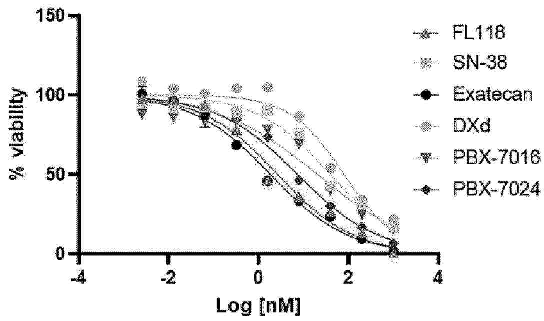


| Drug | $IC_{50}$ (nM) |
|---|---|
| FL118 | 2.670 |
| SN-38 | 39.70 |
| Exatecan | 1.845 |
| DXd | 80.25 |
| PBX-7016 | 20.47 |
| PBX-7024 | 7.387 |

FaDu (3days)

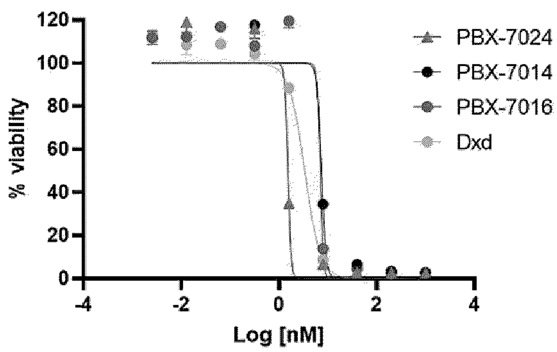


| Drug | $IC_{50}$ (nM) |
|---|---|
| PBX-7024 | 1.528 |
| PBX-7014 | 7.460 |
| PBX-7016 | 7.022 |
| Dxd | 3.369 |

A549 (3days)

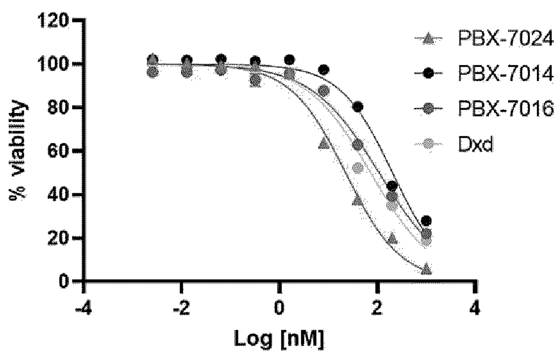


| Drug | $IC_{50}$ (nM) |
|---|---|
| PBX-7024 | 22.51 |
| PBX-7014 | 205.4 |
| PBX-7016 | 109.0 |
| Dxd | 73.24 |

[FIG. 10]

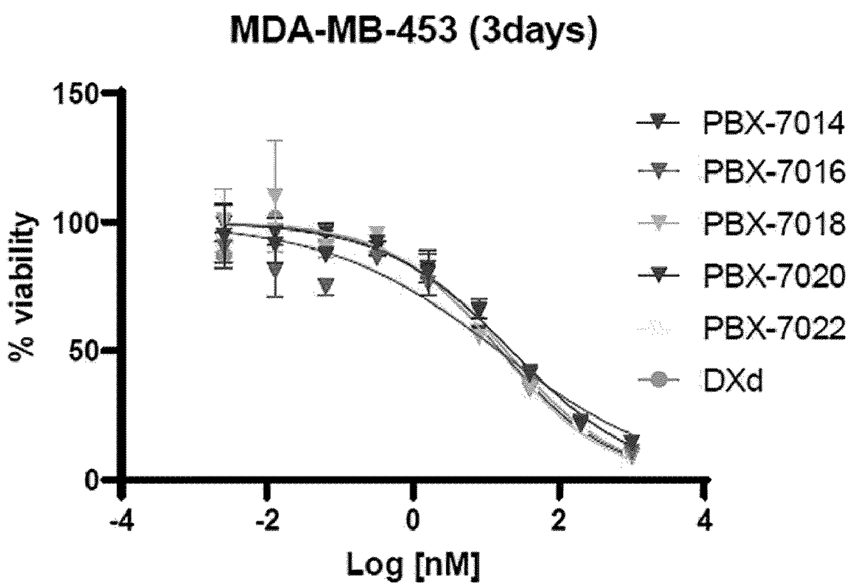


| Drug | $IC_{50}$ (nM) |
|---|---|
| PBX-7014 | 22.35 |
| PBX-7016 | 14.98 |
| PBX-7018 | 14.91 |
| PBX-7020 | 15.78 |
| PBX-7022 | 16.33 |
| DXd | 18.48 |

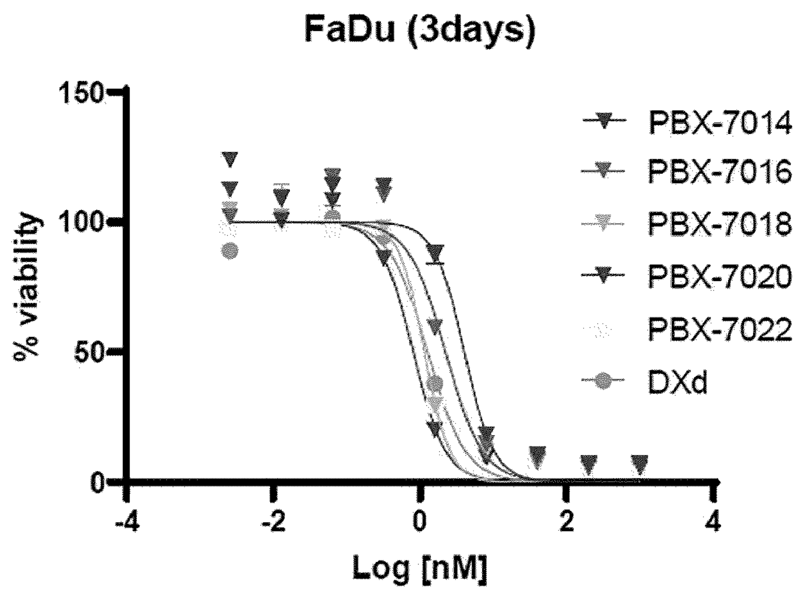


| Drug | $IC_{50}$ (nM) |
|---|---|
| PBX-7014 | 4.037 |
| PBX-7016 | 2.223 |
| PBX-7018 | 1.145 |
| PBX-7020 | 0.8072 |
| PBX-7022 | 1.145 |
| DXd | 1.262 |

[FIG. 11]

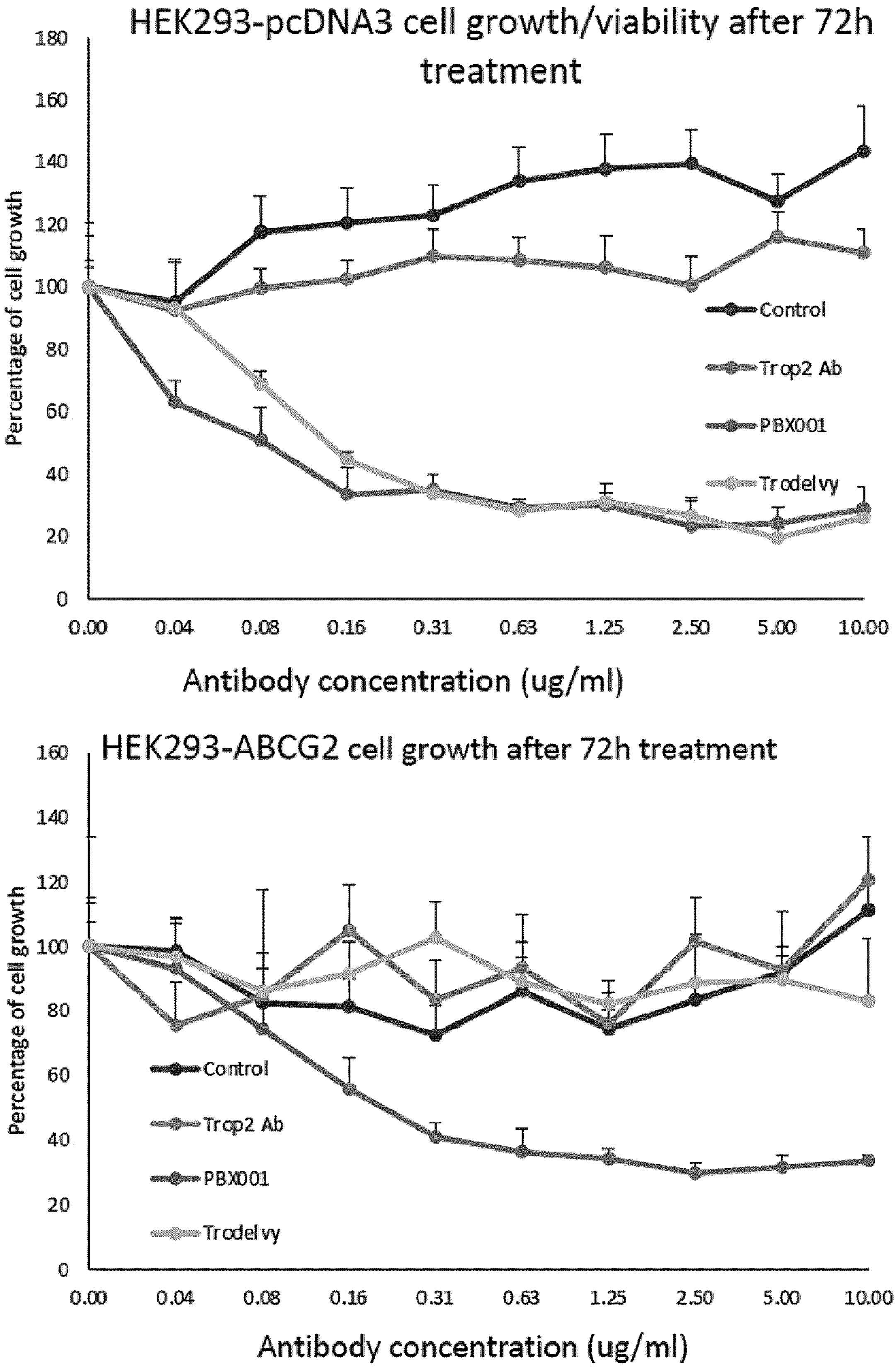
HEK293-pcDNA3 cell growth/viability after 72h treatment
180
160
140
120
100
80
60
40
20
0
Percentage of cell growth
Control
Trop2 Ab
PBX001
Trodelvy
0.00
0.04
0.08
0.16
0.31
0.63
1.25
2.50
5.00
10.00
Antibody concentration (ug/ml)
HEK293-ABCG2 cell growth after 72h treatment
160
140
120
100
80
60
40
20
0
Percentage of cell growth
Control
Trop2 Ab
PBX001
Trodelvy
0.00
0.04
0.08
0.16
0.31
0.63
1.25
2.50
5.00
10.00
Antibody concentration (ug/ml)

[FIG. 12]

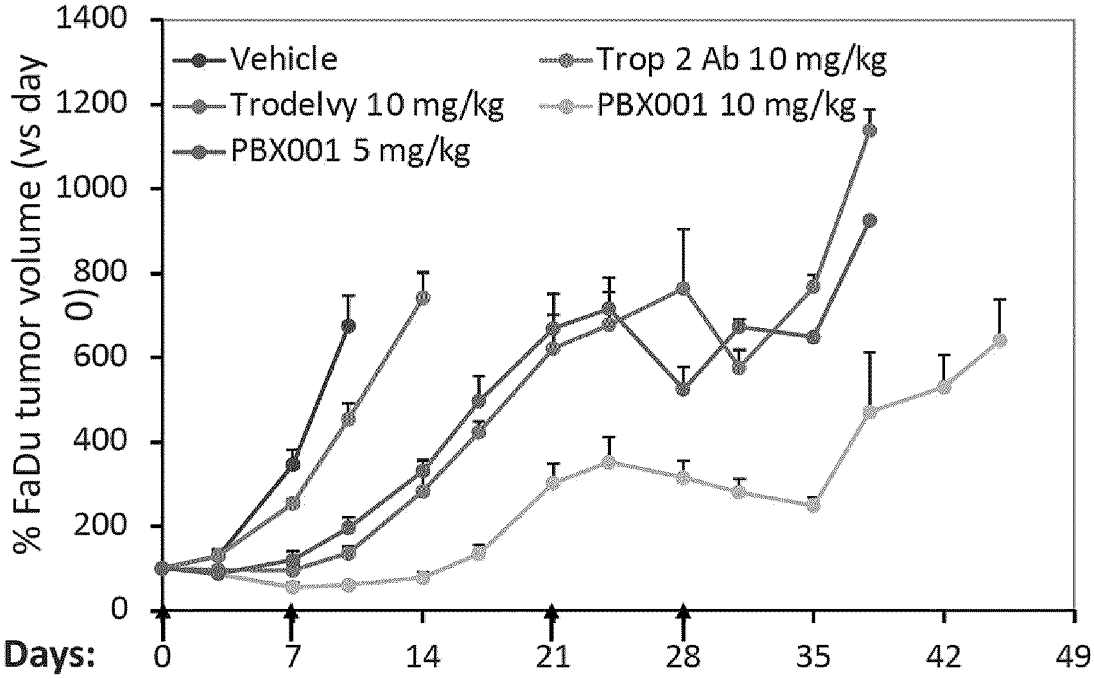
Vehicle
Trop 2 Ab 10 mg/kg
Trodelvy 10 mg/kg
PBX001 10 mg/kg
PBX001 5 mg/kg
% FaDu tumor volume (vs day 0)
1400
1200
1000
800
600
400
200
0
Days:
0
7
14
21
28
35
42
49

[FIG. 13]

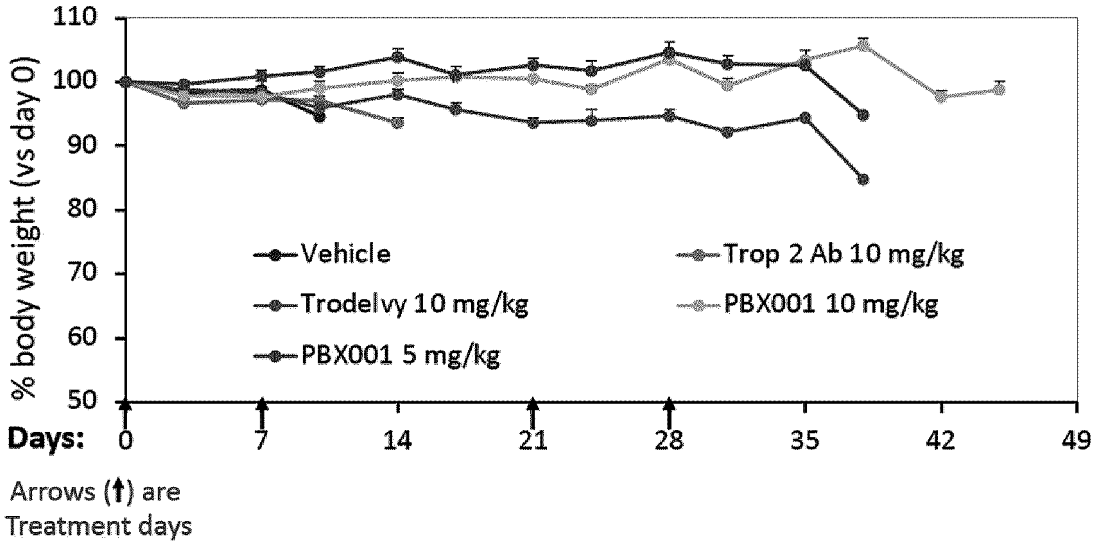
110
100
90
80
70
60
50
% body weight (vs day 0)
Vehicle
Trop 2 Ab 10 mg/kg
Trodelvy 10 mg/kg
PBX001 10 mg/kg
PBX001 5 mg/kg
Days:
0
7
14
21
28
35
42
49
Arrows (↑) are
Treatment days

[FIG. 14]

## Cetuximab-CL2A-FL118

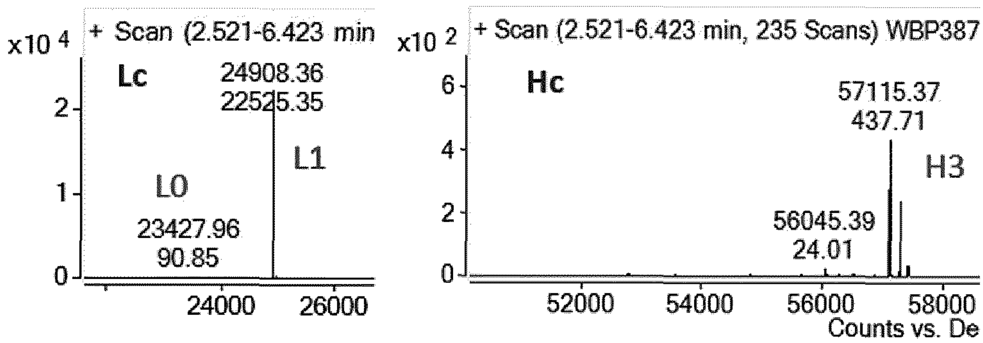


| Drug Distribution | L0 | L1 | H0 | H1 | H2 | H3 | MS-DAR |
|---|---|---|---|---|---|---|---|
| Mass | 23428 | 24908 | 52670 | 54150 | 55630 | 57110 | 7.99 |
| Peak Area | 1% | 99% | 0% | 0% | 0% | 100% | |

[FIG. 15]

## Nimotuzumab-CL2A-FL118

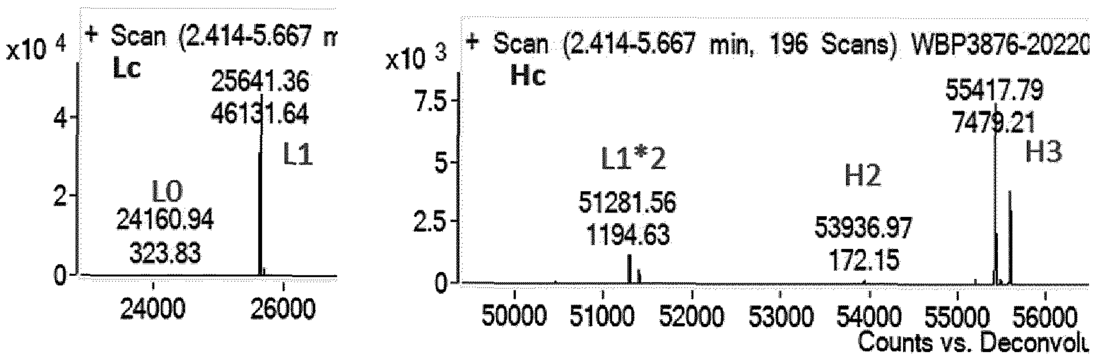


| Drug Distribution | L0 | L1 | H0 | H1 | H2 | H3 | MS-DAR |
|---|---|---|---|---|---|---|---|
| Mass | 24161 | 25641 | 50974 | 52454 | 53934 | 55414 | 7.94 |
| Peak Area | 1% | 99% | 0% | 0% | 2% | 98% | |

[FIG. 16]

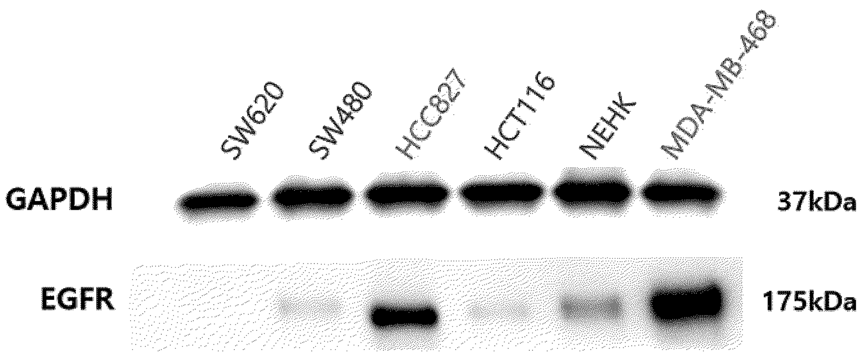


| EGFR Expression | Cell | EGFR level (RPPA score log2) |
|---|---|---|
| Positive Cell Line | MDA-MB-468 (Breast) | High |
| | HCC827 (Del19) (NSCLC) | High (3.576) |
| Negative Cell Line | SW620 (CRC) | Low (-1.34) |
| | SW480 (CRC) | Low (0.098) |
| | HCT116 (CRC) | Low (-0.356) |
| | Human Epidermal Keratinocyte (w/ EGFR +/++ over-expression) | |

| Cell line (tumor type) | EGFR (receptor/cell) | RN765C | Free payload |
|---|---|---|---|
| MDA-MB-468 (Breast) | ++++ | 0.013 | 0.020 |
| HCC827 (NSCLC) | +++ (358,000) | 0.030 | 0.293 |
| A431 (Epidermoid) | +++ (183,900) | 0.029 | 0.036 |
| FaDu (HNSCC) | ++ (53,800) | 0.806 | 0.060 |
| BxPC3 (Pancreas) | ++ (51,400) | 2.892 | 0.143 |
| NCI-H1975 (NSCLC) | +/++ (48,800) | 5.364 | 0.067 |
| HT29 (CRC) | +/++ (36,100) | 71.937 | 0.032 |
| NCI-H1650 (NSCLC) | +/++ (32,500) | 107.775 | 0.617 |
| SW620 (CRC) | - (0) | >200 (PR) | 0.085 |
| Human Epidermal Keratinocyte (Normal) | +/++ (37,800) | 72.618 | 0.098 |

[FIG. 17]

## Nimotuzumab ADC

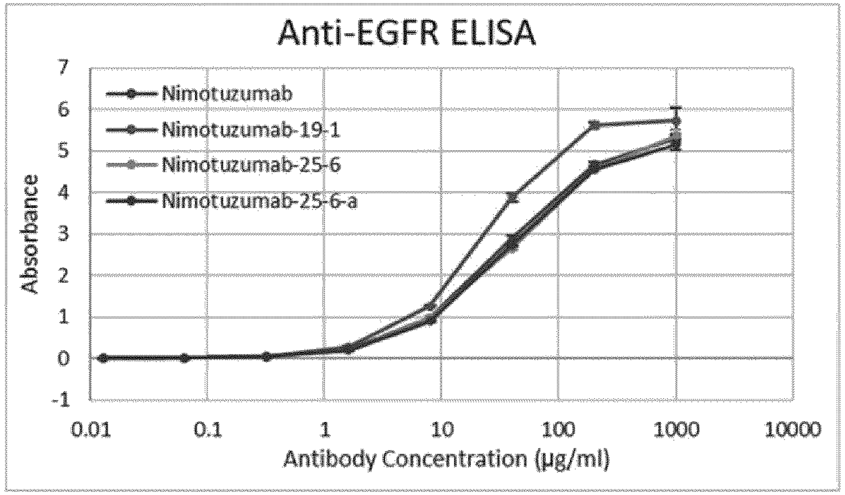


| ADC / AB | $BC_{50}$ (μg/ml) |
|---|---|
| Nimotuzumab | 35.99 |
| Nimotuzumab-19-1 | 22.99 |
| Nimotuzumab-25-6 | 44.81 |
| Nimotuzumab-25-6-a | 37.88 |

## Nimotuzumab(YTE) ADC

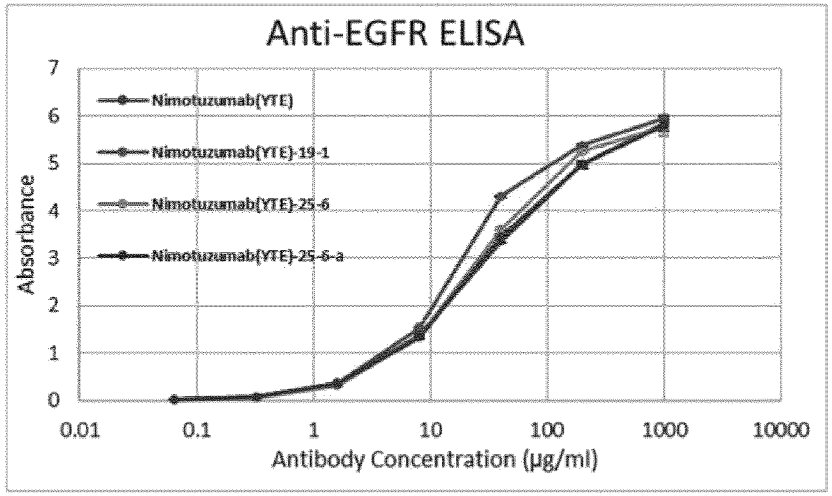


| ADC / AB | $BC_{50}$ (μg/ml) |
|---|---|
| Nimotuzumab(YTE) | 32.32 |
| Nimotuzumab(YTE)-19-1 | 18.41 |
| Nimotuzumab(YTE)-25-6 | 25.72 |
| Nimotuzumab(YTE)-25-6-a | 29.62 |

**$BC_{50}$ : binding concentration at 50% maximum**

[FIG. 18]

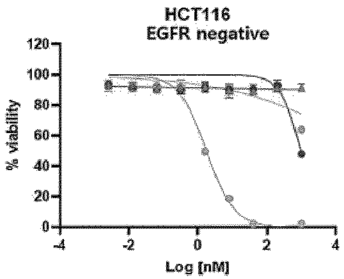


Nimotuzumab
NIM-19-1
NIM-25-6
NIM-25-6-a

| Drug | $IC_{50}$ (nM) |
|---|---|
| Nim | >10uM |
| NIM-19-1 | 1.776 |
| NIM-25-6 | 955.6 |
| NIM-25-6-a | >10uM |

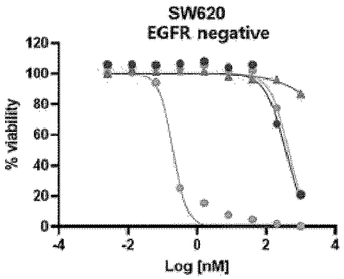


Nimotuzumab
NIM-19-1
NIM-25-6
NIM-25-6-a

| Drug | $IC_{50}$ (nM) |
|---|---|
| Nim | >10uM |
| NIM-19-1 | 0.2005 |
| NIM-25-6 | 362 |
| NIM-25-6-a | 443.4 |

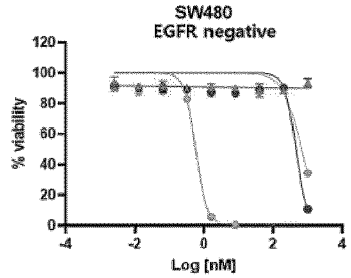


Nimotuzumab
NIM-19-1
NIM-25-6
NIM-25-6-a

| Drug | $IC_{50}$ (nM) |
|---|---|
| Nim | >10uM |
| NIM-19-1 | 0.5748 |
| NIM-25-6 | 453.7 |
| NIM-25-6-a | 675.3 |

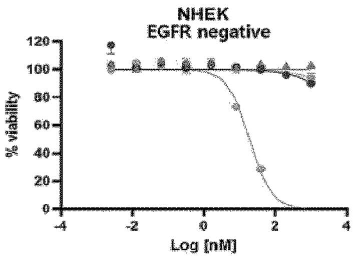


Nimotuzumab
NIM-19-1
NIM-25-6
NIM-25-6-a

| Drug | $IC_{50}$ (nM) |
|---|---|
| Nim | - |
| NIM-19-1 | 19.05 |
| NIM-25-6 | >10uM |
| NIM-25-6-a | >10uM |

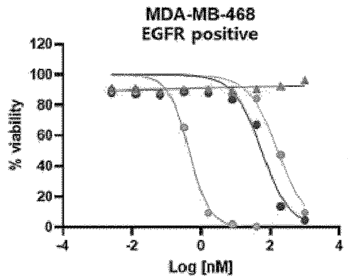


Nimotuzumab
NIM-19-1
NIM-25-6
NIM-25-6-a

| Drug | $IC_{50}$ (nM) |
|---|---|
| Nim | >10uM |
| NIM-19-1 | 0.4454 |
| NIM-25-6 | 56.44 |
| NIM-25-6-a | 167.1 |

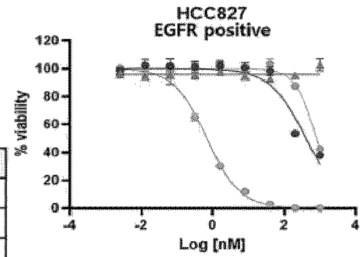


Nimotuzumab
NIM-19-1
NIM-25-6
NIM-25-6-a

| Drug | $IC_{50}$ (nM) |
|---|---|
| Nim | - |
| NIM-19-1 | 0.6883 |
| NIM-25-6 | 407.1 |
| NIM-25-6-a | 803.7 |

| ADC samples code name | |
|---|---|
| 1) Nim-FL118-8 | Nim-19-1 |
| 2) Nim-7016-8 | Nim-25-6 |
| 3) Nim-7016(Br)-4 | Nim-25-6-a |

1. Assay: Cell Titer Glo assay (Promega, cat# G7571)
2. Starting concentration: ~ 1 uM
3. Duplicate, 1/5 dilution, 96 well white opaque plate Triplicate
4. Treatment time: 6 days

[FIG. 19]

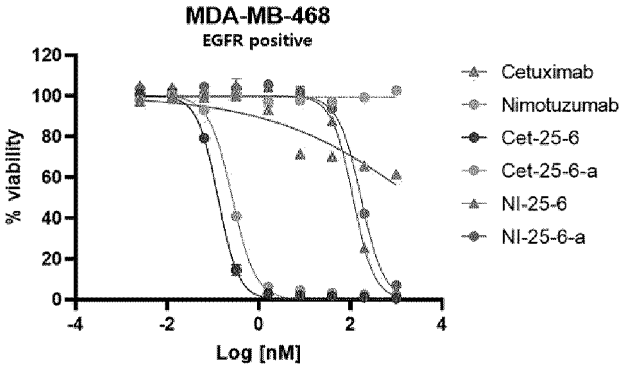


| Drug | $IC_{50}$ (nM) |
|---|---|
| Cetuximab | >10uM |
| Nimotuzumab | Interrupted |
| Cet-25-6 | 0.1283 |
| Cet-25-6-a | 0.2623 |
| NI-25-6 | 113.7 |
| NI-25-6-a | 169.9 |

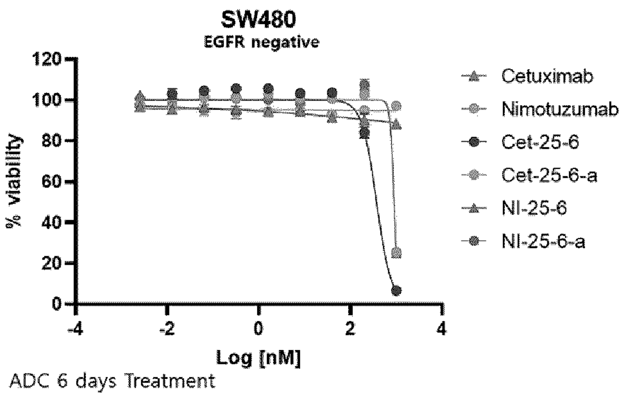


ADC 6 days Treatment

| Drug | $IC_{50}$ (nM) |
|---|---|
| Cetuximab | >10uM |
| Nimotuzumab | >10uM |
| Cet-25-6 | 371.9 |
| Cet-25-6-a | 904.5 |
| NI-25-6 | 894.9 |
| NI-25-6-a | 895.0 |

| ADC samples code name | |
|---|---|
| 1) Nim-FL118-8 | Nim-19-1 |
| 2) Nim-7016-8 | Nim-25-6 |
| 3) Nim-7016(Br)-4 | Nim-25-6-a |

[FIG. 20]

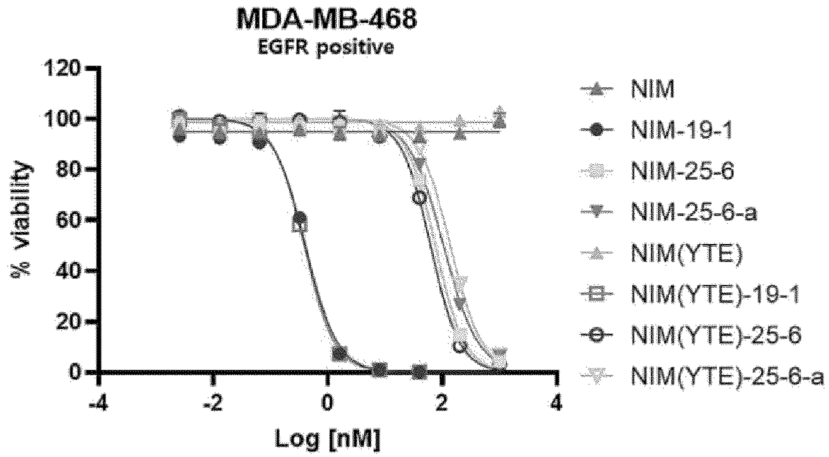


| Drug | $IC_{50}$ (nM) |
|---|---|
| Nimotuzumab | >10uM |
| NIM-19-1 | 0.4024 |
| NIM-25-6 | 75.11 |
| NIM-25-6-a | 104.5 |
| Nimotuzumab(YTE) | >10uM |
| NIM(YTE)-19-1 | 0.3831 |
| NIM(YTE)-25-6 | 61.89 |
| NIM(YTE)-25-6-a | 137.7 |

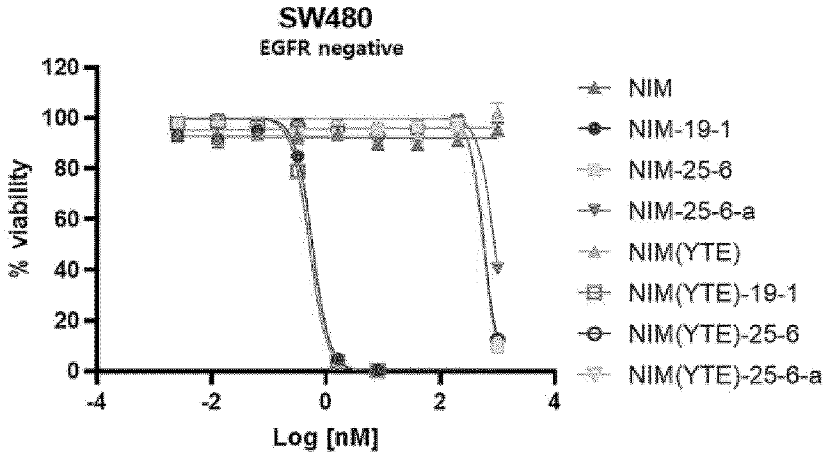


| Drug | $IC_{50}$ (nM) |
|---|---|
| Nimotuzumab | >10uM |
| NIM-19-1 | 0.5780 |
| NIM-25-6 | 548.4 |
| NIM-25-6-a | 881.6 |
| Nimotuzumab(YTE) | >10uM |
| NIM(YTE)-19-1 | 0.5093 |
| NIM(YTE)-25-6 | 573.7 |
| NIM(YTE)-25-6-a | 560.1 |

| ADC samples code name | |
|---|---|
| 1) Nim-FL118-8 | Nim-19-1 |
| 2) Nim-7016-8 | Nim-25-6 |
| 3) Nim-7016(Br)-4 | Nim-25-6-a |
| 4) Nim(YTE)-FL118-8 | Nim(YTE)-19-1 |
| 5) Nim(YTE)-7016-8 | Nim(YTE)-25-6 |
| 6) Nim(YTE)-7016(Br)-4 | Nim(YTE)-25-6-a |

ADC 6 days Treatment

[FIG. 21]

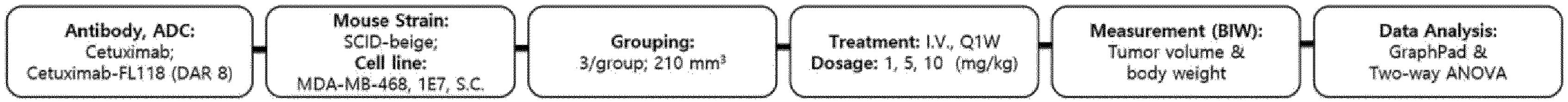


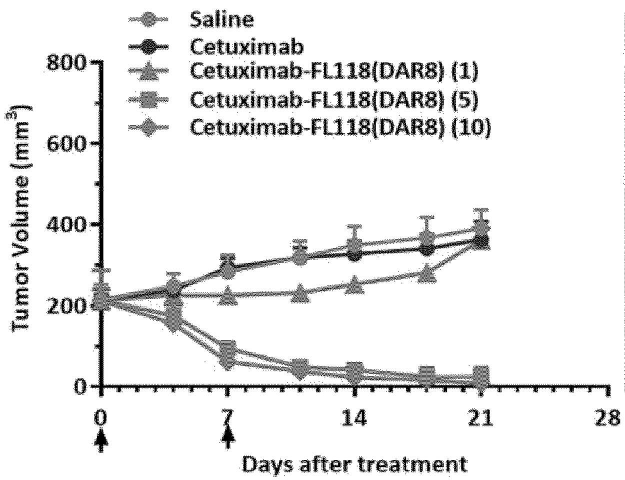


| Group (mg/kg) | TGI (%) |
|---|---|
| saline | NA |
| Cetuximab (10) | 15.92 |
| Cetuximab-FL118 (DAR 8) (1) | 14.71 |
| Cetuximab-FL118 (DAR 8) (5) | 207.18 |
| Cetuximab-FL118 (DAR 8) (10) | 216.60 |

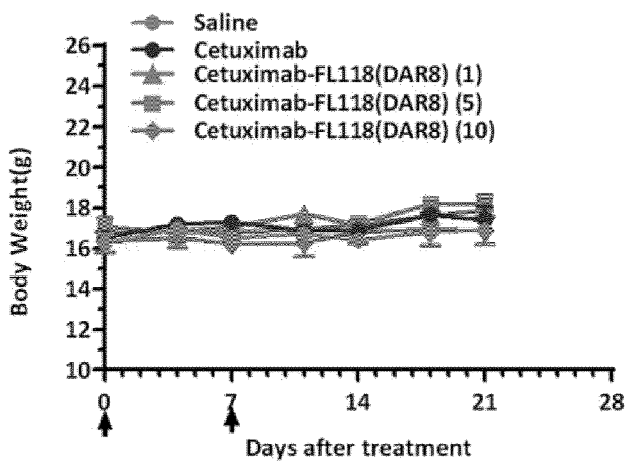

[FIG. 22]

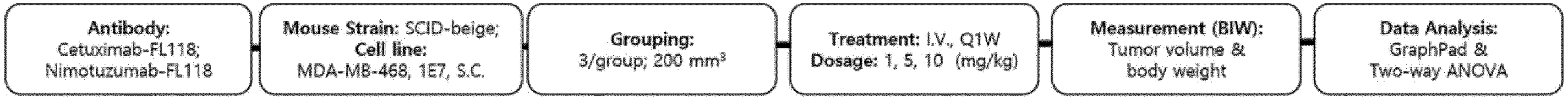


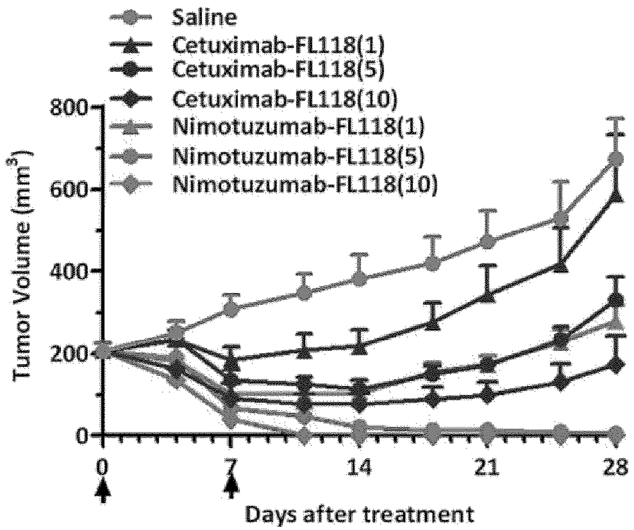


| Group (mg/kg) | TGI (%) |
|---|---|
| saline | NA |
| Cetuximab-FL118(DAR8) (1) | 18.40 |
| Cetuximab-FL118(DAR8) (5) | 73.29 |
| Cetuximab-FL118(DAR8) (10) | 106.93 |
| Nimotuzumab-FL118(DAR8) (1) | 84.47 |
| Nimotuzumab-FL118(DAR8) (5) | 142.98 |
| Nimotuzumab-FL118(DAR8) (10) | 144.11 |

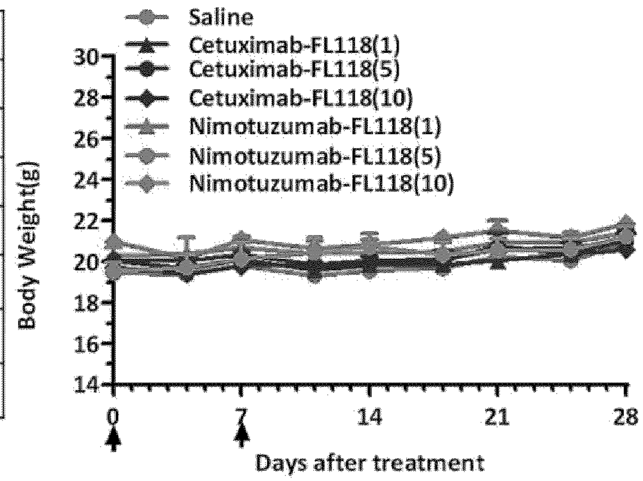

[FIG. 23]

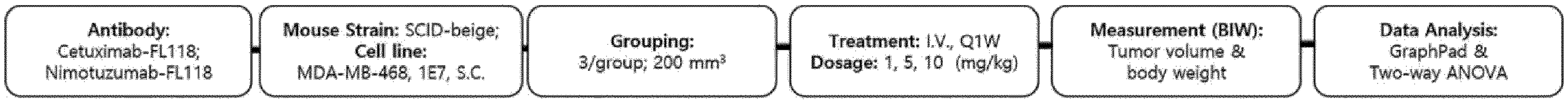


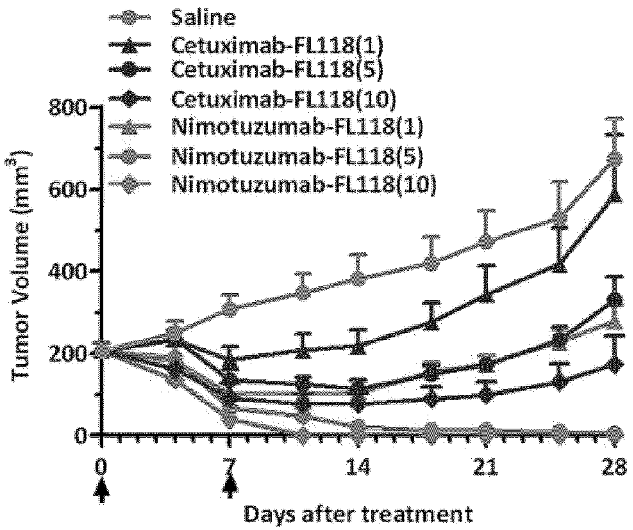


| Group (mg/kg) | TGI (%) |
|---|---|
| saline | NA |
| Cetuximab-FL118(DAR8) (1) | 18.40 |
| Cetuximab-FL118(DAR8) (5) | 73.29 |
| Cetuximab-FL118(DAR8) (10) | 106.93 |
| Nimotuzumab-FL118(DAR8) (1) | 84.47 |
| Nimotuzumab-FL118(DAR8) (5) | 142.98 |
| Nimotuzumab-FL118(DAR8) (10) | 144.11 |

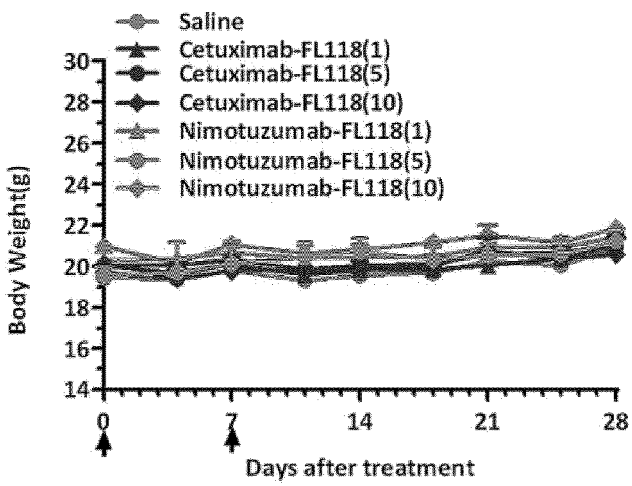

[FIG. 24]

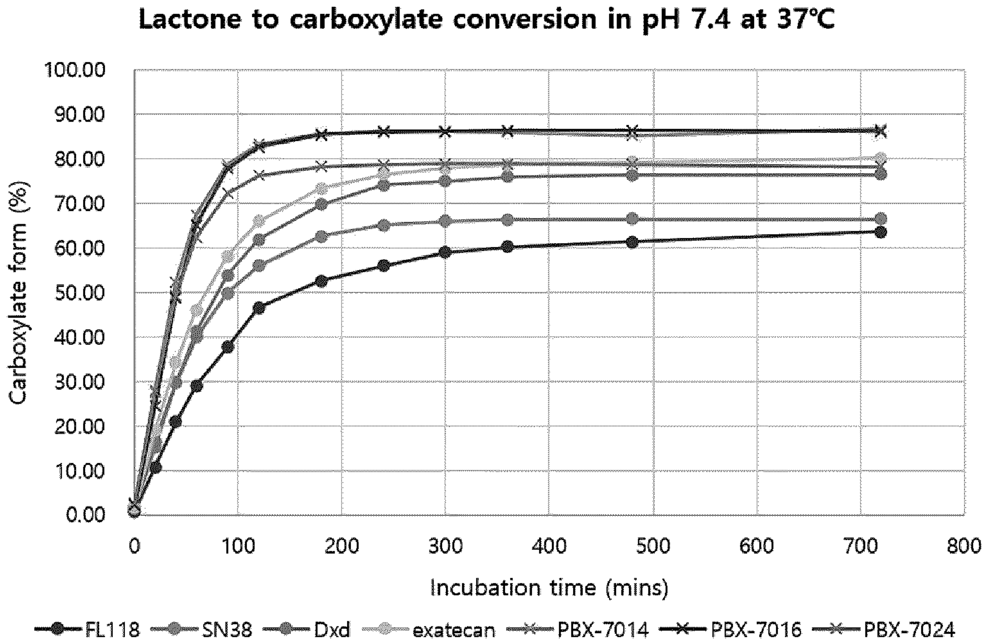


| Time (mins) | FL118 | SN38 | Dxd | exatecan | PBX-7014(1차) | PBX-7014(2차) | PBX-7014 | PBX-7016(1차) | PBX-7016 (2차) | PBX-7016 | PBX-7024(1차) | PBX-7024(2차) | PBX-7024 |
|---|---|---|---|---|---|---|---|---|---|---|---|---|---|
| 0 | 0.73 | 1.71 | 1.28 | 1.68 | 1.64 | 2.53 | 2.08 | 2.42 | 2.22 | 2.32 | 2.71 | 2.61 | 2.66 |
| 20 | 10.72 | 16.44 | 15.26 | 18.92 | 30.08 | 25.81 | 27.95 | 30.16 | 18.50 | 24.33 | 29.74 | 25.24 | 27.49 |
| 40 | 20.86 | 29.68 | 29.61 | 34.19 | 53.89 | 50.78 | 52.33 | 55.26 | 42.26 | 48.76 | 50.95 | 47.56 | 49.26 |
| 60 | 29.02 | 39.89 | 41.12 | 46.07 | 68.24 | 66.40 | 67.32 | 69.94 | 60.24 | 65.09 | 63.95 | 60.76 | 62.35 |
| 90 | 37.64 | 49.86 | 53.88 | 58.02 | 79.07 | 78.06 | 78.57 | 80.13 | 75.39 | 77.76 | 73.74 | 70.78 | 72.26 |
| 120 | 46.56 | 55.97 | 61.82 | 66.01 | 83.52 | 82.97 | 83.24 | 84.11 | 81.26 | 82.69 | 77.39 | 74.98 | 76.18 |
| 180 | 52.55 | 62.59 | 69.77 | 73.41 | 86.06 | 85.24 | 85.65 | 86.03 | 84.68 | 85.36 | 79.30 | 77.25 | 78.28 |
| 240 | 56.00 | 65.14 | 74.06 | 76.48 | 85.61 | 86.38 | 85.99 | 86.76 | 85.56 | 86.16 | 79.51 | 77.80 | 78.65 |
| 300 | 58.93 | 66.00 | 74.93 | 77.84 | 86.80 | 85.35 | 86.08 | 86.41 | 85.89 | 86.15 | 79.65 | 77.99 | 78.82 |
| 360 | 60.23 | 66.32 | 76.00 | 78.66 | 86.21 | 85.83 | 86.02 | 86.53 | 86.29 | 86.41 | 79.65 | 78.00 | 78.82 |
| 480 | 61.35 | 66.51 | 76.33 | 79.22 | 86.26 | 84.33 | 85.29 | 86.68 | 86.04 | 86.36 | 79.65 | 77.95 | 78.80 |
| 720 | 63.62 | 66.46 | 76.50 | 80.08 | 86.75 | 86.44 | 86.60 | 85.77 | 86.54 | 86.16 | 79.26 | 77.22 | 78.24 |

[FIG. 25]

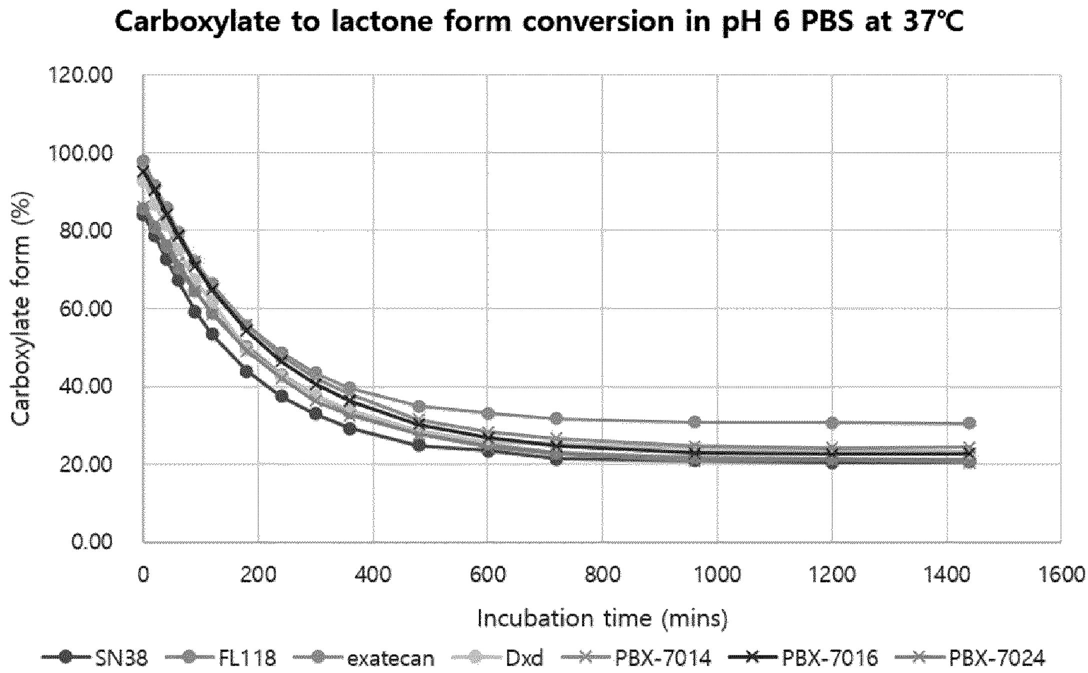


| Time (mins) | SN38 | FL118 | exatecan | Dxd | PBX-7014(1차) | PBX-7014(2차) | PBX-7014 | PBX-7016 (1차) | PBX-7016 (2차) | PBX-7016 | PBX-7024(1차) | PBX-7024(2차) | PBX-7024 |
|---|---|---|---|---|---|---|---|---|---|---|---|---|---|
| 0 | 84.02 | 85.67 | 97.90 | 92.78 | 94.97 | 94.60 | 94.79 | 95.40 | 95.01 | 95.21 | 85.88 | 86.45 | 86.17 |
| 20 | 78.64 | 80.70 | 91.68 | 86.70 | 90.23 | 91.13 | 90.68 | 90.38 | 90.11 | 90.25 | 80.96 | 82.37 | 81.67 |
| 40 | 72.63 | 76.03 | 85.82 | 81.70 | 84.62 | 84.40 | 84.51 | 84.61 | 83.61 | 84.11 | 75.58 | 78.26 | 76.92 |
| 60 | 67.09 | 70.14 | 79.59 | 75.10 | 79.09 | 78.83 | 78.96 | 79.30 | 77.97 | 78.63 | 70.34 | 73.28 | 71.81 |
| 90 | 59.20 | 64.39 | 71.96 | 67.12 | 71.91 | 71.88 | 71.89 | 71.40 | 70.50 | 70.95 | 63.57 | 65.58 | 64.57 |
| 120 | 53.26 | 58.90 | 66.17 | 61.67 | 65.73 | 65.54 | 65.64 | 64.83 | 64.57 | 64.70 | 57.45 | 59.68 | 58.56 |
| 180 | 43.78 | 50.10 | 55.61 | 49.46 | 55.86 | 55.65 | 55.75 | 54.34 | 54.16 | 54.25 | 48.29 | 49.45 | 48.87 |
| 240 | 37.43 | 42.95 | 48.59 | 42.58 | 48.45 | 47.06 | 47.76 | 46.52 | 46.25 | 46.39 | 40.90 | 43.07 | 41.99 |
| 300 | 32.84 | 37.57 | 43.33 | 37.57 | 42.55 | 41.83 | 42.19 | 40.38 | 40.80 | 40.59 | 35.34 | 37.06 | 36.20 |
| 360 | 29.18 | 33.22 | 39.53 | 33.77 | 38.39 | 37.51 | 37.95 | 36.06 | 36.40 | 36.23 | 31.86 | 32.93 | 32.40 |
| 480 | 24.73 | 28.36 | 34.90 | 28.59 | 32.43 | 30.37 | 31.40 | 30.26 | 29.94 | 30.10 | 26.68 | 28.42 | 27.55 |
| 600 | 23.41 | 25.06 | 33.07 | 25.66 | 29.16 | 27.37 | 28.26 | 26.91 | 26.57 | 26.74 | 23.16 | 25.51 | 24.34 |
| 720 | 21.37 | 22.83 | 31.62 | 25.24 | 27.35 | 25.64 | 26.50 | 24.64 | 24.66 | 24.65 | 22.28 | 22.67 | 22.47 |
| 960 | 20.86 | 21.78 | 30.73 | 24.05 | 25.57 | 23.66 | 24.62 | 23.05 | 22.43 | 22.74 | 19.90 | 21.45 | 20.67 |
| 1200 | 20.46 | 21.35 | 30.65 | 23.36 | 25.15 | 23.20 | 24.17 | 22.45 | 22.57 | 22.51 | 20.80 | 21.24 | 21.02 |
| 1440 | 20.56 | 21.05 | 30.45 | 23.17 | 24.96 | 23.53 | 24.24 | 22.09 | 23.30 | 22.70 | 19.48 | 21.39 | 20.44 |

[FIG. 26]

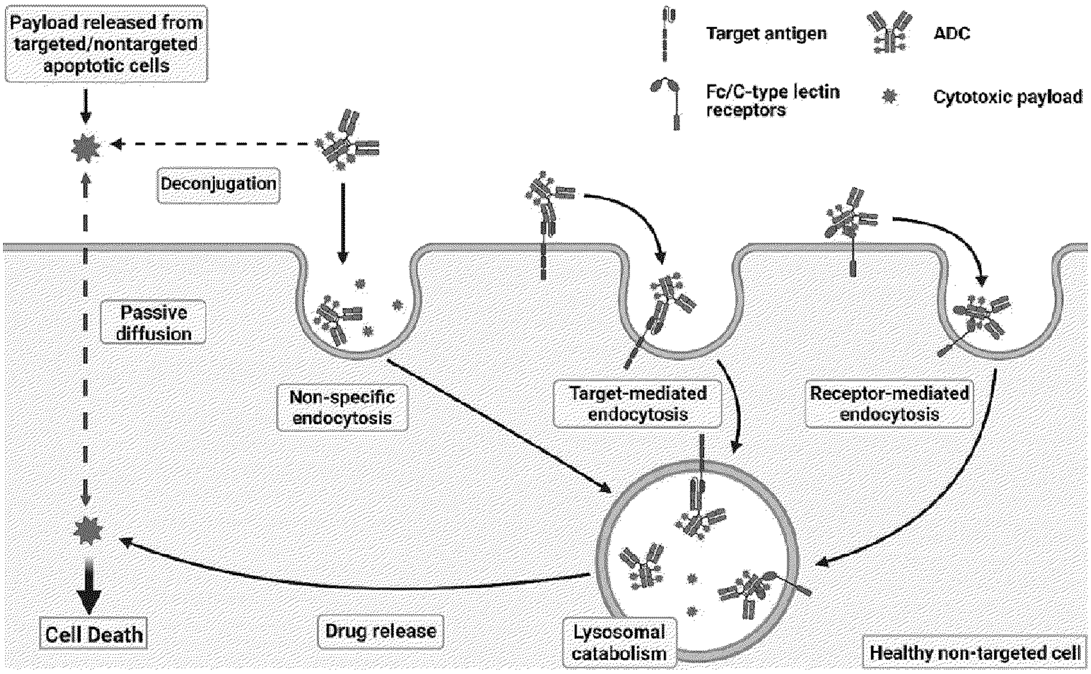
Payload released from targeted/nontargeted apoptotic cells
Target antigen
ADC
Fc/C-type lectin receptors
Cytotoxic payload
Deconjugation
Passive diffusion
Non-specific endocytosis
Target-mediated endocytosis
Receptor-mediated endocytosis
Cell Death
Drug release
Lysosomal catabolism
Healthy non-targeted cell

[FIG. 27]

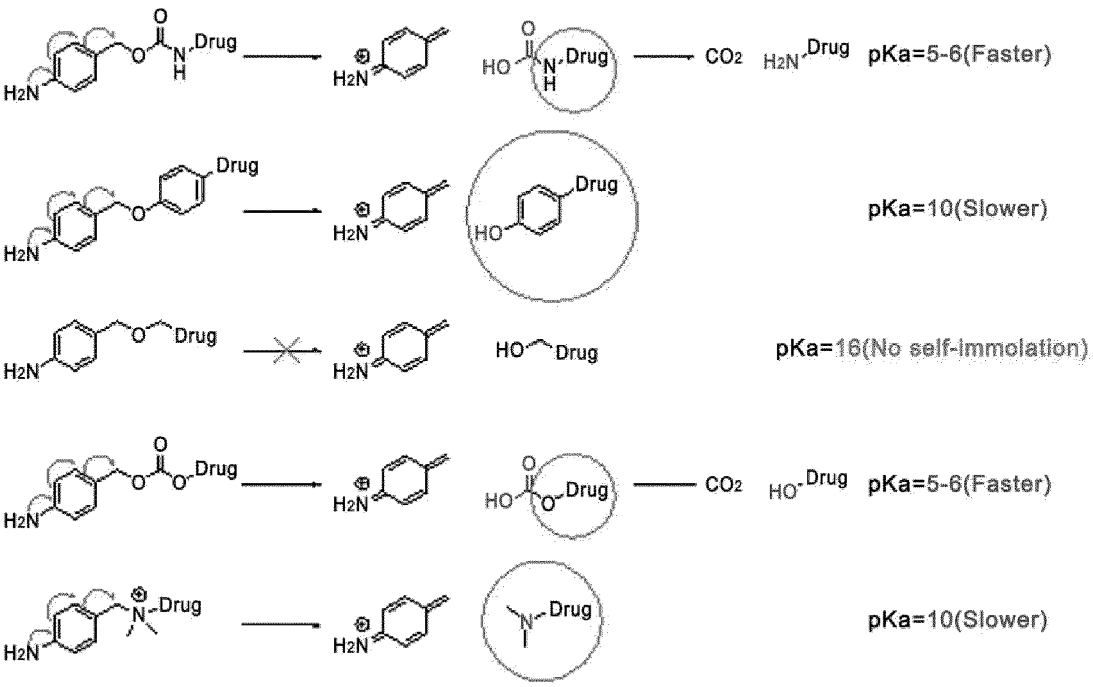
pKa=5-6(Faster)
pKa=10(Slower)
pKa=16(No self-immolation)
pKa=5-6(Faster)
pKa=10(Slower)

[FIG. 28]

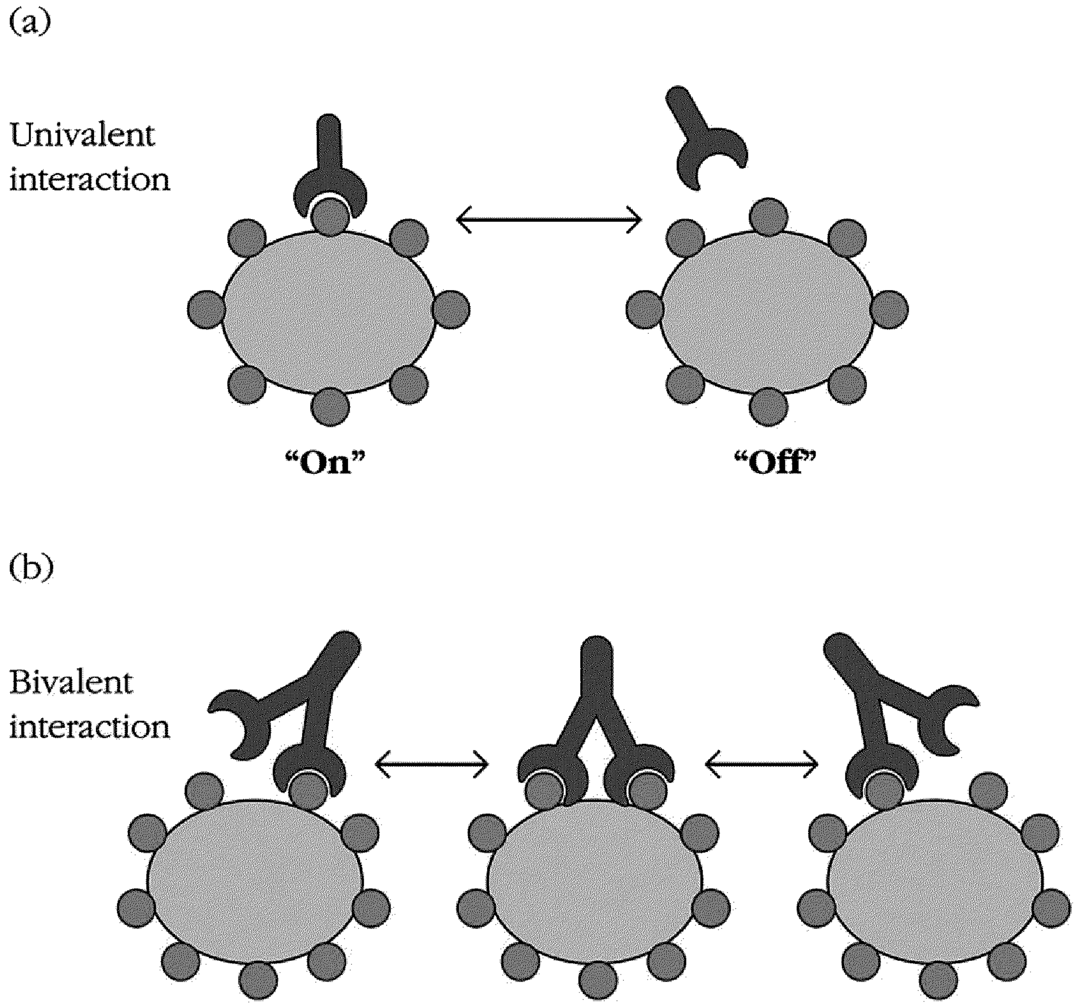
(a)
Univalent
interaction
"On"
"Off"
(b)
Bivalent
interaction

[FIG. 29]

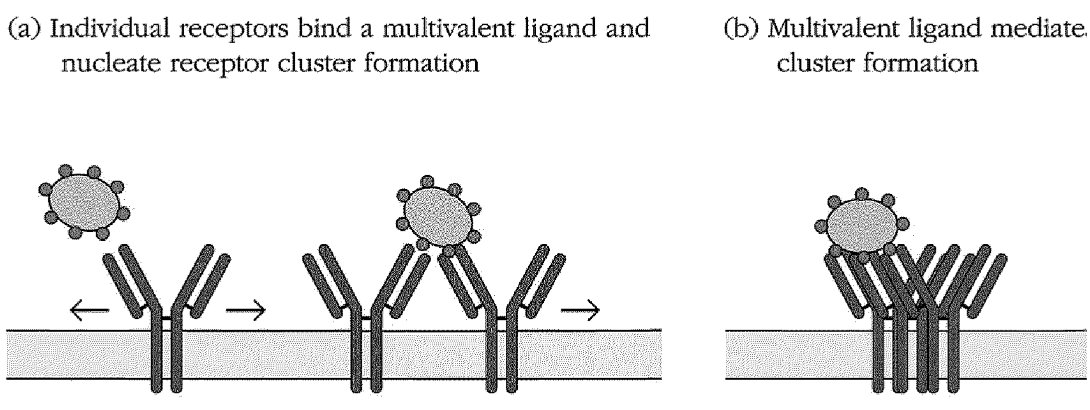
(a) Individual receptors bind a multivalent ligand and
nucleate receptor cluster formation
(b) Multivalent ligand mediate
cluster formation

## INTERNATIONAL SEARCH REPORT

International application No.
**PCT/KR2023/012935**

### A. CLASSIFICATION OF SUBJECT MATTER

**A61K 47/68**(2017.01)i; **A61K 31/4745**(2006.01)i; **A61P 35/00**(2006.01)i; **C07D 491/22**(2006.01)i; **A61K 47/65**(2017.01)i; **G01N 33/574**(2006.01)i

According to International Patent Classification (IPC) or to both national classification and IPC

### B. FIELDS SEARCHED

Minimum documentation searched (classification system followed by classification symbols)

A61K 47/68(2017.01); A61K 31/4745(2006.01); A61K 31/506(2006.01); A61K 31/519(2006.01); A61K 47/65(2017.01); A61P 35/00(2006.01); C07F 7/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 항암(anticancer), 캄토테신(camptothecin), 항체-약물 접합체(antibody-drug conjugate, ADC), DDX5 단백질(DDX5 protein), 제1형 토포이소머라제(topoisomerase-1), 니모투주맙(nimotuzumab)

### C. DOCUMENTS CONSIDERED TO BE RELEVANT

| Category* | Citation of document, with indication, where appropriate, of the relevant passages | Relevant to claim No. |
|---|---|---|
| X<br>Y | WO 2021-190581 A1 (JIANGSU HENGRUI MEDICINE CO., LTD. et al.) 30 September 2021 (2021-09-30)<br>See claims 1, 11, 13 and 19; and pages 4, 21 and 100-106. | 1-8,10-12,18,19<br>9,13-17 |
| Y | CN 111001012 A (SICHUAN BAILI PHARM CO., LTD.) 14 April 2020 (2020-04-14)<br>See abstract; claims 1, 9 and 10; and paragraphs [0004], [0135], [0136], [0182], [0183], [0244] and [0245]. | 9,13-17 |
| A | KR 10-2022-0079606 A (SEAGEN INC.) 13 June 2022 (2022-06-13)<br>See entire document. | 1-19 |
| DA | WO 2022-015110 A1 (PINOTBIO, INC.) 20 January 2022 (2022-01-20)<br>See entire document. | 1-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 November 2023** | **27 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No. PCT/KR2023/012935

C. DOCUMENTS CONSIDERED TO BE RELEVANT

| Category* | Citation of document, with indication, where appropriate, of the relevant passages | Relevant to claim No. |
|---|---|---|
| A | WO 2021-149817 A1 (TAIHO PHARMACEUTICAL CO., LTD. et al.) 29 July 2021 (2021-07-29) See entire document. | 1-19 |

Form PCT/ISA/210 (second sheet) (July 2022)

**INTERNATIONAL SEARCH REPORT**
**Information on patent family members**

International application No.
**PCT/KR2023/012935**

| Patent document cited in search report | Publication date (day/month/year) | Patent family member(s) | Publication date (day/month/year) |
|---|---|---|---|
| WO 2021-190581 A1 | 30 September 2021 | AU 2021-243073 A1 | 13 October 2022 |
| | | BR 112022019073 A2 | 08 November 2022 |
| | | CA 3175733 A1 | 30 September 2021 |
| | | CN 115103691 A | 23 September 2022 |
| | | EP 4129345 A1 | 08 February 2023 |
| | | JP 2023-518583 A | 02 May 2023 |
| | | KR 10-2022-0157998 A | 29 November 2022 |
| | | TW 202144013 A | 01 December 2021 |
| | | US 2023-0165969 A1 | 01 June 2023 |
| CN 111001012 A | 14 April 2020 | None | |
| KR 10-2022-0079606 A | 13 June 2022 | AU 2020-356955 A1 | 14 April 2022 |
| | | CA 3152316 A1 | 08 April 2021 |
| | | CN 114929284 A | 19 August 2022 |
| | | EP 4037717 A1 | 10 August 2022 |
| | | IL 291686 A | 01 May 2022 |
| | | JP 2022-550851 A | 05 December 2022 |
| | | MX 2022003930 A | 04 July 2022 |
| | | WO 2021-067861 A1 | 08 April 2021 |
| WO 2022-015110 A1 | 20 January 2022 | AU 2021-308834 A1 | 16 February 2023 |
| | | AU 2021-308834 A2 | 20 January 2022 |
| | | CA 3189462 A1 | 20 January 2022 |
| | | CN 116075321 A | 05 May 2023 |
| | | EP 4183418 A1 | 24 May 2023 |
| | | JP 2023-534292 A | 08 August 2023 |
| | | KR 10-2022-0009916 A | 25 January 2022 |
| | | KR 10-2022-0009928 A | 25 January 2022 |
| | | KR 10-2349925 B1 | 12 January 2022 |
| | | US 2023-0270867 A1 | 31 August 2023 |
| WO 2021-149817 A1 | 29 July 2021 | EP 4093406 A1 | 30 November 2022 |
| | | TW 202140024 A | 01 November 2021 |
| | | US 2023-0146795 A1 | 11 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9629926 B
- KR 2023005380 W
- WO 2022015110 A1
- KR 2023009854 W
- KR 1020220049158
- KR 1020220103591
- KR 102349925

### Non-patent literature cited in the description


- **CROMBET T** ; **OSORIO M** ; **CRUZ T et al.** Use of the humanized anti-epidermal growth factor receptor monoclonal antibody h-R3 in combination with radiotherapy in the treatment of locally advanced head and neck cancer patients. *J Clin Oncol*, 2004, vol. 22 (9), 1646-54
- **GARRIDO G** ; **RABASA A** ; **GRACIA E et al.** Binding properties of the anti-EGFR monoclonal antibody, nimotuzumab, limit its interaction with the EGFR in renal and epidermal cells. *AACR 100th Annual Meeting*, 22 June 2009, http://www.ymbiosciences.com/upload_files/nimo_poster_AACR2009.pdf
- *Cancers*, 2023, vol. 15 (3), 713, https://doi.org/10.3390/cancers15030713